(19) Europäisches Patentamt
European Patent Office
Office européen des brevets

(11) Publication number: **0 096 214 B1**

(12) # EUROPEAN PATENT SPECIFICATION

(45) Date of publication of patent specification: **27.02.91**

(51) Int. Cl.5: **A61K 31/505**, C07D 401/06, C07D 403/06, C07D 405/06, C07D 409/06, C07D 239/48, C07D 215/12, C07D 215/22, C07D 209/24, C07D 217/14, C07D 333/60

(21) Application number: **83104240.3**

(22) Date of filing: **29.04.83**

The file contains technical information submitted after the application was filed and not included in this specification

(54) **Antibacterial pyrimidine compounds.**

(30) Priority: **01.05.82 GB 8212727**
**01.05.82 GB 8212728**
**07.05.82 GB 8213248**
**07.05.82 GB 8213249**
**07.05.82 GB 8213250**

(43) Date of publication of application:
**21.12.83 Bulletin 83/51**

(45) Publication of the grant of the patent:
**27.02.91 Bulletin 91/09**

(84) Designated Contracting States:
**BE CH DE FR GB IT LI NL SE**

(56) References cited:
**EP-A- 0 051 879**
**GB-A- 957 797**
**US-A- 3 049 544**

(73) Proprietor: **THE WELLCOME FOUNDATION LIMITED**
**Unicorn House 160 Euston Road**
**London NW1 2BP(GB)**

(72) Inventor: **Daluge, Susan Mary**
**297 Azalea Drive**
**Chapel Hill North Carolina(US)**
Inventor: **Skonezny, Paul Marcel**
**6151 Wynmoor Drive**
**Clay New York(US)**
Inventor: **Roth, Barbara**
**7 Lone Pine Road**
**Chapel Hill North Carolina(US)**
Inventor: **Rauckman, Barbara Seavey**
**2024 Sohi Drive**
**Durham North Carolina(US)**

(74) Representative: **Sandmair, Kurt, Dr. et al**
**Patentanwälte Schwabe, Sandmair, Marx**
**Stuntzstrasse 16**
**D-8000 München 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 096 214 B1

## Description

The present invention relates to novel 2,4-diamino-5-(substituted)pyrimidines, to pharmaceutical compositions containing them, to processes for preparing them and their compositions, to certain intermediates for making them and to their use in the treatment of microbial infections.

Certain 2,4-diamino-5-benzylpyrimidines have been demonstrated to be potent inhibitors of dihydrofolate reductase (DHFR) which catalyses the reduction of dihydrofolic acid to tetrahydrofolic acid (THFA). This property has been shown frequently to result in useful pharmaceutical properties particularly in the treatment of bacterial infections. Thus, U.K. Patent Specification No.875,562 discloses *inter alia* 2,4-diamino-5-benzylpyrimidines wherein the benzyl moiety is substituted by three $C_{1-4}$ alkoxy groups.

Trimethoprim, 2,4-diamino-5-(3,4,5-trimethoxybenzyl)pyrimidine, is specifically disclosed in U.K. Patent No. 875,562 and is the most active general antibacterial agent amongst the 2,4-diamino-5-benzylpyrimidines known to date. Due to their mode of action, these benzylpyrimidines potentiate the antibacterial activity of the sulphonamides and trimethoprim has been used extensively over the last decade in human therapy in combination with various sulphonamides, and in particular with sulphamethoxazole, for the treatment of bacterial infections.

European Patent Application No. 81109631.2 discloses *inter alia* compounds of the formula (I):

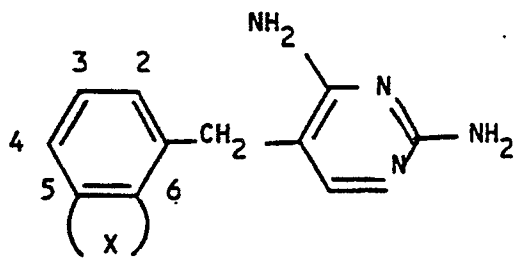

(I)

or a salt, N-oxide or acyl derivative thereof, wherein ($\bar{X}$) is a six membered ring containing a nitrogen atom, both the phenyl ring and the ($\bar{X}$) being optionally substituted except that when ($\bar{X}$) does not contain a hetero atom either or both the phenyl ring or ($\bar{X}$) must be substituted other than solely by a hydroxy group at the 4-position of the phenyl ring, and that there are no substituents attached to the atom of ($\bar{X}$) adjacent to the 6-position of the phenyl ring. It has now been found that a further group of novel 2,4-diamino-5-(substituted)-pyrimidines has advantageous properties for the treatment of microbial infections.

Accordingly, the present invention provides a compound of the formula (II):

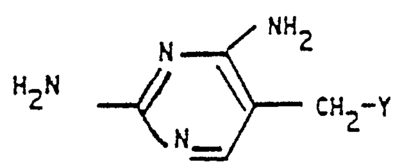

(II)

or a salt, N-oxide or acyl derivative thereof,
wherein Y is a group:

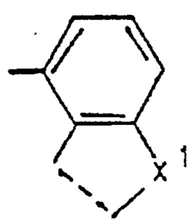

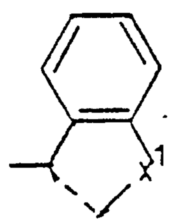

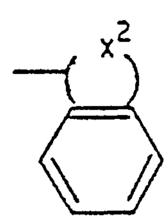

or

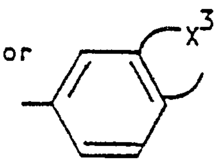

which is optionally substituted;

$X^1$ is an oxygen or sulphur atom, a group $CH_2$, a group S(O)n where n = 1 or 2, a group $NR^1$ wherein $R^1$ is hydrogen or $C_{1-4}$ alkyl; $(X^2)$ is a six-membered ring containing a nitrogen atom; $(X^3)$ is a six-membered ring optionally containing a nitrogen, and the dotted lines represent single or double bonds.

Substitution may occur on either or both of the rings forming the bicyclic ring system.

Substituents for the group Y are halogen, particularly chlorine or bromine, $C_{2-4}$ alkenyl, $C_{1-4}$ alkylthio, amino, mono-$C_{1-4}$ alkyl substituted amino, di-$C_{1-4}$ alkyl substituted amino, morpholino, piperidino, pyrrolidino, piperazino, hydroxy, nitro, $C_{1-4}$ alkoxycarbonyl, or $C_{1-4}$ alkyl or $C_{1-4}$ alkoxy each optionally substituted by halogen, particularly chlorine or bromine, hydroxy or $C_{1-3}$ alkoxy.

One preferred group of compounds of the present invention is that of the formula (III):

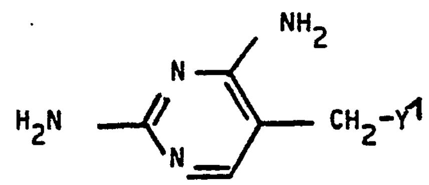

wherein $Y^1$ is a group

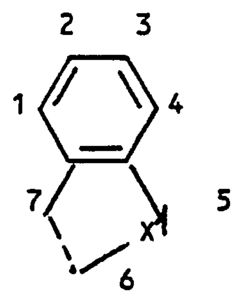

which is linked to the pyrimidinylmethyl moiety at the 1 or 7 position and is optionally substituted at the 2,3,4 or 6 positions or at the 7 position when the linkage to the pyrimidinyl moiety is at the 1 position, wherein $X^1$ and the dotted line are as hereinbefore defined.

Suitable substituents are as hereinbefore defined and these are preferably at one or more of the 3,4 and 6 positions.

A preferred group of compounds of the formula (III) is that wherein $Y^1$ is a group:

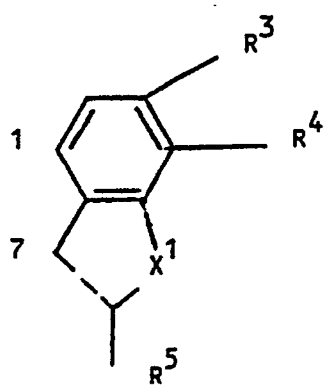

or a salt, N-oxide or acyl derivative thereof; which group is linked to the pyrimidinylmethyl moiety at the 1 or 7 position; $X^1$ is oxygen, sulphur or a group $NR^1$ or $S(0)_n$ as hereinbefore defined in claim 1; and $R^3$, $R^4$ and $R^5$ are the same or different and are selected from hydrogen, halogen atoms, $C_{2-4}$ alkenyl, $C_{1-4}$ alkylthio, amino, mono- $C_{1-4}$ alkylamino, di-$C_{1-4}$ alkyl substituted amino, morpholino, piperidino, pyrrolidino, piperazino, hydroxy, nitro, $C_{1-4}$ alkoxycarbonyl or $C_{1-4}$alkyl or $C_{1-4}$ alkoxy, each optionally substituted by halogen, hydroxy or $C_{1-3}$ alkoxy, and the dotted line is as hereinbefore defined.

Suitably $R^5$ is not halogen, hydroxy or alkoxy when $X^1$ is oxygen, sulphur or a group $NR^1$.

Most suitably $X^1$ is oxygen, sulphur or a group $NR^1$, and preferably $X^1$ is oxygen or sulphur. Suitably $R^3$ and $R^4$ are the same or different and each is hydrogen, methyl, methoxy, amino, dimethylamino, methylthio, bromo or chloro and most suitably $R^3$ and $R^4$ are the same or different and each is hydrogen,

methoxy or dimethylamino. It is preferred that $R^3$ and $R^4$ are not both hydrogen.

Suitably $R^5$ is hydrogen or methyl. Suitably $R^1$ is methyl. Suitably the dotted line represents a double bond.

One preferred group of compounds of the present invention is that of the formula (IV):

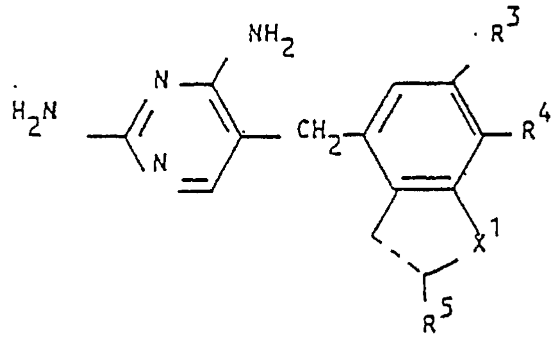

(IV)

or a salt, N-oxide or acyl derivative thereof, wherein $X^1$, $R^3$ to $R^5$ and the dotted line are as hereinbefore defined.

A further preferred group of compounds of the present invention is that of the formula (VI):

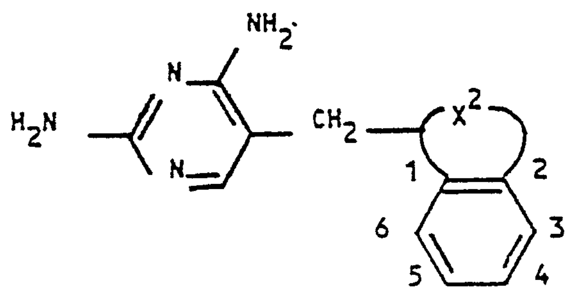

(VI)

or a salt, N-oxide or acyl derivative thereof, wherein ($\bar{x}_2$ ) is a six-membered ring containing a nitrogen atom, both the phenyl ring and the ($\bar{x}_2$ ) ring being optionally substituted other than at the 6-position of the phenyl ring.

The ($\bar{x}_2$ ) ring may contain one, two or three double bonds. It will be apparent that when ($\bar{x}_2$ ) contains three double bonds the nitrogen atom cannot be substituted. However, when ($\bar{x}_2$ ) contains one or two double bonds the nitrogen atom may be optionally substituted.

Substitution of the phenyl ring and the ($\bar{x}_2$ ) ring is preferably with suitable substituents as defined above.

A preferred group of compounds is that of the formula (VII)

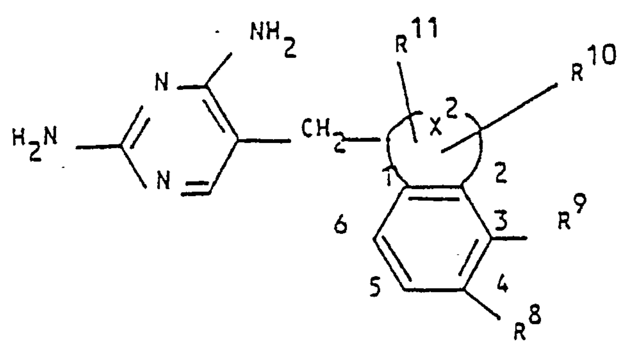

(VII)

or a salt, N-oxide or acyl derivative thereof, wherein ($\bar{x}_2$ ) is a six-membered ring containing three double bonds in which case $X^2$ is -N=, two double bonds in which case $X^2$ is -N=, or $NR^1$-, or one double bond in which case $X^2$ is $-NR^{12}$, wherein $R^{12}$ is a group $R^1$ as hereinbefore defined or is a bond to the 5-methylene bridge to the pyrimidine ring;

$R^8$ and $R^9$ are the same or different and each is hydrogen, halogen, $C_{2-4}$ alkenyl, nitro, hydroxy, a

group $SR^6$ wherein $R^6$ is $C_{1-3}$ alkyl a group $-COR^7$wherein $R^7$ is methoxy or ethoxy, or each is amino optionally substituted by one or two $C_{1-4}$ alkyl groups; morpholino, piperidino, pyrrolidino or $C_{1-4}$ alkyl or $C_{1-4}$ alkoxy each optionally substituted by halogen, hydroxy, or $C_{1-2}$ alkoxy; $R^{10}$ and $R^{11}$ are the same or different and each is defined with respect to $R^8$ and $R^9$, or $R^{10}$ and $R^{11}$ are linked to the same carbon atom to form a group - C = O, -C = S or

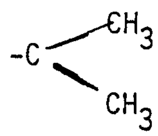

It will be readily apparent that $R^{10}$ and $R^{11}$ will not be =O, =S, or gem dimethyl when ($\bar{\bar{x}}_2$ ) is an aromatic ring. Preferred values for halogen are chlorine and bromine.

Most suitably $R^8$, $R^9$, $R^{10}$ and $R^{11}$ are the same or different and each is hydrogen, hydroxy, methoxy, ethoxy, methoxyethoxy, methyl, ethyl, propyl, amino, methylamino, dimethylamino, ethylamino, diethylamino, vinyl, allyl, propenyl, halogen, methylthio, ethylthio, pyrrolyl. Preferably $R^8$, $R^9$, $R^{10}$ and $R^{11}$ are hydrogen, methyl, methoxy or ethoxy, amino, mono- or dimethylamino or methylthio. Most preferably $R^8$ and $R^9$ are hydrogen. Preferably the ($\bar{\bar{x}}_2$ ) ring contains three double bonds.

Preferably the 5-methylene bridge to the pyrimidine ring is joined to the bicyclic ring system at the position in the heterocyclic ring ∝ or $\beta$ to the 1-position of the phenyl ring. A preferred group of compounds of the formula (VI) wherein the 5-methylene bridge is joined $\beta$ to the l-position of the phenyl ring is that of the formula (VIII):

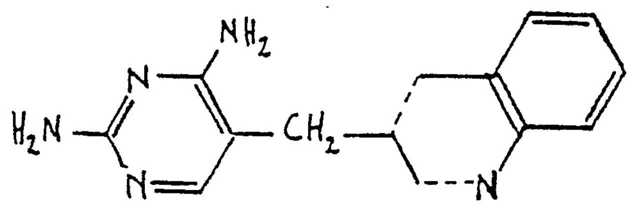

(VIII)

or a salt, N-oxide or acyl derivative thereof wherein the dotted lines represent single or double bonds. Suitably there are one to three substituents, as hereinbefore defined, on the phenyl ring. Preferred substituents include $C_{1-4}$ alkyl or $C_{1-4}$alkoxy each optionally substituted by halogen, hydroxy or $C_{1-2}$ alkoxy; halogen, $C_{1-4}$ alkylthio, $C_{1-4}$ alkoxy carbonyl, amino, mono ( $C_{1-4}$ alkyl) substituted amino, di-( $C_{1-4}$ alkyl) substituted amino, morpholino, piperidino, pyrrolidino or piperazino.

A further preferred group of compounds of the present invention is that of the formula (IX):

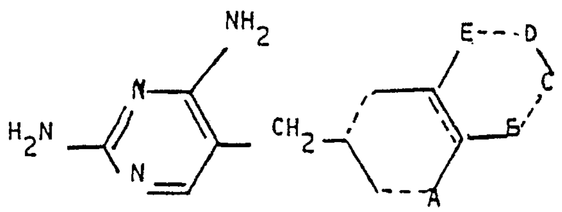

(IX)

or a salt, N-oxide or acyl derivative thereof optionally containing a nitrogen atom at one of positions A, B, C, D or E, in which the dotted lines represent double bonds unless one of the rings contains a nitrogen atom in which case the dotted lines in this ring represents single or double bonds. Suitable substituents are as hereinbefore defined. Preferred substituents include halogen, $C_{1-4}$alkyl, $C_{2-4}$ alkenyl, $C_{1-4}$ alkoxy, amino, mono- or di- $C_{1-4}$ alkyl substituted amino, morpholino, piperidino, pyrrolidono, piperazino $C_{1-4}$ alkoxycarbonyl, $C_{1-4}$ alkylthio or $C_{1-4}$ alkyl or $C_{1-4}$ alkoxy substituted by halogen, hydroxy or $C_{1-3}$ alkoxy groups.

A preferred group of compounds of the formula (IX) is that of the formula (X):

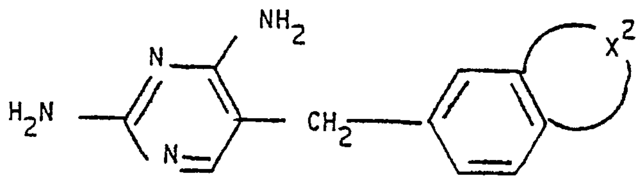

(X)

or a salt, N-oxide or acyl derivative thereof, wherein ($\bar{\bar{x}}_2$ ) is a six-membered ring containing a nitrogen atom. Suitably there are one to three substituents selected from those hereinbefore defined. The substituents are preferably attached to a carbon atom of the ($\bar{\bar{x}}_2$ ) ring, or to the nitrogen atom in the B position when that ring is reduced or to a carbon atom of the phenyl.

The substituents are selected from methoxy, ethoxy, methyl, ethyl, amino, dimethylamino, pyrrolyl, morpholino, methoxyethoxy, chlorine, bromine, methoxycarbonyl or ethoxycarbonyl.

When the nitrogen atom is adjacent to the phenyl ring, ($\bar{\bar{x}}_2$ ) is suitably a partially saturated or an aromatic ring. When the nitrogen atom is at either of the other two possible positions, ($\bar{\bar{x}}_2$ ) is suitably an aromatic ring.

Particularly preferred compounds are those of the formula (XI):

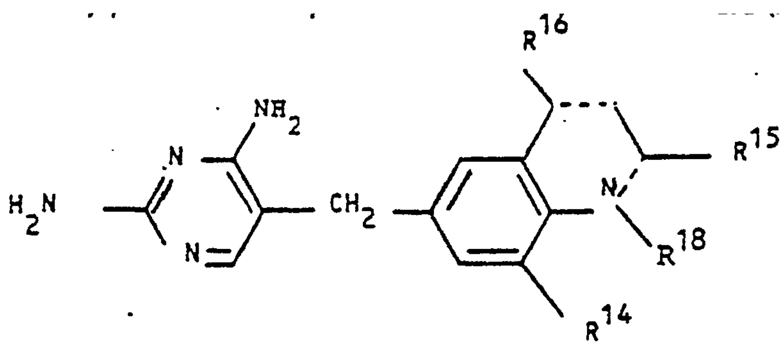

(XI)

or a salt, N-oxide or acyl derivative thereof
wherein the dotted lines represent single or double bonds,$R^{14}$,$R^{15}$ and $R^{16}$ are the same or different and each represents hydrogen or one of the particularly preferred substituents defined in relation to formula (X) and when the dotted lines represent single bonds the nitrogen atom has a hydrogen atom or a $C_{1-4}$alkyl group attached. Suitably one and preferably two of $R^{14}$,$R^{15}$,$R^{16}$ and $R^{18}$ is other than hydrogen.

The compounds of the formula (II) are bases and, as such, form acid addition salts with acids. Suitable acid addition salts of the compounds of the formula include those formed with both organic and inorganic acids. Such acid addition salts will normally be pharmaceutically acceptable. Thus, preferred salts include those formed from hydrochloric, sulphuric, citric, tartaric, phosphoric, lactic, benzoic, glutamic, aspartic, pyruvic, acetic, succinic, fumaric, maleic, oxaloacetic, isethionic, stearic, methanesulphonic, p-toluenesulphonic, lactobionic and glucuronic acids.

Suitable acyl derivatives are those wherein an amino group is substituted by a group -COM wherein M is hydrogen or $C_{1-11}$ alkyl or $C_{2-11}$ alkenyl, preferably $C_{1-4}$ alkyl or $C_{2-4}$ alkenyl, optionally substituted by carboxy, carb-$C_{1-4}$ alkoxy, nitrile, amino, chlorine or phenoxy optionally substituted by halogen, methyl or methoxy, the alkyl or alkenyl groups being optionally interspersed with one or more oxygen atoms or forming part or the whole of a cycloaliphatic ring or M may represent a $C_{6-10}$ aromatic or $C_{6-10}$ araliphatic residue optionally substituted by one or more chlorine atoms or methyl, $OCH_2COOH$, carb-$C_{1-4}$alkoxy or a heterocyclic group containing one or more nitrogen, oxygen or sulphur atoms.

Certain compounds of the formula (II) whilst having some antibacterial activity in their own right are also useful as intermediates in the preparation of other compounds of the formula (II) having interesting antibacterial activity. Preferred acyl derivatives are those wherein the amino group at the 2-position of the pyrimidine ring is substituted, particularly those wherein the amino group is substituted by acetyl or by an acyl group derived from an amino acid such as glycyl.

Suitable N-oxides of compounds of the formula (II) include those formed by oxidation of either or both of the nitrogen atoms in the pyrimidine ring or by oxidation of $X^1$ when this is nitrogen or the nitrogen atom in the

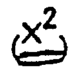

($\bar{X}_2$) ring when this is present.

The preparation of salts, acyl derivatives and N-oxides is carried out by conventional methods well known to those skilled in the art.

Pharmaceutically acceptable acid addition salts of compounds of the formula (II) form a particularly preferred aspect of the present invention.

In a further aspect, the present invention provides a pharmaceutical composition comprising a compound of the formula (II) in combination with a pharmaceutically acceptable carrier. By the terms "pharmaceutical composition" and "pharmaceutically acceptable carrier" are meant those compositions and carriers suitable for use in human and/or veterinary medicine.

The compounds of the formula (II) can conveniently be presented in the compositions of the present invention in an effective unit dosage form, that is to say in an amount sufficient to be effective against the bacterial organism in vivo .

The pharmaceutically acceptable carriers present in the compositions of the present invention are materials recommended for the purpose of administering the medicament. These may be liquid, solid or gaseous materials, which are otherwise inert or medically acceptable and are compatible with the active ingredient.

These pharmaceutical compositions may be given parenterally, orally, used as a suppository, applied as an ophthalmic solution, or applied topically as an ointment, cream or powder. However, oral and parenteral administration of the compositions is preferred for human use. For veterinary use, intramammary as well as oral and parenteral administration is preferred.

For oral administration, fine powders or granules will contain diluting, dispersing and/or surface active agents, and may be presented in a draught, in water or in a syrup, in capsules or cachets in the dry state or in a non-aqueous suspension wherein suspending agents may be included, or in a suspension in water or syrup. Where desirable or necessary, flavouring, preserving, suspending, thickening or emulsifying agents can be included.

For parenteral administration, the compounds may be presented in sterile aqueous injection solutions which may contain antioxidants or buffers.

As stated above, the free base or a salt thereof may be administered in its pure form unassociated with other additives in which case a capsule or cachet is the preferred carrier.

Other compounds which may be included are, for example, medically inert ingredients, e.g. solid and liquid diluents such as lactose, glucose, starch or calcium phosphate for tablets or capsules; olive oil or ethyl oleate for soft capsules; and water or vegetable oil for suspensions or emulsions; lubricating agents such as talc or magnesium stearate; gelling agents such as colloidal clays; thickening agents such as gum tragacanth or sodium alginate; and other therapeutically acceptable accessory ingredients such as humectants, preservatives, buffers, and antioxidants which are useful as carriers in such formulations.

Alternatively the active compound may be presented in a pure form as an effective unit dosage, for instance, compressed as a tablet or the like.

For veterinary use, different intramammary formulations will normally be prepared for use in dry cows and for use in milking cows. Thus, formulations for dry cow use will normally be in an oil, such as peanut oil, gelled with a gelling agent such as aluminium monostearate. Formulations for milking cow use will usually contain an emulsifying agent (for example Tween 20 or a polysorbate) and a milk miscible carrier such as peanut oil or a mineral oil.

It may be advantageous to include the compounds of formula (II) in a pharmaceutical composition which includes other active ingredients for example p-aminobenzoic acid competitors such as sulphonamides.

Of known p-aminobenzoic acid competitors, the following sulphonamide compounds (or pharmaceutically acceptable salts thereof) are particularly useful:

Sulfanilamide, Sulfadiazine, Sulfamethisazole, Sulfapyridine, Sulfathiazole, Sulfamerazine, Sulfamethazine, Sulfisoxazole, Sulformethoxine, 2-( p -Aminobenzene-sulfonamide-3-methoxypyrazine (Kelfizina), Mafenide, 5-Sulfanilamido-2,4-dimethyl pyrimidine, 4-($N^1$-Acetylsulfanilamido)-5,6-dimethoxypyrimidine, 3-Sulfanilamido-4,5-dimethylisoxazole, 4-Sulfanilamido-5-methoxy-6-decyloxypyrimidine sulfamono-methoxine, 4-p -(8-Hydroxyquinolinyl-4-azo)-phenylsulfanilamido-5,6-dimethoxy-pyrimidine, Sulfadimethoxine, Sulfadimidine, Sulfamethoxazole, Sulfamoxole, Sulfadoxine, Sulfaguanidine, Sulfathiodimethoxine, Sulfaquinoxaline, and p -(2-Methyl-8-hydroxyquinolinyl-5-azo)phenylsulfanilamido-5,6-dimethoxy-pyrimidine.

However, the most preferred combinations include those containing Sulfadiazine, Sulfamethoxazole,

Sulfadoxine, Sulfamoxole or Sulfadimidine. The ratio of the compound of the formula (II) to sulphonamide will normally be from 3:1 to 1:10, for example 1:1 to 1:5. A particularly preferred composition of the present invention comprises a compound of formula (II) and a sulphonamide in a ratio of 1:2 to 1:5 preferably together with a pharmaceutically acceptable carrier.

Tablets or other forms of presentation provided in discrete units may conveniently contain an amount of compound of the formula (II) which is effective at a dosage or as a multiple of the same, for instance for human use, units containing 2.5 to 200 mg usually around 30 to 100 mg and for veterinary use, units containing 30 to 500 mg.

The pharmaceutical compositions of the present invention can be prepared by the admixture of a compound of the formula (II) with a pharmaceutically acceptable carrier. Other active ingredients, such as a sulfonamide, or conventional pharmaceutical excipients may be admixed as required.

The compounds of the present invention are useful for the treatment of microbial infections and, in particular, gram negative aerobic, gram positive aerobic or anaerobic bacterial infections in mammals. They are particularly useful in the treatment of Staphylococcal infections for example mastitis in cattle, Neisseria infections in humans, for example N. gonorrhea, acne in humans, and anaerobic infections. Most compounds also have an excellent level of general antibacterial activity.

Still another aspect of the present invention provides a method for the treatment or prophylaxis of bacterial infections in mammals by the administration of an effective non-toxic antibacterial amount of a compound of formula (II) or a pharmaceutically acceptable salt thereof, or a composition as hereinbefore described.

As indicated above, the compounds of the formula (II) are generally useful in treating bacterial infections by rectal, parenteral, topical or oral administration. The compounds of formula (II) are normally administered at a dose from 0.1 mg/kg to 30 mg/kg per day and preferably 1 mg/kg to 10 mg/kg. The dose range for adult humans is generally from 25 to 300 mg/day and preferably 100 to 200 mg/day.

The dose range for intramammary administration of the compounds of the formula (II) is generally from 100 to 500 mg, preferably 200 mg to 400 mg, per quarter of the udder to dry cows. Milking cows will normally receive four to six medications of a composition of the present invention, a dose being conveniently administered at milking time (i.e. twice daily) to each of the desired quarters of the udder. Dry cows will normally receive only one medication of a composition of the present invention, one dose being provided to each of the four quarters of the udder.

The compounds of formula (II) and their pharmaceutically acceptable salts may be prepared by methods known for the synthesis of compounds of analogous structure.

Thus the present invention provides a process for preparation of compounds of the formula (II) as hereinbefore defined which process comprises:

(a)(i) the reaction of a guanidine salt with a compound of the formula (A) or (B):

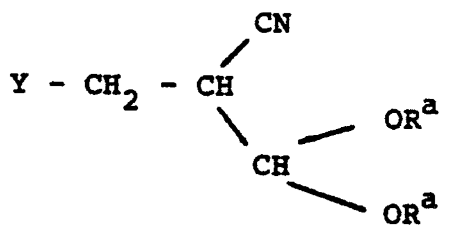

(A)

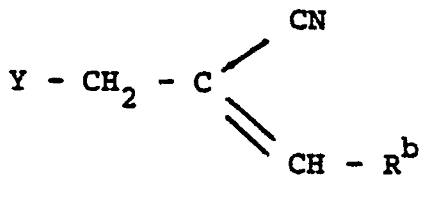

(B)

Wherein Y is as hereinbefore defined, $R^a$ is a $C_{1-4}$ alkyl group and $R^b$ is a nucleophilic leaving group such as a $C_{1-4}$ alkoxy group, for example a methoxy, ethoxy or methoxyethoxy group, or an amino, $C_{1-4}$ alkylamino, benzyl-amino, di-$C_{1-4}$ alkylamino, naphthylamino, optionally substituted anilino, morpholino,

piperidino or N-methylpiperazino group and most preferably $R^b$ is an anilino group:

(ii) the reaction of a compound of formula (C):

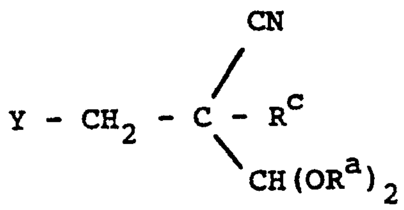

(C)

wherein Y and $R^a$ are as hereinbefore defined and $R^c$ is an alkoxycarbonyl or aldehyde group, with potassium or sodium hydroxide in a $C_{1-4}$ alkanol followed by addition of guanidine;

(iii) the reaction of a compound of the formula (D):

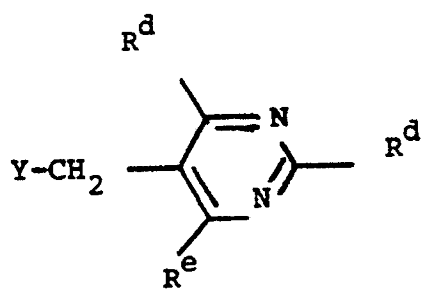

(D)

wherein $R^d$ is an amino group or a leaving group, such as a $C_{1-4}$ alkylthio group or a halogen atom, $R^e$ is a hydrogen or halogen atom, except that both groups $R^d$ cannot be amino groups and Y is as hereinbefore defined with an aminating agent such as ammonia and thereafter when $R^e$ is a halogen atom removing this by hydrogenolysis:

(iv) the reaction of a compound of the formula (E)

$$Y - CH_2Z \qquad (E)$$

wherein Z is a halogen atom or hydroxy or di-$C_{1-4}$ alkyl substituted amino or other leaving group; and Y is as hereinbefore defined, with a compound of the formula (F):

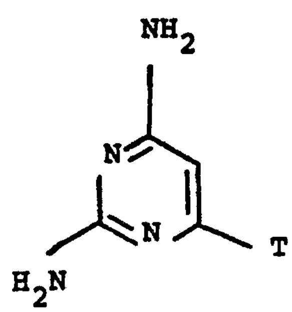

(F)

wherein T is hydrogen or a hydroxy or $C_{1-4}$ alkylthio group, and then converting the group T to hydrogen by hydrogenolysis when T is a $C_{1-4}$ alkylthio group or, when T is a hydroxy group, by first converting it to

the mesylate or tosylate derivative or to thio, alkylthio or halogen and then removing this by hydrogenolysis;

(b) when it is required to prepare a compound of the formula (IV) wherein $R^4$ is other than hydrogen, the cyclisation of a compound of the formula (G)

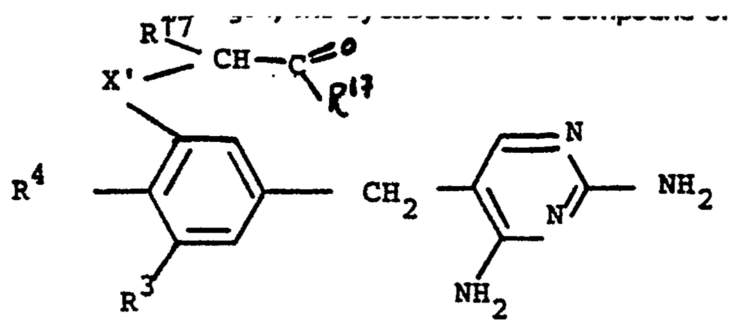

(G)

wherein $X^1$, $R^3$ and $R^4$ are as hereinbefore defined except that $R^4$ is not hydrogen and the two groups $R^{17}$ are the same or different and each is hydrogen or $C_{1-4}$alkyl;

(c) when it is required to prepare a compound of the formula (IV) wherein the 4-position of the phenyl ring is optionally substituted by hydroxy, alkoxy, amino or substituted amino, or formula (X) wherein ($\bar{\bar{x}}_2$ ) is a partially saturated aromatic ring in which the nitrogen atom is adjacent to the phenyl ring the reaction of a compound of the formula (H):

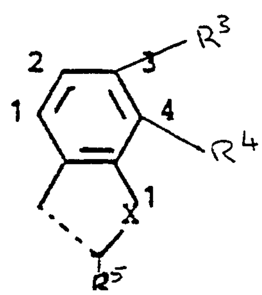

(H)

wherein the 4- position of the phenyl ring is optionally substituted by hydroxy, alkoxy, amino, substituted amino and the ($\bar{\bar{x}}_1$ ) ring and the phenyl ring are substituted by other substituents as hereinbefore defined, or a compound of the formula (L):

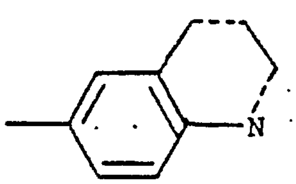

(L)

wherein at least one of the dotted lines is a single bond and, when the dotted line to the nitrogen is a single bond, the nitrogen is substituted with hydrogen or an alkyl group, with 2,4-diamino-5-hydroxy-methylpyrimidine or an ether thereof;

(d) the conversion of one compound of the formula (II) to a different compound of the formula (II) for example by the reduction or isomerization of one or two of the double bonds, conversion of a hydroxy group to a $C_{1-4}$ alkylthio group or an optionally substituted $C_{1-4}$ alkoxy group or conversion of an amino group to a $C_{1-4}$ alkylthio group or hydrogen, halogen or hydroxy via a diazo group or to a substituted amino group by methods well known to those skilled in the art.

e) when it is required to prepare a compound of formula (IX) wherein a nitrogen atom is at position B and the dotted lines represent double bonds, the condensation/cyclization of a compound of the formula (M):

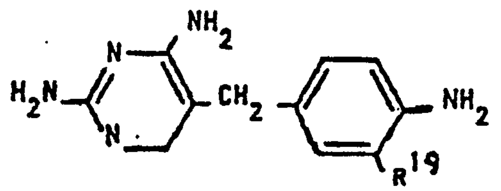

(M)

wherein $R^{19}$ is halogen, $C_{1-4}$ alkyl, $C_{1-4}$ alkoxy or $C_{1-4}$ alkylthio with a compound of the formula (N):

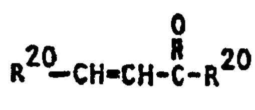

(N)

wherein the groups $R^{20}$ are the same or different and each is hydrogen or $C_{1-4}$ alkyl, under acidic conditions such as dilute ethanolic hydrochloric acid.

f) When it is required to prepare a compound of Formula (IX) wherein a nitrogen atom is at position A,B,C,D or E and all of the dotted lines represent double bonds, the oxidation of a compound of Formula (IX) wherein not all of the dotted lines represent double bonds under suitable dehydrogenation conditions, such as elevated temperatures in the presence of a dehydrogenation catalyst such as platinum oxide.

The reaction of guanidine with a compound of the formula (A) or (B) will take place under conditions analogous to those described in U.K. Patent Nos. 1 133 766 and 1 261 455 respectively for the preparation of structurally related benzyl-pyrimidines. Conveniently the reaction is carried out in a $C_{1-4}$ alkanol, for example methanol or ethanol. The compounds of the formula (A) or (B) may be prepared by methods known in the art. It is preferred to avoid the use of aprotic solvents in the preparation of compounds of the formula (A) when $X^1$ is oxygen.

The reaction of a compound of the formula (C) with guanidine and the preparation of the compounds of the formula (C) will be carried out by methods analogous to those described in Belgian Patent No. 855 505.

In the compounds of the formula (D) when $R^d$ or $R^e$ are halogen atoms, these are suitably chlorine or bromine atoms. The reaction may conveniently be carried out under the reaction conditions described in U.K. Patent Nos. 875 562 and 1 132 082. The reduction of $R^e$ when this is a halogen will suitably be carried out under the conditions described in German Offenlegungschrift 2,258,238. This is not a preferred method for preparating those compounds wherein Y contains groups that are susceptible to catalytic hydrogenation.

The compounds of formula (D) may be prepared by methods known in the art, for example as described in U.K. Patents No. 875,562 and 1,132,082 or German Offenlegungschrift 2,258,238. The compounds of the formula (D) wherein $R^d$ and/or $R^e$ are halogen atoms may conveniently be prepared from the corresponding compounds wherein $R^d$ and/or $R^e$ are hydroxy. These compounds may be prepared by methods analogous to those described in the art.

Suitably Z is a dialkylamino or cyclic amino group containing up to 10 carbon atoms; a dimethylamino group is particularly convenient. The reaction will be carried out under conditions well known to those skilled in the art of Mannich reactions. It has been found that the reaction may suitably be carried out at an elevated temperature, suitably between 100° and 200°C in a solvent having a suitably high boiling point, for example a glycol such as ethylene glycol. The dethiation is suitably carried out by hydrogenolysis in the presence of a transition metal catalyst; Raney nickel is particularly suitable for this purpose. This reaction will normally be carried out in a polar solvent, for example a $C_{1-4}$ alkanol such as methanol or ethanol.

Again, this is not a preferred method of preparing those compounds of the formula (II) wherein there are groups that are susceptible to a catalytic hydrogenation.

The compounds of formula (G) wherein $X^1$ is sulphur or oxygen may be prepared by the following reaction scheme:

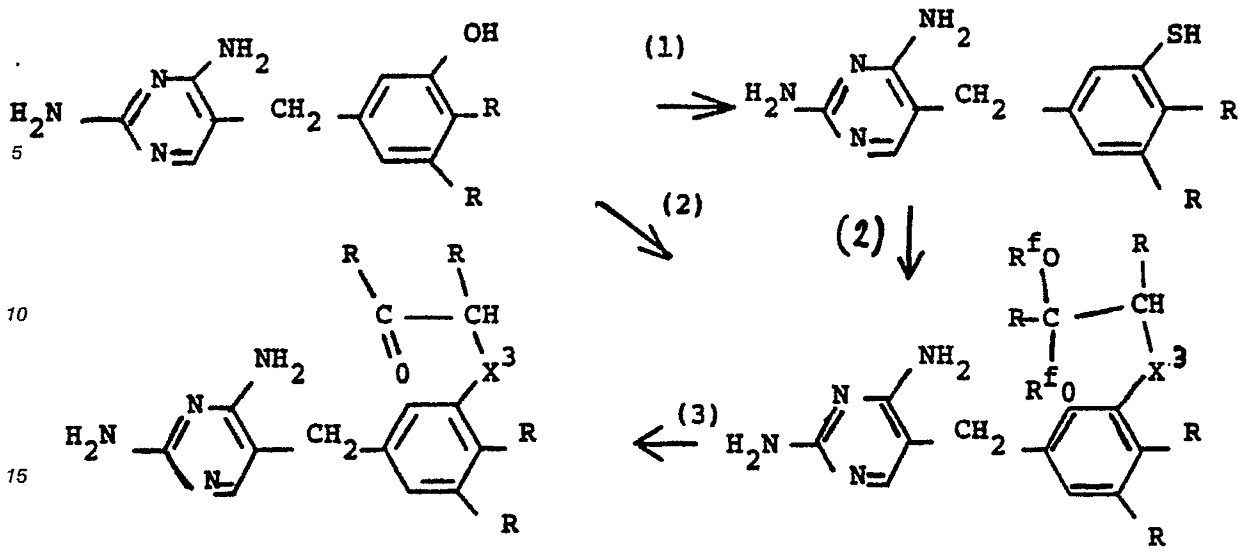


wherein $X^3$ is oxygen or sulphur and $R^f$ is $C_{1-4}$ alkyl.

1) reaction with 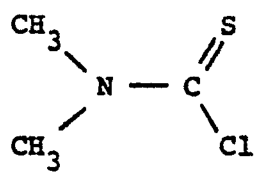

2) reaction with 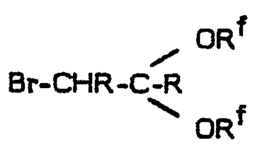

3) acid

The cyclisation of a compound of the formula (G) takes place under conventional conditions, for example those described in "The Chemistry of Heterocyclic Compounds", Wiley-Interscience, John Wiley and Sons, Inc., N.Y.; "Heterocyclic Compounds", vol 2 R.C.Elderfield, ed., John Wiley and Sons, Inc., N.Y. p.11 ff, p.146 ff (1951); "Advances in Heterocyclic Chemistry", vol 1, A.R.Katritsky and A.J.Boulton, ed., Academic Press, N.Y. p.217 ff (1970) and vol 18, A.J. Katritsky and A.J.Boulton, ed., Academic Press, N.Y., p. 362 ff (1975).

The preparation of a compound of the formula (G) from the corresponding acetal and its conversion to the corresponding compound of formula (IV) conveniently takes place in situ.

The reaction of a compound of the formula (H) with 2,4-diamino-5-hydroxymethyl-pyrimidine is normally carried out under the reaction conditions described for analogous reactions in U.K. Patent No. 1,413,471. Thus, the reacticn is conveniently carried out in a polar solvent capable of dissolving both reactants at a non-extreme temperature, for example between 50° and 150°C. The reaction is preferably carried out in the presence of a strong acid catalyst, such as hydrochloric, acetic, methanesulphonic or p-toluenesulphonic acids.

In the case where there is an alkoxy group at the 4-position, it may be necessary to separate the desired compound of the formula (IV) or (X) from other substances present in the reaction mixture by conventional methods, for example by chromatography or crystallisation.

It will be apparent to those skilled in the art that when certain ring substituents are present in the final compounds of the formula (II) or when an unsaturated ring system is present certain methods of preparation will preferably not be used to make these compounds due to the possibility of the reaction conditions changing the final product group, for example hydrogenolysis or addition across the double bond when a

double bond is present.

Certain compounds of the formula (II) whilst having some antibacterial activity in their own right are useful also as intermediates in the preparation of other compounds of the formula (II) having interesting antibacterial activity.

The intermediates of the formulae (A) to (E) and (G) which are novel form a further aspect of the present invention.

In yet another aspect, the present invention provides the first use of the compounds of the formula (II) in human and veterinary medicine. The preferred human use of the compounds of the formula (II) is in the treatment or prophylaxis of bacterial infections.

The following Examples illustrate the preparation of the compounds of the present invention and their pharmacological properties. All temperatures are in degrees Centigrade.

Example 1

2,4-Diamino-5-(1,2,3,4-tetrahydro-6-quinolylmethyl)pyrimidine dihydrochloride

A mixture of 1,2,3,4-tetrahydroquinoline (2.66 g, 0.02 mol), 2,4-diamino-5-hydroxy-methylpyrimidine (2.80 g, 0.02 mol), glacial acetic acid (35 ml) and concentrated hydrochloric acid (3.45ml) was heated under reflux for 3.5 hours. The solution was filtered from a small precipitate, and the solvent was removed. The residue was dissolved in water and made basic with ammonium hydroxide, which resulted in the precipitation of a gummy solid. This was extracted into 3:1 methylene chloride:methanol several times. The extract was evaporated leaving a residue (3.45g) which was dissolved in a mixture of 9:1 methylene chloride: methanol and put through a short silica gel column. There was isolated 2,4-diamino-5-(1,2,3, 4-tetrahydro-6-quinolylmethyl)pyrimidine (2.85g), which was converted into the dihydrochloride with ethanol-HCl, m.p. 284-287° C. Anal. Calcd. for $C_{14}H_{17}N_5$.2HCl: C, 51.23; H, 5.83; N, 21.34; Cl, 21.60. Found: C, 51.22; H, 5.86; N, 21.32; Cl, 21.54.

Example 2

2,4-Diamino-5-(1,2,3,4-tetrahydro-8-methoxy-6-quinolylmethyl)pyrimidine

8-Methoxy-1,2,3,4-tetrahydroquinoline (J.L.Neumeyer and J.G.Cannon, J.Pharm. Sci., 51 , 804(1962))-(2.88g) was treated by the method of Example 1 with 2,4-diamino-5-hydroxymethylpyrimidine. The product crystallised from the reaction mixture as an off-white solid, which was washed with water and treated with ammonia, followed by recrystallisation from absolute ethanol(5.0g). A slight impurity was removed on a silica gel column, which was eluted with 19:1 methylene chloride:methanol, taken to dryness, and recrystallised once more from absolute ethanol; m.p., 201-203° C. Anal. Calcd. for $C_{15}H_{19}N_5O$: C, 63.14; H, 6.71; N, 24.54. Found: C, 63.23; H, 6.75; N, 24.47.

Example 3

2,4-Diamino-5-(1,2,3,4-tetrahydro-8-(2-methoxyethoxy)-6-quinolylmethyl)pyrimidine

A. 8-Methoxyethoxyquinoline

To 8-hydroxyquinoline (9.47g, 0.065 mol) in dimethyl sulfoxide (50 ml) was added 2-methoxyethyl bromide (8.96 g, 0.065 mol). The mixture was stirred at room temperature for two hours, and turned a dark red. The solvent was removed under vacuum, and the residue dissolved in water. The aqueous solution was extracted several times with ethyl acetate, and the ethyl acetate solution was then washed well with water, dried, and the solvent removed; the residual oil weighed 7.45g. This was purified on a silica gel column, eluted with heptane:ethyl acetate, with increasing proportions of the latter. There was isolated 8-methoxyethoxyquinoline (7.11g) as a light blue oil. NMR: ($CDCl_3$) $\delta$ 3.51 (s, 3, OMe), 4.01 (tr, 2, $CH_2$), 4.48 (tr, 2, $CH_2$), 7.20 (m, 1, beta-pyridine-H), 7.45 (m, 3, Ar), 8.15 (dd, 1, gamma-pyridine-H), 9.98 (dd, 1, alpha-pyridine-H). Anal . Calcd. for $C_{12}H_{13}NO_2$: C, 70.92; H, 6.45; N, 6.89. Found: C, 70.80; H, 6.49; N, 6.90.

B. 1,2,3,4-Tetrahydro-8-methoxyethoxyquinoline

8-Methoxyethoxyquinoline (6.48g, 31.9 mmol) was dissolved in methanol (50 ml) and reduced on a Parr hydrogenation apparatus using $PtO_2$ catalyst. The catalyst was removed, and the solution was taken to dryness. The dark brown oil which remained was purified on a short silica gel column using 4:1 heptane: ethyl acetate for elution. The isolated oil (1,2,3,4-tetrahydro-8- methoxyethoxyquinoline) had the following NMR spectrum: ($CDCl_3$) $\delta$ 1.93 (quintet, 2, $CH_2$ (beta-H)), 2.76 (tr, 2, $CH_2$), 3.32 (tr, 2, $CH_2$), 3.43 (s, 3, OMe), 3.72 (tr, 2, $OCH_2$), 4.11 (tr, 2, $CH_2O$), 4.31 (br, 1, NH), 6.57 (s +sh, 3, Ar-3H). Anal . Calcd. for $C_{12}H_{17}NO_2$: C, 69.54; H, 8.27; N, 6.76. Found: C, 69.42; H, 8.26, N, 6.70.

C. 2-4-Diamino-5-(1,2,3,4-tetrahydro-8-(2-methoxyethoxy)-6-quinolylmethyl)-pyrimidine

The procedure of Example 1 was used to react 1,2,3,4-tetrahydro-8-methoxyethoxy-quinoline (3.11g) with 2,4-diamino-5-hydroxymethylpyrimidine, and 2,4-diamino-5-(1,2,3,4-tetrahydro-8-(2-methoxyethoxy)-6-quinolylmethyl)-pyrimidine was isolated and purified as in this example. The free base was recrystallised from absolute ethanol; m.p. 149-151 °C. NMR($Me_2SO-d_6$)$\delta$1.76(m,2,$CH_2$), 2.61 (tr, 2, $CH_2$),3.22(m,2,$CH_2$)-,3.31(s,3,OMe),3.39(s,2,bridge $CH_2$), 3.65 (m,2,$CH_2CH_2O$),3.97(m,2,O $CH_2CH_2$),4.60(br.s.,1, NH),5.60 and 5.90 (2 br s, 4, $(NH_2)_2$), 6.36 (d, 1, J=1-2, Ar), 6.53 (d, 1, J=1-2, Ar), 7.45(s, 1, pyrimidine-6-H). Anal. Calcd. for $C_{17}H_{23}N_5O_2$: C, 61.99; H, 7.04; N, 21.26. Found: C, 61.82; H, 7.06; N, 21.25.

Example 4

2,4-Diamino-5-(1,2,3,4-tetrahydro-4-methyl-6-quinolylmethyl)pyrimidine Dihydrochloride

A. 4-Methyl-1,2,3,4-tetrahydroquinoline

A solution of lepidine (7.16g) in methanol (50 ml)was reduced in a Parr hydrogenation apparatus with a total of 1,25g of $PtO_2$ catalyst, added in 3 portions, at intervals. The reduction was very slow. After 36 hours, the catalyst was removed, and then the solvent was removed; the residual oil proved to be a mixture which still contained considerable lepidine. This was separated on a silica gel column using 10:1 hexane:ethyl acetate for elution. A 0.91g portion was isolated which was 4-methyl-1,2,3,4-tetrahydroquinoline. NMR: ($CDCl_3$) $\delta$ 1.26 (d, 3, CH Me ), 1.5-2.2 (m, 2, $CH_2$), 2.89 (septet, 1, C H Me), 3.26 (tr, 2, $NCH_2$), 3.78 (br s, 1, NH), 6.37-7.2 (m, 4, $ArH_4$). MS:147 ($M^+$). Anal. Calcd. for $C_{10}H_{13}N$: C, 81.58; H, 8.90; N, 9.51. Found: C, 81.54; H, 8.93; N, 9.47.

B. 2,4-Diamino-5-(1,2,3,4-tetrahydro-4-methyl-6-quinolyimethyl)pyrimidine Dihydrochloride

4-Methyl-1,2,3,4-tetrahydroquinoline (0.74g, 0.005 mole) was treated with 2,4-diamino-5-hydroxymethyl-pyrimidine in the manner of example 1, and purified similarly, followed by conversion to the dihydrochloride salt in absolute ethanol-HCl. The dihydrochloride (0.54g) melted at 280-282 °C. NMR: ($Me_2SO-d_6$)$\delta$ 1.26 (d, 3, CH Me , J=7 Hz), 1.70 (m, 2, $CH_2$), 2.90 (m, 1, C H Me), 3.30 (tr, 2, $NCH_2$), 3.69 (s, 2, bridge $CH_2$), ca. 4.0 (v br, 2, $NH_2^+$), 7.13 (s, 2, Ar), 7.33 (s, 1, Ar, J=1-2), 7.54 (s, 1, pyrimidine-6-H), 7.61 (br s, 2, $NH_2$), 7.77 and 8.25 (2 br s, NH, NH), ca 12.0 (v br $NH^+$). Anal. Calcd. for $C_{15}H_{19}N_5HCl$: C, 52.64; H, 6.18; N, 20.46; Cl, 20.72. Found: C, 52.52; H, 6.24; N, 20.36; Cl, 20.60.

Example 5

2,4-Diamino-5-(2-naphthylmethyl)pyrimidine

A. 2-(2-Naphthylmethyl)-3-anilinoacrylonitrile

To a mixture of 2-naphthaldehyde (4.69g, 30 mmol) and beta-anilino propionitrile (4.82g, 33 mmol) in dimethyl sulfoxide (35 ml) was added potassium t-butoxide (3.70g, 33 mmol). The solution immediately turned dark red. It was heated at 100° for 30 minutes, cooled, and diluted with methanol (15 ml) and water

(25 ml). A copious yellow precipitate formed, which was chilled and isolated. The precipitate, 2-(2-naphthylmethyl)3-anilinoacrylonitrile, was washed with dilute methanol and hexane; yield, 7.65g. A portion was recrystallised from absolute ethanol; m.p. 148-153° C. Anal. Calcd. for $C_{20}H_{16}N_2$: C, 84.48; H, 5.67; N, 9.85. Found: C, 84.38; H, 5.69; N, 9.82.

B. 2,4-Diamino-5-(2-naphthylmethyl)pyrimidine

2-(2-Naphthylmethyl)-3-anilinoacrylonitrile (6.65g, 23•4 mmol) was dissolved in absolute ethanol (225 ml)and heated to reflux. A solution of sodium methylate (4.27g, 79 mmol) in ethanol (75 ml) was mixed with guanidine hydrochloride (6.87g, 72 mmol), filtered from salt, and added to the solution of 2-(2-naphthylmethyl)-3-anilinoacrylonitrile. The mixture was then refluxed for 8 hours, and allowed to stand at room temperature overnight. A yellow precipitate formed, which was isolated (2.66g). An additional 1.33g was obtained by concentration of the mother liquor. The combined fractions were recrystallised from absolute ethanol, yielding light cream coloured 2,4-diamino-5-(2-naphthylmethyl)-pyrimidine (3.0g), m.p. 231-233° C. Anal. Calcd for $C_{15}H_{14}N_4$: C, 71.98; H, 5.64; N, 22.38. Found: C, 71.93; H, 5.68; N, 22.35.

Example 6

2,4-Diamino-5-(3-quinolylmethyl)pyrimidine

A. 2-(3-Quinolylmethyl)-3-anilinoacrylonitrile

A mixture of 3-quinolinecarboxaldehyde (5.0g, 31.8 mmol) and anilinopropionitrile (5.12g, 35 mmol) was treated in the manner of Example 5A to give 7.38g (81%) of 2-(3-quinolylmethyl)-3-anilinoacrylonitrile. A sample was recrystallised from methyl cellosolve; m.p. 202-203°. Anal. Calcd. for $C_{19}H_{15}N_3$: C, 79.98; H, 5.30; N, 14.73. Found: C, 79.80; H, 5.24; N, 14.73.

B. 2,4-Diamino-5-(3-quinolylmethyl)pyrimidine

2-(3-Quinolylmethyl)-3-anilinoacrylonitrile (6.38g) was treated with guanidine in the manner of example 5B. There was isolated 4.90g (87%) of 2,4-Diamino-5-(3-quinolylmethyl)pyrimidine which was recrystallised from methyl cellosolve with the aid of decolorising charcoal; m.p. 279-282° C(dec). Anal. Calcd. for $C_{14}H_{13}N_5$: C, 66.92; H, 5.21; N, 27.87. Found: C, 66.57; H, 5.36; N, 27.54.

Example 7

2,4-Diamino-5-(1,2,3,4-tetrahydro-8-methoxy-4-methyl-6-quinolylmethyl)pyrimidine

A. 2-Chloro-8-methoxy-4-methylquinoline

8-Methoxy-4-methyl-2-quinolone (R.M.Forbis and K.L.Rinehart, Jr., J. Am. Chem. Soc ., 95 , 5003 (1973)), 3.93g., was treated with 6 ml. of phosphorus oxychloride at 120° for 2 hours, neutralised with 15 ml of concentrated ammonium hydroxide in 100 ml of ice to pH 9, and extracted with 2 times 100 ml of ethyl acetate; 4.24g of product was obtained, which was purified on a silica gel column, eluted with heptane:ethyl acetate 3:1, to give 4.2g. (97%) of 2-chloro-8-methoxy-4-methylquinoline (A), m.p. 106-108°. NMR ($CDCl_3$) δ 2.66 (s, 3, Me), 4.05 (s, 3, OMe), 7.02 (m, 4, $ArH_3$,pyr-beta-H). Anal. Calcd. for $C_{11}H_{10}ClNO$: C, 63.62; H, 4.85; N, 6.75. Found: C, 63.59; H, 4.89; N, 6.72.

B. 8-Methoxy-4-methylquinoline

The product of A, 1.85g was dissolved in 50 ml of absolute ethanol and dechlorinated on a Parr hydrogenation apparatus using 5% Pd/C catalyst. After removal of the catalyst, the solvent was evaporated, and the residue neutralised with 50 ml of 0.5 M sodium bicarbonate, and extracted twice with 50 ml of

methylene chloride. The extract was dried over $MgSO_4$, filtered, and evaporated, giving 1.35g (88%) of (B), m.p. 68-72°. NMR: ($CDCl_3$) δ 2.52 (s, 3, Me), 4.03 (s, 3, OMe), 6.9-7.05 (m, 1, Ar), 7.18 (d, 1, pyr-beta-H, J=4.5 Hz), 7.45 (m, 2, $ArH_2$), 8.73 (d, 1, pyr-alpha-H, J=4.5 Hz). Anal. Calcd. for $C_{11}H_{11}NO$; C, 76.28; H, 6.40; N, 8.09. Found: C, 76.00; H, 6.73; N, 7.74.

C. 1,2,3,4-Tetrahydro-8-methoxy-4-methylquinoline

The product of B, 1.25 g was reduced in 40 ml of absolute ethanol using 4 equivalents of sodium cyanoborohydride plus 4 equivalents of concentrated hydrochloric acid. The reaction was stirred at room temperature for 1 hour, heated at 60° for two hours, and then allowed to stir overnight at room temperature. The reaction mixture was made basic with ammonia, diluted with 50 ml of water, and extracted three times with 75 ml portions of methylene chloride, followed by drying the extracts and removal of the solvent. The residual orange oil, 1.26g. was purified on a silica gel column using 2% ethyl acetate in heptane, which produced a light yellow oil, 0.95g (74%) of C. NMR: ($CDCl_3$) δ 1.25 (d, 3, Me, J=7 Hz), 1.6 - 2.3 (m, 2, $C^3H_2$), 2.6-3.2 (m, 1, $C^4$-H), 3.27 (tr, 2, $C^2H_2$, J= 5.5 Hz), 3.74 (s, 3, OMe), 4.07 (br, 1, NH), 6.53 (m, 3, $ArH_3$). Anal. Calcd. for $C_{11}H_{15}NO$: C, 74.54; H, 8.53; N, 7.90. Found: C, 74.57; H, 8.57; N, 7.81.

D. 2-4-Diamino-5-(1,2,3,4-tetrahydro-8-methoxy-4-methyl-6-quinolylmethyl)pyrimidine

The procedure of Example 1 was used to react product C (0.86g) with 2,4-diamino-5-hydroxymethyl-pyrimidine in acetic acid, with 2 equivalents of hydrochloric acid. The reaction was refluxed for 3.5 hours, the solvent removed; and the residue dissolved in water and made basic with ammonia, followed by extraction with methylene chloride. The extract was purified on a silica gel column which was eluted with methylene chloride: methanol/19:1, giving 1.20g (93%) of the title compound. A portion was converted to the dihydrochloride salt with ethanol-hydrochloric acid; m.p. 221.223°. Anal. Calcd. for $C_{16}H_{21}N_5O.2HCl.H_2O$: C, 49.24; H, 6.46; N, 17.94. Found: C, 49.34; H, 6.48; N, 17.92.

Example 8

2,4-Diamino-5-(8-methoxy-6-quinolylmethyl)pyrimidine

The product of Example 2 was oxidised using 20% palladium on charcoal in 50 ml Of cumene, by heating at 150° C for 21 hours. After the catalyst was removed and the solvent evaporated, the residue was purified on a silica gel column which was eluted with methylene chloride:methanol/19:1. This produced 0.51g (35%) of the title compound, which melted at 285-287° after recrystallisation from betamethoxyethanol. Anal. Calcd. for $C_{15}H_{15}N_5O$: C, 64.04; H, 5.37; N, 24.89. Found: C, 63.88; H, 5.37; N, 24.84.

Example 9

2,4-Diamino-5-(8-(2-methoxyethoxy)-6-quinolylmethyl)pyrimidine

The product of Example 3 was oxidised in the manner of Example 8, and purified similarly. After recrystallisation from beta-methoxyethanol, there was obtained 0.18g (20%) of title compound, melting at 253-255° C. Anal. Calcd. for $C_{17}H_{19}N_5O_2$: C, 62.76; H, 5.89; N, 21.52. Found: C, 62.58; H, 5.92; N, 21.45.

Example 10

2,4-Diamino-5-(8-methoxy-4-methyl-6-quinolylmethyl)pyrimidine

The product of Example 7 was oxidised in the manner described in Example 8. The product was recrystallised from betamethoxyethanol, giving 0.11g (17%) of the title compound,which melted at 287-290°. Anal. Calcd. for $C_{16}H_{17}N_5O$; C, 65.07; H, 5.80; N, 23.71. Found: C, 65.01; H, 5.83; N, 23.69.

Example 11

2,4-Diamino-5-(4-methyl-6-quinolylmethyl)pyrimidine

The product of Example 4 was oxidised in the manner of Example 8, and purified similarly. After recrystallisation from beta-methoxyethanol, there was obtained 1.44g (55%) of the title compound, melting at 265-268° C. NMR: ($Me_2SO-d_6$) δ2.64 (s, 3, Me), 3.83 (s, 2, $CH_2$), 5.70 (br s, 2, $NH_2$), 6.16 (br s, 2, $NH_2$), 7.33 (d, 1, pyridine-beta H, J=4.5), 7.56 (dd, 1, $ArH^7$, J=2•8), 7.59 (s,1, pyrimidine-$H^6$), 7.92 (d, 1, $ArH^8$, J=8.8), 7.98 (d, 1, $ArH^5$, J=1.6), 8.68 (d, 1, pyridine-alpha H, J=4.4). Anal . Calcd. for $C_{15}H_{15}N_5$: C, 67.91; H, 5.70; N 26.40. Found: C, 67.82; H, 5.74; N, 26.35.

Example 12

2,4-Diamino-5-(4-methyl-8-nitro-6-quinolylmethyl)pyrimidine

The product of Example 11 (0.53g, 2 mmol) was dissolved in 7 ml of concentrated sulfuric acid, and chilled to 0°. Then 0.3 ml (6.4 mmol) of fuming nitric acid (d=1.5) in 0.5 ml of concentrated sulfuric acid was added dropwise to the solution. The reaction was stirred at 0-5° for 30 minutes, then at 25° for 1 hour. It was then poured onto 50 ml of ice and neutralised to pH 9 with concentrated ammonium hydroxide. The precipitate which formed was filtered and dried, and then purified on a silica gel column which was eluted with methylene chloride: methanol/12:1, giving 0.33g (53%) of the title compound, which melted at 256-258° after recrystallisation from beta methoxyethanol:water/2:1. NMR ($Me_2SO-d_6$) δ 2.72 (s, 3, Me), 3.90 (s, 2, $CH_2$), 5.77 (br s, 2, $NH_2$), 6.24 (br s, 2, $NH_2$), 7.55 (d, 1, pyridine-beta H, J=4.1), 7.71 (s, 1, pyrimidine-$H^6$), 8.05 (d, 1, $ArH^5$, J=1.8), 8.28 (d, 1, $ArH^7$, J=1.6), 8.81 (d, 1, pyridine-alpha H, J=4.4). Anal . Calcd. for $C_{15}H_{14}N_6O_2 0.5H_2O$: C, 56.42; H, 4.73; N, 26.32. Found: C, 56.34; H, 4.82; N, 26.21.

Example 13

2,4-Diamino-5-(5,6,7-trimethoxy-2-naphthylmethyl)pyrimidine hydrochloride

A. 5,6,7-Trimethoxynaphthalene-2-carboxaldehyde

Methyl 5,6,7-trimethoxynaphthalene-2-carboxylate (C.L. Chen, F.D. Hostettler, Tetrahedron, 1969, 25 , 3223) was reduced to the aldehyde in the following manner. Sodium bis(2-methoxyethoxy)aluminium hydride in toluene (5.6 ml, 20 mmol, 70% solution) was chilled to -20° C. under nitrogen in 20 ml of dry toluene and then 1.74 ml (20 mmol) of morpholine in 5 ml of toluene was added dropwise to it. The reaction was allowed to stir at -5° for 30 minutes, after which it was rechilled to -20° and added slowly to a solution of methyl 5,6,7-trimethoxy-naphthalene-2-carboxylate (2.76g, 10 mmol) in 20 ml of toluene at -20°. The reaction was stirred for 30 minutes at -10°, then rechilled to -20°, basified with 20 ml (40 mmol) of 2N sodium hydroxide, and extracted with three times 25 ml of toluene. The combined toluene layers were washed with 50 ml of 1 N hydrochloric acid, then with 50 ml of 0.5 M sodium bicarbonate, and finally with 50 ml of water, and dried over magnesium sulfate, filtered and evaporated to give 2.55 g (52% crude yield) of the title compound. This was purified on a silica gel column eluting with hexane:ethyl acetate/12:1 to give 2.15g (44%) of the product, m.p. 96-98°. Anal . Calcd. for $C_{14}H_{14}O_4$: C, 68.28; H, 5.73. Found: C, 68.16; H, 5.78.

B. 2,4-Diamino-5-(5,6,7-trimethoxy-2-naphthylmethyl)pyrimidine hydrochloride

The aldehyde from above was converted to 2-(5,6,7-trimethoxy-2-naphthyl-methyl)-3-anilinoacrylonitrile on a 8 mmol scale in the same manner as Example 5-A. The crude product from this reaction was cyclised with guanidine hydrochloride and sodium methylate in ethanol as in Example 5-B to give the title compound as the free base, 0.74g (27% yield). This was recrystallised from ethanol plus an equivalent of hydrochloric acid, 0.26g, mp 252-254°. Anal . Calcd. for $C_{18}H_{20}N_4O_3HCl$: C57.37; H, 5.62; N, 14.87; Cl, 9.41. Found: C, 57.11; H, 5.67; N, 14.79; Cl, 9.31.

Example 14

2,4-Diamino-5-(8-methoxy-2,4-dimethyl-6-quinolylmethyl)pyrimidine

A. 2,4-diamino-5-(3-methoxy-4-aminobenzyl)pyrimidine

A solution of 6.30 g (0.045 mol) of 2,4-diamino-5-hydroxymethylpyrimidine, 6.15g (0.05 mol)of σ-anisidine and 3.75 ml of concentrated hydrochloric acid in 55 ml of glacial acetic acid was heated to reflux for 6 hours. The mixture was stirred at room temperature overnight. The solvent was removed under vaccum and the residue was taken up in water, made basic with ammonium hydroxide, and the aqueous solution was extracted with dichloromethane:methanol/3:1. The organic layers were combined, dried and concentrated to a purple glass. This was purified on a silica gel column to give 7.81g of the 4-N-acetylated product.

This product was dissolved in 400 ml of 2N sodium hydroxide and heated to reflux for a total of 6 hours. The mixture was cooled and the solid was filtered. This was dissolved in water, taken to pH 8.5, and the aqueous solution was extracted with dichloromethane. The organic extract was dried and concentrated to give 4.0g of the title compound; m.p. 210-212 °C. Anal . Calcd for $C_{12}H_{15}N_5O$: C, 58.76; H, 6.16; N, 28.55. Found: C, 58.66; H, 6.22; N, 28.48.

B. 2,4-Diamino-5-(8-methoxy-2,4-dimethyl-6-quinolylmethyl)pyrimidine

To a solution of 1,5g (0.006 mol) of the product from A in 30 ml of 95% ethanol, 0.5 ml of concentrated hydrochloric acid, and 2.43g (0.0089 mol)of ferric chloride hydrate was added 0.5g (0.006 mol) of 3-penten-2-one. Following the dropwise addition the solution was refluxed for 6 hours. The solvent was removed under vaccum and the residue was dissolved in water and neutralised with ammonium hydroxide. The black solid which precipitated was collected by filtration. Purification on a silica gel column followed by recrystallisation from ethanol gave 0.1094g (5.88%) of the title compound; m.p. 289-290 °C. Anal Calcd for $C_{17}H_{19}N_5O$: C, 66.00; H, 6.19; N, 22.64. Found: C, 65.75; H, 6.26; N, 22.56.

Example 15

2,4-Diamino-5-(8-chloro-1,2,3,4-tetrahydro-2,4-dimethyl-6-quinolylmethyl)pyrimidine hemihydrate

A. 8-chloro-2,4-dimethylquinoline

To 4.0g (0.03 mol) of o-chloroaniline in 50 ml of hydrochloric acid at 100 °C was added dropwise 3.4 (0.04 mol) of 3-penten-2-one. The mixture was refluxed for 12 hours, then neutralised with 5N sodium hydroxide and extracted with dichloromethane. The organic extract was dried and concentrated to an oil. This was purified on a silica gel column to give 2.54g (42%) of the title product; m.p. 66 °-68 °C. Anal . Calcd. for $C_{11}H_{10}NCl$: C, 68.94; H, 5.26; N, 7.31; Cl, 18.50. Found C, 68.87; H, 5.27; N, 7.29; Cl, 18.48.

B. 1,2,3,4-Tetrahydro-8-chloro-2,4-dimethylquinoline

To 1.7g (0.0088 mol) of the product from A dissolved in 25 ml of ethanol was added 2.23g (0.035 mol) of sodium cyanoborohydride and 3.5g of concentrated hydrochloric acid. After heating 1 hour at 80 °, one equivalent more of each of sodium cyanoborohydride and acid were added and the mixture was heated at 80 ° for 1 hour longer. Water was added, the reaction mixture was made basic with ammonium hydroxide, and extracted with dichloromethane. The organic extract was dried and concentrated to an oil. This was purified on a short silica gel column to give 1.23 g of the title compound.

C. 2,4-Diamino-5-(8-chloro-1,2,3,4-tetrahydro-2,4-dimethyl-6-quinolylmethyl)-pyrimidine hemihydrate

A mixture of 0.9g (0.0046 mol) of the product from B, 0.6g (0.0043 mol) of 2,4-diamino-5-hydroxymethyl pyrimidine, 0.8 ml of concentrated hydrochloric acid, and 10ml of glacial acetic acid was heated under

reflux for 6 hours. The solvent was removed under vacuum, the residue was dissolved in water and made basic with ammonium hydroxide. The gummy solid which resulted was extracted with dichloromethane:methanol/3:1. The extracts were dried and evaporated to leave a green crystalline solid (1.22g). Purification on a short silica gel column followed by recrystallisation gave 0.058g of the title compound; m.p. 195°-197°C. Anal . Calcd. for $C_{16}H_{20}N_5Cl.\frac{1}{2}H_2O$: C, 58.80; H, 6.48; N, 21.43; Cl, 10.85. Found: C, 58.75; H, 6.47; N, 21.39; Cl, 10.83.

Example 16

2,4-Diamino-5-(1,2,3,4-tetrahydro-N-methyl-6-quinolylmethyl)pyrimidine

A. 1,2,3,4-Tetrahydro-N-methylquinoline

1,2,3,4-Tetrahydroquinoline (6.66g, 50 mmol) was added to 40ml of water, 40 ml of ethyl acetate, and 5.04g (60 mmol) of sodium bicarbonate to which was added 5.68 ml (60 mmol) of dimethylsulfate dropwise. The reaction was stirred at room temperature for 2½ hours, and the organic layer was separated. The aqueous layer was extracted with ethyl acetate, then the organic layers were combined and evaporated to give 4.56 g (62% yield) of the title compound. NMR ($CDCl_3$) $\delta$ 1.93 (quintet, 2, $CH_2$), 2.74 (tr, 2, $CH_2$), 2.81 (s, 3, NMe), 3.17 (tr, 2, $CH_2$), 6.55 (m, 2, Ar), 6.97 (m, 2, Ar).

B. 2,4-Diamino-5-(1,2,3,4-tetrahydro-N-methyl-6-quinolylmethyl)pyrimidine

The product from above (0.86g, 5.8 mmol) and 0.82g (5.8 mmol) of 2,4diamino-5-hydroxymethylpyrimidine were dissolved in 10 ml of glacial acetic acid and 0.5 ml of concentrated hydrochloric acid and refluxed for 1 hour. The solvent was evaporated, and the residue was dissolved in water and made basic with ammonium hydroxide to pH 9. The aqueous layer was extracted with methylene chloride: methahol/3:1, which was dried and evaporated to give 1.53g of the crude product. This was purified on a silica gel column eluted with methylene chloride:methanol/19:1, followed by recrystallisation to give 1.07g (68% yield) of the title compound, mp 190-191° (absolute ethanol). NMR: ($Me_2SO-d_6$) $\delta$ 1.84 (quintet, 2, $CH_2$), 2.63 (tr, 2, $CH_2$), 2.77 (s, 3, NMe), 3.11 (tr, 2, $NCH_2$), 3.41 (s, 2, pyrimidine-$CH_2$), 5.63 (br s, 2, $NH_2$), 5.93 (br s, 2, $NH_2$), 6.47 (d, 1, $ArH^8$), 6.73 (d, 1, $ArH^5$), 6.84 (dd, 1 $ArH^7$), 7.44 (s, 1, pyrimidine-$H^6$). Anal . Calcd. for $C_{15}H_{19}N_5$: C, 66.89; H, 7.11; N, 26.00. Found: C, 66.84; H, 7.13; N, 25.95.

Example 17

2,4-Diamino-5-(N-ethyl-1,2,3,4-tetrahydro-4-methyl-6-quinolylmethyl)pyrimidine dihydrochloride hydrate

A. N-Ethyl-1,2,3,4-tetrahydro-4-methylquinoline

4-Methylquinoline (lepidine) (1.43g, 10 mmol) and 50 ml of glacial acetic acid were added together and cooled to 10°C. Sodium cyanoborohydride (2.64g, 42 mmol) was added gradually, and the reaction was stirred at 25° for 2 hours, and then heated at 55° for 1½ hours. After stirring overnight at 25°, the reaction was neutralised with concentrated ammonium hydroxide to pH 10.5, and then the product was extracted into methylene chloride and evaporated. The crude product was purified on a silica gel column eluting with hexane to give 0.55g (31% yield) of the title compound. MS: 175 ($M^+$), 160 ($M^+$ - Me); NMR: $CDCl_3$ $\delta$ 1.22 (tr, J=7 Hz, 3, $NCH_2Me$ ), 1.30 (d, J=3.5, 3, CH Me ), 1.5-2.3 (m, 2, $CH_2$) 2.89 (sextet, 1, C H Me), 3.29 (tr, 2, $NCH_2$), 3.30 (quartet, 2, N CH $_2$Me), 6.59 (m-tr, 2, Ar), 7.10 (m-tr, 2, Ar).

B. 2,4-Diamino-5-(N-ethyl-1,2,3,4-tetrahydro-4-methyl-6-quinolylmethyl)-pyrimidine dihydrochloride hydrate

The product from above (0.38g, 2.2 mmol) was condensed with 2,4diamino5-hydroxy-methylpyrimidine as in Example 17. The crude product from the reaction was purified on a silica gel column eluted with methylene chloride:methanol/19:1, giving 0.53g of product (83% yield). This was recrystallised from absolute ethanol with two equivalents of hydrochloric acid to give 0.16g of the title compound, mp 250-

252°. NMR: ($Me_2SO-d_6$) (free base from column) δ 1.02 (tr, 3, $NCH_2Me$), 1.15 (d, 3, CH Me), 1.6-1.85 (m, 2, $CH_2$), 2.72 (m, 1, CH Me), 3.15 (m, 4, N $CH_2$, N $CH_2Me$), 3.42 (s, 2, pyrimidine-$CH_2$), 5.61 (br s, 2, $NH_2$), 5.94 (br s, 2, $NH_2$), 6.48 (d, J=9 Hz, 1, $ArH^8$), 6.81 (dd, J=2, 9 Hz, 1, $ArH^7$), 6.84 (d, J=2 Hz, 1, $ArH^5$), 7.44 (s, 1, pyrimidine-$H^6$). Anal. Calcd. for $C_{17}H_{23}N_5.2HCl.H_2O$: C, 52.58; H, 7.01; N, 18.03; Cl, 18.25. Found: C, 52.63; H, 7.03; N, 18.01; Cl, 18.10.

Example 18

2,4-Diamino-5-(8-amino-4-methyl-6-quinolyimethyl)pyrimidine dihyrochloride

The product of Example 12 (0.78g, 2.5 mmol) was dissolved in 35 ml of β-methoxyethanol, and then 0.06g of 5% Pd/C and 0.3 ml of 95% hydrazine were added, and the reaction was refluxed for 1 hour. The Pd/C was filtered off, the solvent removed, and the product was purified on a silica gel column which was eluted with 7% methanol in methylene chloride. This gave 0.48g (69% yield) of the free base which was recrystallised as the dihydrochloride salt from ethanol, mp 303-305° dec. Anal. Calcd. for $C_{15}H_{16}N_6.2HCl.0.5H_2O$: C, 49.73; H, 5.29; N, 23.20; Cl, 19.57. Found: C, 49.71; H, 5.30; N, 23.18; Cl, 19.51.

Example 19

2,4-Diamino-5-(5-amino-4-methyl-6-quinolylmethyl)pyrimidine dihydrochloride

When the product of Example 11 was nitrated and only partially purified by a silica gel column without recrystallisation, and then reduced as in Example 18, a second amino-quinoline product was detected and isolated from a column. On a 2.5 mmol scale, there was obtained 0.235g (32%) of the title compound, mp 290° dec. (HCl in absolute ethanol). NMR of the free base: ($Me_2SO-d_6$) δ 2.96 (s, 3, Me), 3.60 (s, 2, $CH_2$), 5.08, 5.70, 6.14 (3 broad bands, 6, $(NH_2)_3$), 6.99 (d, J=8 Hz, 1, Ar), 7.16 (d, J=8 Hz, 1, Ar), 7.24 (s, 1, pyrm-$H_6$), 7.27 (d, J=4 Hz, 1, pyr- H), 8.51 (d, J=4 Hz, 1, pyr- H). Anal. Calcd. for $C_{15}H_{16}N_6.2HCl$: C, 51.00; H, 5.14; N, 23.79; Cl, 20.07. Found: C, 50.95; H, 5.17; N, 23.72; Cl, 19.97.

Example 20

2,4-Diamino-5-(1,2,3,4-tetrahydro-8-methoxy-1,4-dimethyl-6-quinolylmethyl)-pyrimidine dihydrochloride

A. 1,2,3,4-Tetrahydro-8-methoxy-1,4-dimethylquinoline

The product of Example 7-C (1,2,3,4-tetrahydro-8-methoxy-4-methylquinoline) was methylated by dissolving the compound (0.71g, 4 mmol) in 15 ml of tetrahydro-furan under nitrogen, chilling to 0° C, and then 1.14g (30 mmol) of sodium borohyride was added, followed by a slow addition of 12 ml of formic acid. The reaction was allowed to warm to room temperature, and then it was stirred overnight. The solvent was removed, the residue was slurried in water and basified to pH 9 with ammonium hydroxide and extracted into methylene chloride. The product was purified on a silica gel column which was eluted with hexane:ethyl acetate/19:1 giving a light brown oil. Anal. Calcd. for $C_{12}H_{17}NO$: C, 75.35; H, 8.96; N, 7.32. Found: C, 75.44; H, 8.98; N, 7.28.

B. 2,4-Diamino-5-(1,2,3,4-tetrahydro-8-methoxy-1,4-dimethyl-6-quinolylmethyl)-pyrimidine dihydrochloride

The product from above (0.5g, 2.6 mmol) was condensed with 2,4-diamino-5-hydroxy-methylpyrimidine as in Example 17 and 18. The product was worked up as before in Example 17, and then purified on a silica gel column which was eluted with methylene chloride:ethanol/19:1 giving 0.27g (33% yield) of the title compound as the free base as well as recovering 0.17g of unreacted tetrahydro-quinoline starting material (34%). The product was recrystallised from absolute ethanol as the dihydrochloride, mp 219-221°. Anal. Calcd for $C_{17}H_{23}N_5O.2HCl.0.5\ H_2O$: C, 51.65; H, 6.63; N, 17.71; Cl, 17.94. Found: C, 51.58; H, 6.64; N, 17.66; Cl, 17.87.

Example 21

Ethyl 6-(2,4-diamino-5-pyrimidinylmethyl)-1,2,3,4-tetrahydro-5,8-dimethoxy-3-quinolinecarboxylate

A. Ethyl 1,2,3,4-tetrahydro-5,8-dimethoxy-3-quinolinecarboxylate

Ethyl 5,8-dimethoxy-3-quinolinecarboxylate was formed as described in the literature (E.H.Erickson, C.F.Hainline, L.S.Lenon, et. al. J. Med. Chem. 1979, 22 , 816). 2,5-Dimethoxyaniline and diethyl ethoxymethylenemalonate condense and then cyclise at high temperature (250° ) in diphenyl ether to form ethyl 1,4-dihydro-5,8-dimethoxy-4-oxo-3-quinolinecarboxylate which is chlorinated at the 4- position with phosphorus oxychloride to give ethyl 4-chloro-5,8-dimethoxy-3-quinolinecarboxylate. The dehalogenation of this quinoline (20.34g, 0.069 mol) in 150 ml of absolute ethanol with 1g of 5% Pd/C and 22.53 ml of triethylamine (0.156 mol) was done with a Parr hydrogenator apparatus which gave the correct product, ethyl 5,8-dimethoxy-3-quinolinecarboxylate, as well as the 1,4-dihydro and the 1,2,3,4-tetrahydro quinoline products. This result of further reduction to the 1,4-dihydro product was indicated in the article listed above on page 817, but the formation of the 1,2,3,4-tetrahydro product had not been mentioned there. These three quinoline products were separated on a silica gel column which was eluted with hexane, followed by hexane:ethyl acetate/4:1 and 1:1, giving 0.46g (2.6% yield) of the title compound, NMR: $CDCl_3 \delta$ 1.23 (tr, 3, $CH_2Me$ ), 2.94 (br-m, 2, $CH_2$), 3.2-3.6 (m, 3, $CH_2$, CH), 3.76 (s ,6, $(OMe)_2$), 4.21 (quartet, 2, $CH_2Me$), 4.2 (br, 1, NH), 6.12 (d, 1, Ar), 6.57 (d, 1, Ar); 3.31 g of the 1,4-dihydro quinoline product (18.5%), NMR: $CDCl_3 \delta$1.28 (tr, 3, $CH_2Me$ ), 3.61 (s, 2, $CH_2$), 3.73 (s, 6, $(OMe)_2$), 4.19 (quartet, 2, $CH_2Me$), 6.2-6.7 (br, 1, NH), 6.29 (d, 1, Ar), 6.60 (d, 1, Ar), 7.32 (d, 1, pyr- H); and 2.04g (11%) of the ethyl 5,8-dimethoxy-3-quinolinecarboxylate.

B. Ethyl 6(2,4-diamino-5-pyrimidinylmethyl)-1,2,3,4-tetrahydro-5,8-dimethoxy-3-quinolinecarboxylate

Ethyl-1,2,3,4-tetrahydro-5,8-dimethoxy-3-quinolinecarboxylate (0.38g, 1.43 mmol) was reacted with 2,4-diamino-5-hydroxymethylpyrimidine as in Example 17, and the product worked up as before. The product was purified on a silica gel column eluted with methylene chloride:methanol/19:1 to give 0.27g (49%) of the title compound, mp 186-188° (absolute ethanol). Anal . Calcd. for $C_{19}H_{25}N_5O_4$: C, 58.90; H, 6.50; N, 18.08. Found: C, 58.95; H, 6.52; N, 18.08.

Example 22

2,4-Diamino-5-(4,8-dimethoxy-2-methyl-6-quinolylmethyl)pyrimidine

A. 4-Bromo-2-methoxyaniline

2-Methoxyaniline (o-anisidine) (15g, 0.122 mol) was brominated with 2,4,4,6-tetra-bromo-2,5-cyclohexadienone (50g, 0.122 mol) by dissolving the aniline in 250 ml of methylene chloride, chilling the solution to -10°, and slowly adding and brominating agent, keeping the termperature below -5°. The reaction was allowed to warm at room temperature, and then washed with 2N sodium hydroxide (2 x 75 ml), then washed with water (2 x 25 ml), dried over magnesium sulfate, and evaporated to dryness. The product was purified on a silica gel column, eluted with methylene chloride giving 23.68g (96%) of the title compound, mp 56.5-58° (petroleum ether). Anal . Calcd for $C_7H_8BrNO$: C, 41.61; H, 3.99; Br, 39.55; N, 6.93. Found: C, 41.59; H, 3.99; Br, 39.49; N, 6.92.

B. Ethyl 3-(4-bromo-2-methoxyphenylimino)butyrate

The product from above (5.25g, 26 mmol) and ethyl acetoacetate (3.39g, 26 mmol) were added together in 20 ml of absolute ethanol with 0.06 ml of glacial acetic acid and 7g of drierite and refluxed for 4 hours. The drierite was filtered off, the solvent removed, and the product was purified on a silica gel column eluted with hexane:ethyl acetate/19:1 to give 6.47g (79%) of the title compound as a colourless oil. NMR: $CDCl_3$ $\delta$ 1.28 (tr, 3, $CH_2Me$ ), 1.97 (s, 3, =CH-Me ), 3.85 (s, 3, OMe), 4-17 (quartet, 2, $CH_2Me$), 4.75 (s, 1, =CH), 7.00 (s, 3, Ar). Anal , Calcd. for $C_{13}H_{16}BrNO_3$: C, 49.70; H, 5.13; N, 4.46. Found: C, 49.52; H, 5.16;

N, 4.43.

C. <u>6-Bromo-4-hydroxy-8-methoxy-2-methylquinoline</u>

The product from above (6.32g, 20.1 mmol) was cyclised in diphenyl ether (30 ml) when heated at 255° for 25 minutes. The product precipitated out of the diphenyl ether 3.80g (70.5%) which was washed well with diethyl ether, and then recrystallised from absolute ethanol, mp 293-296°. Anal. Calcd. for $C_{11}H_{10}BrNO_2$: C, 49.28; H, 3.76; N, 5.22. Found: C, 49.17; H, 3.78; N, 5.21.

D. <u>6-Bromo-4-chloro-8-methoxy-2-methylquinoline</u>

The product from above (2.79g, 10.4 mmol) was chlorinated by refluxing with 13 ml of phosphorus oxychloride at 120° for 2 hours, neutralised with 8 ml of ammonium hydroxide in 100 ml of ice to pH 9, and extracted into methylene chloride. The crude product was purified on a silica gel column eluted with hexane:ethyl acetate/5:1 to give 2.76g (93%) of the title compound, mp 140-142°. Anal. Calcd for $C_{11}H_9BrClNO$: C, 46.11; H, 3.17; N, 4.89. Found: C, 46.04; H, 3.19; N, 4.88.

E. <u>6-Bromo-4,8-dimethoxy-2-methylquinoline</u>

The product from above (0.85g, 3.0 mmol) was dissolved in 40 ml of methanol with 0.8g (15 mmol) of sodium methylate and heated in a steel bomb at 120° for 5 hours. The solvent was removed, water added, and the product was extracted into methylene chloride and then purified on a silica gel column, eluting with hexane:ethyl acetate/1:3 to give 0.72g (86%), mp 167-168°. Anal. Calcd. for $C_{12}H_{12}BrNO_2$: C, 51.09; H, 4.29; N, 4.96. Found: C, 50.95; H, 4.34; N, 4.90.

F. <u>6-Formyl-4,8-dimethoxy-2-methylquinoline</u>

The product from above (0.67g, 2.4 mmol) was dissolved in 25 ml of dry tetrahydrofuran and chilled to -70° under nitrogen. Then 1.63 ml (1.1 equiv.) of 1.6 M n-butyl lithium in hexane was added dropwise via a syringe, and the reaction was stirred for 2 minutes followed by the addition of 0.21g (1.2 equiv) of dry dimethylformamide. The reaction was allowed to warm to -40°, and then it was quenched with 8 ml of 1N hydrochloric acid. The reaction was extracted with ether, the aqueous phase was basified to pH 12 with 1N sodium hydroxide and extracted into methylene chloride and evaporated to dryness. The product was purified on a silica gel column which was eluted with 2% methanol in methylene chloride to give 0.33g (60%) of the title compound, NMR: $CDCl_3$ δ2.23 (s, 3, Me), 4.01 (s, 3, OMe), 4.06 (s, 3, OMe), 6.70 (s, 1, pyr-β H), 7.40 (d, 1, Ar), 8.14 (d, 1, Ar), 9.98 (s, 1, CHO).

G. <u>2,4-Diamino-5-(4,8-dimethoxy-2-methyl-6-quinolylmethyl)pyrimidine</u>

The aldehyde from above was converted to 2-(4,8dimethoxy-2-methyl-6-quinolyl-methyl)-3-anilinoacrylonitrile with anilinopropionitrile and sodium methylate in dimethyl sulfoxide on a 1.4 mmol scale in the same manner as in Example 5-A. The crude product from this reaction was condensed with guanidine hydrochloride and sodium methylate in ethanol as in Example 5-B to give the crude product. This was purified through a silica gel column, eluting with methylene chloride: methanol/9:1, and then recrystallised to give the title compound, mp 288-293° (abs. ethanol); NMR: ($Me_2SO-d_6$) δ 2.56 (s, 3, Me), 3.74 (s, 2 $CH_2$), 3.89 (s, 3, OMe), 3.96 (s, 3, OMe), 5.70 (br s, 2, $NH_2$), 6.08 (br s, 2, $NH_2$), 6.89 (s, 1, pyr- H), 7.04 (d, 1, Ar), 7.37 (d, 1, Ar), 7.57 (s, 1, pyrm-6-H). Anal. Calcd. for $C_{17}H_{19}N_5O_2 0.75H_2O$: C, 60.25; H, 6.10; N, 20.67. Found: C, 60.10; H, 6.07; N, 20.50.

<u>Example 23</u>

<u>2,4-Diamino-5-(4-dimethylamino-8-methoxy-2-methyl-6-quinolylmethyl)pyrimidine</u>

A. 6-Bromo-4-dimethylamino-8-methoxy-2-methylquinoline

The product from Example 22-D (1.0g, 3.45 mmol) was dissolved in 50 ml of a 10% solution of dimethylamine in ethanol (5g/50 ml), and heated in a steel bomb at 120° for 5 hours. The reaction was worked up as in Example 22-E and purified on a silica gel column which was eluted with hexane: ethyl acetate/1:4 to give 0.89g (89%) of the title compound, m.p. 115-117°. Anal . Calcd. for $C_{13}H_{15}BrN_2O$: C, 52.90; H, 5.12; N, 9.49. Found: C, 52.85; H, 5.16; N, 9.48.

B. 4-Dimethylamino-6-formyl-8-methoxy-2-methylquinoline

The product from above (0.78g, 2.6 mmol) was formylated as in Example 22-F to give the title compound, mp 127-129°. Anal . Calcd. for $C_{14}H_{16}N_2O_2\frac{1}{3}$ $H_2O$: C, 67.18; H, 6.71; N, 11.19. Found: C, 67.40; H, 6.67; N, 11.00.

C. 2,4-Diamino-5-(4-dimethylamino-8-methoxy-2-methyl-6-quinolylmethyl)pyrimidine

The aldehyde from above was condensed with anilinopropionitrile as in Example 22-G, followed by the reaction with guanidine to give the crude product. This was purified on a silica gel column which was eluted with methylene chloride:methanol/4:1 to give the title compound, MS 338($M^+$).

Example 24

2,4-Diamino-5-(2-dimethylamino-4-methyl-6-quinolylmethyl)-pyrimidine

A. N-(4-Bromophenyl)-3-oxobutyramide

To a stirred solution of 34.14g (0.200 moles) of 4-bromoaniline in 130 ml toluene under $N_2$, 18.00g (0.220 moles) of diketene was added dropwise over a 10 minute period followed by 15 ml of toluene. The temperature rose to 80°C during the addition; the solution was then refluxed 20 minutes, cooled to 55°C and 60 ml of petroleum ether added. An immediate precipitation occurred. The tan-white crystals were filtered and washed with three 100 ml portions of 1:1 toluene/petroleum ether. The product was taken up in hot absolute ethanol and crystallisation induced by addition of toluene to the ethanolic solution. Three crops of crystals from the ethanol/toluene solvent system gave 22.90g (43%) and had $R_f$ 0.69 on silica TLC with ethyl acetate; mp 135.8-137.2°C. Anal . Calcd. for $C_{10}H_{10}BrNO_2$: C, 46.90; H, 3.94; N, 5.47; Br, 31.20. Found: C, 47.13; H, 4.00; N, 5.46; Br, 31.31.

B. 6-Bromo-4-methyl-2-(1H)-quinolinone

A mixture of 3.00g (0.0117 moles) of N -(4-bromophenyl)-3-oxobutyramide and 6 ml concentrated sulfuric acid was stirred and heated to 95-100°C ($H_2O$ bath) for 1½ hours. The resulting solution was poured onto an ice/$H_2O$ mixture to yield a white crystalline product. The crystals were collected and taken up in 200 ml absolute ethanol. The volume was reduced to 150 ml followed by chilling; crystals were isolated (0.71g 25.6%), mp 292-299°C. Anal . Calcd. for $C_{10}H_8BrNO$: C, 50.45; H, 3.39; N, 5.88; Br, 33.56. Found: C, 50. 28; H, 3.44; N, 5.83; Br, 33.68. A TLC of the product on silica showed $R_f$ 0.18 with ethyl acetate eluent.

C. 6-Bromo-2-chloro-4-methylquinoline

A 0.46g, (0.0019 mole) sample of 6-bromo-4-methyl-2(1 H )-quinolinone was dissolved in 3.0 ml $POCl_3$ under $N_2$ with stirring and heated to reflux for 2 hours. The solution solidified into a purple gum; 2.0 ml of extra $POCl_3$ was added to dissolve the solid. The solution was then slowly poured onto a vigorously stirred slurry of 8 ml concentrated $NH_4OH$ and approximately 75g of ice. An immediate pink crystalline solid

formed. The slurry was transferred to a separatory funnel and extracted with five 30 ml portions of $CH_2Cl_2$. The extracts were washed with two 40 ml portions of water and dried over $MgSO_4$. The solvent was removed under vacuum, leaving a rusty red crystalline solid, 0.48g (97%). The product was taken up in hot absolute ethanol, Norite A decolourising carbon added, and filtered through Celite to yield a yellow liquid. Slow cooling of the ethanolic solution gave very fine, pink cystals, m.p. 139. 1-139. 80 °C. TLC showed $R_f$ 0.25 on silica with 1:1 hexane: $CH_2Cl_2$ eluent. Anal . Calcd. for $C_{10}H_7BrClN$: C, 46.82; H, 2.75; N, 5.46; Br, 31.15; Cl, 13.82. Found: C, 46.97; H, 2.79; N, 5.42; Br, 31.08; Cl, 13.79.

D. 6-Bromo-2-dimethylamino-4-methylquinoline

A solution of 0.26g (.00101 mole) of 6-bromo-2-chloro-4-methylquinoline in 50 ml absolute ethanol was placed in a glass bomb liner, cooled to -78 °C, and 7.19g (0.159 mole) of dimethylamine bubbled into the cold ethanolic solution. The mixture was heated to 112° in a sealed autoclave for 3 hours. The solvent and excess dimethylamine were then removed under vacuum, to leave 0.36g of residue, which was washed with 40 ml $H_2O$ to remove the $(CH_3)_2NH.HCl$. The product was then taken up in 40 ml absolute ethanol, treated with Norite A alkaline decolourising carbon and filtered through Celite followed by concentration and addition of 7 ml $H_2O$ to induce crystallisation after chilling, the product collected was a light yellow crystalline solid, 0.20g (74.3%), m.p. 81.9-84.1 °C, and $R_f$ 0.11 on silica with 1:1 $CH_2Cl_2$:hexane. Anal . Calcd . $C_{12}H_{13}BrN_2$: C, 54.36; H, 4.94; N, 10.56; Br, 30.14. Found: C, 54.29; H, 4.98; N, 10.54; Br, 30.18

E. 2-Dimethylamino-6-formyl-4-methylquinoline

To a stirred solution of 0.30g (.00113 moles) of 6-bromo-2-dimethylamino-4-methyl-quinoline in 30 ml of dry (freshly distilled from over $LiAlH_4$) THF under $N_2$ at -78° (dry ice/acetone bath), 1.45ml (.00226 moles) of 1.56 M n-BuLi was added dropwise over a 5 minute period. 0.23 ml (.00297 moles) of dry DMF (freshly distilled from over $CaH_2$) was added to the reaction mixture in one portion. The dry ice/acetone bath was removed 12 minutes after the addition of DMF and the solution allowed to warm to -40°. The reaction mixture was poured onto 17 ml of 1N HCl in ice (22 minutes after addition of DMF) and extracted with 30 ml ether. The aqueous solution was made alkaline to pH 12 and extracted with three 20 ml portions of $CH_2Cl_2$. The yellow-orange coloured extract was washed with $H_2O$ and then dried over $MgSO_4$. The solvent was removed under vacuum, and the residue dried in a vacuum oven, wt 0.23g (95.8%), m.p. 96-99 °C. The product was recrystallised three times ($EtOH/H_2O$) . The TLC of the recrystallised product showed several spots, with the predominant component having an $R_f$ 0.38 in 2:1 hexane/ EtOAc and being fluorescent under long hot UV. The product was dissolved in 2:1 hexane/ EtOAc and placed on silica flash column. Fractions were collected; those containing the $R_f$ 0.38 product were combined, and the solvent removed in vacuo. The residue was recrystallised from absolute ethanol; weight 0.04g, (17%) m.p. 116.0-117.8 °C. Anal . Calcd. for $C_{13}H_{14}N_2O$: C, 72.87; H, 6.59; N, 13.07. Found: C, 72.74; H, 6.64; N, 13.05

F. 2,4-Diamino-5-(2-dimethylamino-4-methyl-6-quinolinylmethyl)-pyrimidine

To a solution of 0.5116g (0.00253 mole) of 2-dimethylamino-6-formyl-4-methylquinoline and 0.3703g 0.00253 moles) of 3-anilinopropionitrile in 12 ml dry DMSO was added 0.1450g (0.00268 moles) of $CH_3ONa$ in one portion. The temperature of the reaction mixture was then increased to 90 °C and maintained for 2½ hours. The hot brown-red solution was poured onto 50g ice to give a tan emulsion. The solvent was removed under vacuum and the residue partitioned between 100 ml water and three 20 ml portions of $CHCl_3$. The $CHCl_3$ extract was washed with $H_2O$ and evaporated to dryness in vacuo. The residue was taken up in absolute ethanol and filtered through a fritted glass funnel. The filtrate was refrigerated overnight, yielding 0.91g of solid product, which was again taken up in absolute ethanol and run through a silica pad to remove dark insoluble materials. The filtrate was concentrated under vacuum to leave a brown residue which was dried in a vacuum oven, wt 0.86g (94.5% m.p. 172-180 °C. A TLC using 2:1 hexane/ EtOAc as eluent showed the presence of two minor brightly fluorescent, spots with $R_f$'s 0.51 and 0.38 and two major spots of $R_f$'s 0.28 and 0.11. This product was not further purified but was used directly in the preparation of the pyrimidine.

A solution of free guanidine was prepared by mixing 0.54g (.010 moles)of $CH_3ONa$ and 0.82g (.00858 moles) of guanidine hydrochloride in 20 ml absolute ethanol. The NaCl was removed and resulting

guanidine solution was added to a stirred solution of 0.82g of the anilinopropionitrile adduct in 100ml of absolute ethanol under $N_2$. The reaction solution was refluxed for 4 hours. Approximately 50 ml of the solvent was then removed under vacuum and the solution cooled in an ice bath. A yellow crystalline product formed; 0.29g (32.9%). The product was taken up in 45 ml of 20:3 $CH_2Cl_2/CH_3OH$, placed on silica flash column (15.24 cm), and eluted with 20:3 $CH_2Cl_2/CH_3OH$ The intense yellow band on the column was collected to give 0.27g product (30.7%). This product was recrystallised from ethanol, yielding 0.19g (21.6%), m.p. 218.1-219.0 °C. TLC shows an $R_f$ of 0.14 on silica with 4:1 $CH_2Cl_2/CH_3OH$. Anal . Calcd. for $C_{17}H_{20}N_6$: C, 66.21; H, 6.54; N, 27.25 Found: C, 66.00; H, 6.57; N, 27.21.

## Example 25

### 2,4-Diamino-5-(6,7-dimethoxy-2,3-dimethyl-4-benzofuranylmethyl)pyrimidine

#### A. 2,4-Diamino-5-(3,4-dimethoxy-5-(1-methyl-2-oxopropoxy)benzyl)pyrimidine

To a solution of 2,76g (0.01 mole) of 2,4-diamino-5-(3-hydroxy-4,5-dimethoxybenzyl)-pyrimidine (D.E.Schwartz, W.Vetter, and G.Englert, Arzneim - Forsch (Drug Res.) 1970, 20 , 1867; G.Rey-Bellet and R.Reiner, Helv.Chim.Acta 1970, 53 , 945) in 40 ml of dry dimethyl sulfoxide was added 1.12g (0.01 mole) of potassium t-butoxide. To the resulting suspension was added in one portion 1.16g (0.0109 mole) of 3-chloro-2-butanone. The mixture was stirred at room temperature for 1 hour. The solvent was removed under vacuum and the residue was partitioned between 100 ml of methylene chloride and 100 ml of 0.1N sodium hydroxide. The organic layer was separated and the aqueous layer was extraced with an additional 100 ml of methylene chloride. The organic layers were combined, washed with 100 ml of water, dried ($MgSO_4$) and concentrated to give a quantitative yield (3.58g) of the title compound. Recrystallisation from 95% ethanol gave an analytical sample; mp 167.5-168.5 °. Anal . Calcd for $C_{17}H_{22}N_4O_4$; C, 58.95; H, 6.40; N. 16.17. Found: C, 58.77; H, 6.42; N, 16.13.

#### B. 2,4-Diamino-5-(6,7-dimethoxy-2,3-dimethyl-4-benzofuranylmethyl)pyrimidine

A mixture of 0.70g (0.002 mole) of 2,4-diamino-5-(3,4-dimethoxy-5(1-methyl-2-oxo-propoxy)benzyl)-pyrimidine in 11g of polyphosphoric acid was stirred and heated on a steam bath for 20 minutes then poured onto 100g of ice. The resulting mixture was basified with concentrated ammonium hydroxide, then extracted with methylene chloride (100 ml). The extract was washed with water (150 ml), dried over anhydrous magnesium sulfate and concentrated in vacuo to give the title compound (0.52g, 78%). Recrystallisation from absolute ethanol gave an analytical sample; mp> 249 ° dec. Anal . Calcd for $C_{17}H_{20}N_4O_3$: C, 62.18, H, 6.14; N, 17.06. Found: C, 62.01, H, 6.17, N, 17.01.

## Example 26

### 2,4-Diamino-5-(2,3-dihydro-6,7-dimethoxy-2-methyl-4-benzofuranylmethyl)-pyrimidine

#### A. 5-(3-Allyloxy-4,5-dimethoxybenzyl)-2,4-diaminopyrimidine

The title compound was prepared from 2,4-diamino-5-(3-hydroxy-4,5-dimethoxybenzyl)-pyrimidine (20.0g, 72.4 mmol) and allyl bromide by the procedure of Example 25A. Recrystallisation from 95% ethanol gave title compound as white crystals (14.9g); mp 160-162 °. Anal . Calcd for $C_{16}H_{20}N_4O_3$: C, 60.74; H, 6.37; N, 17.71. Found: C, 60.94; H, 6.45; N, 17.62.

#### B. 5-(2-Allyl-3-hydroxy-4,5-dimethoxybenzyl)-2,4-diaminopyrimidine

A mixture of 5-(3-allyloxy-4,5-dimethoxybenzyl)-2,4-diaminopyrimidine (14.6g, 46.2 mmol) and N,N-diethylaniline was maintained at 190 ° (oil bath) under nitrogen for 3.5 h. The resulting solution was cooled and the gummy preciptate slurried with 95% EtOH to give an off-white solid (5.1g), mp 213-214 ° dec, which TLC on silica gel with MeOH : $CH_2Cl_2$/1:4 showed to be identical to the analytical sample. One

recrystallisation from 95% EtOH gave white crystals of the title compound, mp 217-218° dec. Anal . Calcd for $C_{16}H_{20}N_4O_3$: C, 60.74; H, 6.37; N, 17.71. Found: C, 60.70; H, 6.40; N, 17.70. Chromatography of combined mother liquors gave an additional title compound (8.7g).

C. 2,4-Diamino-5-(2,3-dihydro-6,7-dimethoxy-2-methyl-4-benzofuranylmethyl)-pyrmidine

A mixture of 5-(2-allyl-3-hydroxy-4,5-dimethoxybenzyl)-2,4-diaminopyrimidine (1,50g, 4.74 mmol) and polyphosphoric acid (50g) was heated to 95° and maintained at this temperature with stirring until a clear pale yellow syrup was achieved (about 1 h). This syrup was poured into ice-water and the resulting solution basified with ammonium hydroxide. The resulting white solid (1.3g) was chromatographed on silica gel eluted with MeOH : $CH_2Cl_2$/1:9 to give the title compound as white powder (1.2g). Recrystallisation from 95% ethanol gave clusters of white needles, mp 216-219° . Anal . Calcd for $C_{16}H_{20}N_4O_3$ (316.37): C, 60.74; H, 6.37; N, 17.71. Found: C, 60.48; H, 6.41; N, 17.63.

Example 27

2,4-Diamino-5-(6,7-dimethoxy-2-methyl-4-benzofuranylmethyl)pyrimidine

A. 2,4-Diamino-5-(3,4-dimethoxy-5-(2-propynyloxy)benzyl)pyrimidine

The title compound was prepared from 2,4-diamino-5-(3-hydroxy-4,5-dimethoxy-benzyl)pyrimidine and propargyl chloride by·the procedure of Example 25A. Recrystallisation from 95% ethanol gave off-white needles (73%); mp 160-161° . Anal Calcd for $C_{16}H_{18}N_4O_3$: C, 61.13; H, 5.77; N, 17.82. Found: C, 60.96; H, 5.80; N, 17.75.

B. 2,4-Diamino-5-(6,7-dimethoxy-2-methyl-4-benzofuranylmethyl)pyrimidine

A mixture of 2,4diamino-5-(3,4-dimethoxy-5-(2-propynyloxy)benzyl)pyrimidine (1.18g, 3.75 mmol), potassium carbonate (0.518g, 3.75 mmol) and sulfolane (10 ml) was heated to 220-230° under nitrogen over a period of 20 minutes and then maintained at this temperature for an additional 15 minutes. The resulting dark mixture was cooled and the precipitate was adsorbed on silica gel. The title compound was eluted with MeOH : $CH_2Cl_2$/ 1:9 as an off-white powder, after recrystallisation from 95% ethanol; mp 209-212° . Anal . Calcd for $C_{16}H_{18}N_4O_3.\frac{1}{2}H_2O$: C, 59.43; H, 5.92; N, 17.33. Found: C, 59.22; H, 5.97; N, 17.31.

Example 28

2,4-Diamino-5-(1-methyl-3-indolylmethyl)pyrimidine

A. 3-Anilino-2-(1-methyl-3-indolylmethyl)acrylonitrile

To a solution under nitrogen of 6.5g (0.044 mole) of 3-anilinopropionitrile in 20 ml of dimethylsulfoxide was added 2.4g (0.044 mole) of sodium methoxide. After stirring for 5 min, 6.5g (0.041 mole) of 1-methylindole-3-carboxaldehyde (E.Wenkert, J.H.Udelhofen and N.K.Bhattacharya, J.Am.Chem.Soc ., 1959, 81 , 3763) was added to the mixture which was then heated at 135° for 30 min, followed by cooling and dilution with 200 ml of water. The resulting solid was collected, resuspended in 150 ml of water and collected again giving 9.14g (78%) of the title compound; the structure was confirmed by $^1$H-NMR.

B. 2,4-Diamino-5-(1-methyl-3-indolylmethyl)pyrimidine

To 100 ml of an ethanolic guanidine solution prepared from 2.10g (0.022 mole) of guanidine hydrochloride and 1.20g (0.022 mole) of sodium methoxide was added 5.00g (0.017 mole) of 3-anilino-2-(1-methyl-3-indolylmethyl)acrylonitrile. The solution was heated under reflux for ½ hour and then 100 ml of 2-methoxyethanol was added. The internal temperature was allowed to gradually increase to 120° by distillation of the

ethanol, after which it was heated at this temperature for 4.75 hours. The reaction was cooled and the solvent was removed in vacuo. The residue was recrystallised from 95% ethanol to give two crops of the title compound (total 1.82g, 41%) mp 253-256° dec. Anal . Calcd for $C_{14}H_{15}N_5$: C, 66.38, H, 5.97; N, 27.65. Found: C, 66.05; H, 6.04; N, 27.89.

Example 29

2,4-Diamino-5-(5-methoxy-1-methyl-3-indolylmethyl)pyrimidine

A. 3-Anilino-2-(5-methoxy-1-methyl-3-indolylmethyl)acryonitrile

The title compound was prepared from 5-methoxy-1-methylindole-3-carboxaldehyde (S.Misztal, Dissert.Pharm.Pharmacol ., 1972, 24 , 509) and 3-anilinopropionitrile by the procedure of Example 28A (71%); the structure was confirmed by $^1$H-NMR.

B. 2,4-Diamino-5-(5-methoxy-1-methyl-3-indolylmethyl)pyrimidine

The product of Example 29A was used in the procedure of Example 28B to give the title compound (49%) mp 195-198° dec. Anal . Calcd for $C_{15}H_{17}N_5O.1/4\ H_2O$: C, 62.59; H, 6.13; N, 24.33. Found: C, 62.48; H, 6.12, N, 24.29.

Example 30

2,4-Diamino-5-(3-benzo [ b ] thienylmethyl)pyrimidine

A. 3-Anilino-2-(3-benzo [ b ] thienylmethyl)acrylonitrile

The title compound was prepared from thianaphthene-3carboxaldehyde (W.J.King and F.F.Nord, J.Org.Chem . 1948, 13 635) and 3-anilinopropionitrile by the procedure of Example 28A (29%); mp 168-170° dec. Anal . Calcd for $C_{18}H_{14}N_2S$ : 0.1 $H_2O$: C, 73.99; H, 4.90; N, 9.59; S, 10.97. Found: C, 74.03; H, 5.21; N 9.59; S, 11.01.

B. 2,4-Diamino-5-(3-benzo [ b ] thienylmethyl)pyrimidine

The title compound was prepared from 3-anilino-2-(benzo/b/thienylmethyl)-acrylonitrile by the procedure of Example 28B (80%); mp 220-222°; the structure was confirmed by $^1$H-NMR. Anal . Calcd for $C_{13}H_{12}N_4S$ : C, 60.91; H, 4.72; N, 21.86; S,12.51. Found: C, 61.04; H, 4.78; N, 21.79; S, 12.56.

Example 31

Preparation of 2,4-Diamino-5-((6,7-dimethoxybenzo [ b ] thien-4-yl)methyl)pyrimidine

A. 1,2-Dimethoxy-3-(2,2-diethoxyethylthio)benzene

To a flask equipped with addition funnel, Gooch tube, thermometer, condensor, and nitrogen inlet, was added 6.71g (0.04 mol) of veratrole in 75 ml of dry tetrahydrofuran. A solution of n-butyl lithium (27.5 ml, 0.044 mol), 1.6 M in hexane, was added dropwise over a 20 minute period at room temperature. The mixture was stirred for two hours, and chilled to 5° C, followed by the addition of 1.41g (0.044 mol) of sulfur from an Elenmeyer flask attached to the Gooch tube. The mixture was allowed to reach room temperature and stirred for 30 minutes, followed by the addition of 6.62 ml (0.044 mol)of bromoacetaldehyde diethyl acetal. This mixture was allowed to stir at room temperature for three hours, and then heated at 80° for 17 hours. The reaction mixture was then added to 100 ml of water. The tetrahydrofuran layer was separated and evaporated. There remained 11.9g of dark brown oil, which was purified by column chromatography on

silica gel, using heptane:dichloromethane/1:4, then dichloromethane, then chloroform:methanol/19:1, which gave 5.47g (48%) of compound A as an oil. Anal Calcd for $C_{14}H_{22}SO_4$: C, 58.72; H, 7.74; S, 11.20. Found: C, 58.61; H, 7.79; S, 11.27. MS: 286 ($M^+$). NMR, ($CDCl_3$) 1.17 (tr, 6,( $CH_2CH_3$)2); 3.08 (d, 2, $CH_2CH$); 3.58 (d-qt, 4, (O $CH_2Me$)2); 3.82 (d, 6, (O Me )2); 4.64 (tr, 1, $CH_2CH$ ); 6.91 (s plus sh, 3, Ar).

B. 6,7-Dimethoxybenzo [ b ] thiophene

To 2.04g (7.1 mmol) of compound A 30 ml of dioxane was added 0.5 ml of concentrated sulfuric acid, in a nitrogen atmosphere. The mixture was heated at 100°C for 1.5 hours, followed by the addition of 0.3 ml more of concentrated sulfuric acid. After heating for an additional hour, the mixture was cooled, followed by the addition of 50 ml of water. The mixture was neutralised with concentrated ammonium hydroxide and extracted into dichloromethane, followed by drying over magnesium sulphate, and removal of the solvent. The product was purified by column chromatography on silica gel, using hexane:dichloromethane/2:1, which resulted in the separation of 1.02g (60%) of product B as an oil. Anal . Calcd for $C_{10}H_{10}O_2S$: C, 61.83; H, 5.19; S,16.51. Found: C, 61.91; H, 5.25; S, 16.41. MS: 194 ($M^+$), 179 (M-Me). NMR ($CDCl_3$): $\delta$ 3.93 (s, 3, OMe); 4.03 (s, 3, OMe); 7.06 (d, 1, ArH, J = 8.6); 7.25 (d, 2, CH=CH,, J = 5-6); 7.48 (d, 1, ArH, J = 8.6 Hz).

C. 2,4-Diamino-5- ((6,7-dimethoxybenzo [ b ] thien-4-yl)methyl) pyrimidine

A mixture of 0.45g (2.3 mmol) of product B and 0.32g (2.3 mmol) of 2,4-diamino-5-hydroxymethyl-pyrimidine was added to 10 ml of glacial acetic acid and 0.4 ml (4.6 mmol) of concentrated hydrochloric acid, and refluxed for 6 hours. The solvents were evaporated, 50 ml of water was added, and the mixture was neutralised with concentrated aqueous ammonia to about pH 9. The product was extracted into dichloromethane:methanol/3:1 three times using 50 ml portions, followed by drying over $MgSO_4$, filtration, and evaporation of the solvents. The residue, 0.53g, was purified by column chromatography on silica gel, using hexane:dichloromethane/1:1, dichloromethane, and then dichloromethane:methanol/9:1 and 5:1. A 0.19g fraction of recovered starting material (B) was recovered, and 0.22g of a mixture containing the title compound as ascertained by NMR spectroscopy. The mixture was separated by fractional crystallisation from methanol. After separating the least soluble fraction, the more soluble fraction was concentrated and allowed to stand overnight. A crystalline product was isolated with a mp of 230-231°. This was shown to be essentially a single substance by NMR spectroscopy; the pyrimidylmethyl attachment was found to be in the benzene ring, since there was a loss of one of the ortho -coupled protons with a J value of 8.6, whereas the pair of doublets from the thiophene ring remained (J = 5.44 Hz). Nuclear Overhauser NMR studies led to the assignment of the title structure (CI) as the 4-substituted isomer. NMR ($Me_2SO$-$d_6$) $\delta$ 3.84 (s, 3,7-OMe); 3.89 (s, 5,6-OMe plus $CH_2$); 5.72 (br, s, 2, pyrimidine $NH_2$); 6.23 (br, s, 2, pyrimidine $NH_2$); 7.05 (s, 1, ArH-5); 7.34 (s, 1, pyrimidine H-6); 7.39 (d, 1 ArH-3, J = 5.44 Hz); 7.55 (s, 1, ArH-3, J = 5.44 Hz). Irradiation of the singlet at 3.89 ppm (6-OMe plus $CH_2$) resulted in N.O.E. enhancements for H(3), H(5), and H(6'), indicating that this is the desired compound.

Example 32

2,4-Diamino-5-((6,7-dimethoxybenso [ b ] thieno-4-yl)methyl)pyrimidine

A. 3-(2,2-diethoxyethylthio)-4,5-diemethoxybenzaldehyde

Morpholine (2.61g, 0.03 mol) and 50 ml of dry tetrahydrofuran were placed in a flame-dried three neck flask under nitrogen, and chilled to -70°C. Then n-butyl lithium (20.6 ml, 0.033 mol, 1.6 M in hexane) was added dropwise via an addition funnel, keeping the temperature at -70°. Then 5-bromo-3,4-dimethoxybenzaldehyde (0.03 mol) dissolved in 25 ml of tetrahydrofuran was added slowly, and then the reaction was stirred for 1 hour at -50°. A second equivalent of n-butyl lithium (20.6 ml, 0.033 mol) was then added, followed by sulfur (1.06g, 0.033 mol) via a Gooch tube, and the reaction was stirred for 1 hour. Then the reaction was poured onto cold water, acidified to pH 5 with 1 N hydrochloric acid, and extracted with ethyl acetate and evaporated to give 3.35g (56% yield) of crude 3,4-dimethoxy-5-mercaptobenzaldehyde.

The crude mercaptobenzaldehyde from above (2.78g, 14 mmol) was slurried in 1.25 ml of absolute

ethanol under nitrogen. The sodium methylate (0.82g, 15.4 mmol) was added, the reaction was stirred 10 minutes, followed by the addition of 2.69g (14 mmol) of bromoacetaldehyde-diethylacetal. The reaction was reluxed overnight, poured into water, and extracted with ethyl acetate and evaporated to give 2.77g of an orange oil. This was purified on a silica gel column eluted with hexane:ethyl acetate/19:1 to give 1.5g of the title compound. NMR: ($Me_2SO-d_6$) δ 1.16 (tr, 6, $Me_2$), 3.19 (d, 2, $SCH_2$), 3.63 (double quartet, 4, ( $CH_2Me$)-$_2$), 3.88 (s, 3, OMe), 3.94 (s, 3, OMe), 4.69 (tr, 1, CH -$CH_2$), 7.43 (d, 1, Ar), 7.56 (d, 1, Ar), 9.91 (s, 1, CHO). Anal . Calcd for $C_{15}H_{22}O_5S$: C, 57.30; H, 7.05; S, 10.20. Found: C, 57.40; H, 7.05; S, 10.13.

B. 2,4-Diamino-5-(3-(2,2-diethoxyethylthio)-4,5-dimethoxybenzyl)pyrimidine

The aldehyde from above was converted to 2-(3-(2,2-diethoxyethylthio)-4,5-dimethoxy-benzyl)-3-anilinoacrylonitrile with anilinopropionitrile and sodium methylate in dimethyl sulfoxide on a 2.5 mmol scale in the same manner as in Example No.28A. The crude product from this reaction was condensed with guanidine hydrochloride and sodium methylate in ethanol as in Example 28B to give the crude product. After purification on a silica gel column eluting with ethyl acetate:methanol/9:1 to give 0.51g (62% yield), followed by recrystallisation in 30% ethanol/water 0.15g of the title compound was obtained, mp 117-118°. NMR: ($CDCl_3$) δ 1.19 (tr, 6, $Me_2$), 3.07 (d, 2, S $CH_2CH$), 3.61 (double quartet, 4, ( $CH_2Me)_2$), 3.79 (s, 3, OMe), 3.83 (s, 3, OMe), 4.53 (br, s, 2, $NH_2$), 4.63 (tr, 1, $SCH_2CH$ ), 4.68 (br, s, 2, $NH_2$), 6.52 (d, 1, Ar), 6.75 (d, 1, Ar), 7.76 (s, 1, pyrimidine-$H^6$). Anal . Calcd for $C_{19}H_{28}N_4O_4S$: C, 55.86; H, 6.91; N, 13.71; S, 7.85. Found: C, 55.82; H, 6.92; N, 13.69; S, 7.87.

C. 2,4-Diamino-5-((6,7-dimethoxybenzo [ b ] thieno-4-yl)methyl)pyrimidine

The product from section B above (0.31g, 0.75 mmol) was refluxed in 25 ml of water and 5 ml of ethanol under nitrogen, to which was added 0.6 ml of concentrated sulfuric acid in 0.2 ml portions at 0, 3/4 hour, 1.5 hour, and then heated for ½ hour longer. The reaction was neutralised to pH 9.5, extracted with methylene chioride:methanol/3:1, and evaporated. The crude product was purified on a silica gel column eluting with 3% methanol in methylene chloride to give 0.045g of product. NMR: ($Me_2SO-d_6$) δ 3.84 (s, 3, OMe), 3.89 (s, 3, OMe), 5.68 (br, s, 2, $NH_2$), 6.17 (br, s, 2, $NH_2$), 7.05 (s, 1, Ar), 7.35 (s, 1, pyrimidine-$H^6$), 7.39 (d, 1, thieno-H J=5.5 Hz), 7.55 (d, 1, thieno-H, J=5.5 Hz). MS 316 ($M^+$). Anal . Calcd for $C_{15}M_{16}N_4O_2S$: C, 56.95; H, 5.10; N, 17.71. Found: C, 56.88; H, 5.14; N, 17.62.

A sample from a larger scale reaction was recrystallised as the hydrochloride salt from absolute ethanol to give title compound hydrochloride, mp 280-283° C. Anal . Calcd for $C_{15}H_{16}N_4O_2S.HCl$: C, 51.06; H, 4.86; N, 15.88. Found: C, 50.96; H, 4.91; N, 15.81.

Example 33

2,4-Diamino-5-(4-quinolylmethyl)pyrimidine

A. 3-Anilino-2-(4-quinolylmethyl)acrylonitrile

To a solution of 10.0g (0.064 mole) of 4-quinolinecarboxaldehyde and 10.67g (0.073 mole) of 3-anilinopropionitrile in dimethylsulfoxide (25 ml) was added a solution of 3.44g (0.064 mole) of sodium methoxide in methanol (25 ml). The solution was heated to reflux and the methanol was allowed to distill off. After heating for 1 hour, the reaction was cooled, diluted with water (100 ml) and gummy solid separated. This was triturated with methylene chloride (100 ml) to give the title compound (1.47g 8%). The structure was confirmed by NMR spectrometry.

B. 2,4-Diamino-5-(4-quinolylmethyl)pyrimidine

To 11 ml of an ethanolic guanidine solution prepared from 0.62g (6.5 mmol) of guanidine hydrochloride and 0.36g (6.7 mmol) of sodium methoxide was added 1.47g (5.2 mmol) of 3-anilino-2-(4-quinolylmethyl)-acrylonitrile. The solution was heated under reflux for 2 hours and then 11 ml of 2-methoxyethanol were added. The internal temperature was allowed to gradually increase to 128° by distillation of the ethanol,

after which it was heated at this temperature for 1 hour. The reaction was cooled and the solid that precipitated was collected and recrystallised from 95% ethanol in the presence of hydrochloric acid giving the dihydrochloride of the title compound, 1.27g (75%); mp 300°C dec. Anal . Calcd for $C_{14}H_{13}N_5.2HCl$: C, 51.87; H, 4.66; N, 21.60;

Example 34

2,4-Diamino-5-(4-isoquinolylmethyl)pyrimidine

A. 3-Anilino-2-(4-isoquinolylmethyl)acrylonitrile

To a solution under nitrogen of 3.18g (21.7 mmol) of 3-anilinopropionitrile in 10 ml of dimethylsulfoxide was added 1.17g (21.7 mmol) of sodium methoxide. The resulting suspension was stirred for 5 minutes, then 3.1g (20 mmol) of 4-isoquinolinecarboxyaldehyde (J.B.Wommack, T.G.Barbee, Jr., D.J.Thoennes, M.A.McDonald and D.E.Pearson, J.Heterocyclic Chem ., 1969, 6, 243) was added and the mixture was heated at 130° for 30 minutes. The reaction was cooled, diluted with a 30:1 mixture of water:ethanol and the gummy solid that separated was collected, dissolved in ethyl acetate, washed twice with water, dried over anhydrous magnesium sulfate and concentrated to an oily solid. This was triturated with methylene chloride-ethyl acetate to give 0.94g (17%) of the title compound. The structure was confirmed by NMR and IR spectrometry.

B. 2,4-Diamino-5-(4-isoquinolylmethyl)pyrimidine

The title compound was prepared from 3-anilino-2-(4-isoquinolylmethyl)acrylonitrile following the procedure of Example 33B (86%). An analytical sample was obtained by recrystallisation from ethanol-2-methoxyethanol; mp. 309-313°C (dec.) for $C_{14}H_{13}N_5$: C, 66.92; H, 5.21; N, 27.87. Found: C, 66.77; H, 5.27; N, 27.80.

Example 35

Preparation of 6-Bromo-4,8-dimethyl-2-(1H)-quinolinone

Part 1 To a stirred solution of 20g (0.107 moles) of 4-bromo-2-methylaniline in 25 ml of toluene was added 9.0g (0.107 moles) of diketene. The solution was refluxed for 2 hours. The toluene was removed in vacuo and the residue dissolved in $CH_2Cl_2$. The organic solution was extracted with 2 x 250 ml portions of 1N HCl, washed with 300 ml of water, and dried over $MgSO_4$. Removal of the $CH_2Cl_2$ left 24.79g (85.4%) of the product N-(4-bromo-2-methylphenyl)-3--oxobutyramide. The product was recrystallised from ethanol/water to yield 24.0g (82.6%) of yellow crystalline material with an $R_f$ = .38 on silica with 1% $CH_3OH$ in $CH_2Cl_2$ eluent. The product was used directly in the preparation of the 6-bromo-4,8-dimethyl-2(1 H )-quinoline without further purification or analysis.

Part 2 A mixture of 24.0g (.0888 moles) of the product above in 80 ml of concentrated $H_2SO_4$ was heated at 95° ($H_2O$ bath) for 1¼ hours. The resulting mixture was cooled to room temperature and poured onto ice. The tan solid that formed was collected and washed repeatedly with $H_2O$ and dried in vacuo at 100°C. The product was recrystallised from hot DMF to yield 13.62g (60.8%) of tan crystals, m.p. = 246-250°C, $R_f$ = 0.25 on silica TLC with 1% $CH_3OH$ in $CH_2Cl_2$ eluent. Elemental analysis as calculated for $C_{11}H_{10}BrNO$, M.W. 252.118:

Calc: C, 52.41;H, 4.00; N, 5.56 Br, 31.70
Found: C, 52.37; H, 4.05; N, 5.54 Br, 31.63.

Preparation of 6-Bromo-2-chloro-4,8-dimethylquinoline

A solution of 13.52g (0.054 moles) of 6-bromo-4,8-dimethyl-2(1 H )-quinolinone in 60 ml of $POCl_3$ under $N_2$ was refluxed for 5.5 hours and then cooled to yield a yellow precipitate. The solid was collected and washed in ice water to remove $POCl_3$. A white crystalline product resulted, weight 9.38g. The initial filtrate

was poured onto a vigorously stirring slurry of concentrated $NH_4OH$ and ice to generate a tan solid, weight 4.52g. The overall % yield for the reaction was 100%. The first product isolated was sent for elemental analysis, m.p. 168.5-170.5° C, and showed an $R_f$ = 0.78 on silica TLC with 4:1/hexane:ethyl acetate eluent. Elemental analysis as calculated for $C_{11}H_9BrClN$, M.W. 270.563:

Calc: C, 48.83; H, 3.35; N, 5.10; Br, 29.53; Cl, 13.10

Found: C, 48.71; H, 3.39; N, 5.17; Br, 29.48; Cl, 13.08.

Preparation of 6-Bromo-4,8-dimethyl-2-(morpholin-1-yl)quinoline

A solution of 3.0g (0.011 moles) of 6-bromo-2-chloro-4,8-dimethylquinoline in 30 ml of morpholine under $N_2$ was refluxed for 2.5 hours. The solution was cooled to yield 2 crops of white crystals, weight 3.50g (98.3%). The product showed an $R_f$ = 0.38 on silica TLC with $CH_2Cl_2$ eluent. The first crop was sent for elemental analysis, m.p. = 155-58° C. Elemental analysis as calculated for $C_{15}H_{17}BrN_2O$, M.W. 321.225:

Calc: C, 56.09; H, 5.33; N, 8.72; Br, 24.88

Found: C, 56.09; H, 5.35; N, 8.69; Br, 24.94.

Preparation of 4-8-Dimethyl-2-(morpholin-1-yl)-6-quinolinecarbaldehyde

To a stirred solution of 0.70g (0.00218 moles) of 6-bromo-4,8-dimethyl-2-(morpholin-1--yl)-quinoline in 30 ml of freshly distilled THF under $N_2$ at -78° C was added 2.81 ml (0.00450 moles) of 1.6 M nBuli dropwise over a 5 minute period. The solution was stirred for 10 minutes and 0.36 ml (0.00470 moles) of freshly distilled DMF was added in one portion. The reaction solution was continued to stir at -78° C for 30 minutes and then poured onto 10 ml 1N HCl and ice to quench the reaction. The acidic solution was extracted with 3 x 25 ml portions of $CH_2Cl_2$. The organic extract was washed with $H_2O$ and dried over $MgSO_4$. After the solvent was removed under vacuum, the crude product, weight 0.45g (76.3%), was dissolved in approximately 10 ml of 20:1/ $CH_2Cl_2$:EtOAc, placed on an 8 inch silica flash column, and eluted with the solvent. The desired product showed a brightly fluorescent spot of $R_f$ = 0.35 on TLC with 20:1/ $CH_2Cl_2$:EtOAc . The flash column fractions containing this $R_f$ were collected and combined to give 0.35g (59.3%) of product. A portion of this product was recrystallised from absolute EtOH to yield light yellow crystals with a m.p. = 161.5-162.5° C and a single spot on TLC with $R_f$ = 0.35. Elemental analysis as calculated for $C_{16}H_{18}N_2O_2$, M.W. 270.333:

Calc: C, 71.09; H, 6.71; N, 10.36

Found: C, 70.93; H, 673; N, 10.31.

Preparation of 2,4-Diamno-5-(4-8-dimethyl-2-morpholin-1-yl-6-quinolinylmethyl)-pyrimidine

Part 1 To a stirred solution of 0.95g (0.00351 moles) of 4,8-dimethyl-2-(morpholin-1-yl)-6-quinolinecarbaldehyde and 0.54g (0.00396 moles) of 3-anilinopropionitrile in 12 ml dry DMSO under $N_2$ was added 0.21g (0.00386 moles) of sodium methoxide in one portion. The temperature of the reaction mixture was raised to 100° C and maintained with stirring under $N_2$ for 2 hours. The solution was then poured onto approximately 50g ice and stirred to yield tan crystals. The crystalline product was collected and washed with several portions of $H_2O$. The crude product was recrystallised from absolute ethanol to yield 4 crops of light yellow-tan crystals, weight 0.76g (54.3%). The first of these crops, weight 0.58g (41.4%), m.p. = 192-96° C, showed two predominant spots on TLC with 2:1/hexane: EtOAc of $R_f$'s 0.41 and 0.33. The later 3 crops contained these same $R_f$'s plus another spot of $R_f$ = 0.57. NMR indicated the expected product. No further purification was performed on the product.

Part 2 To a solution of 0.64g (0.00161 moles) of the product from the previous step in 70 ml absolute EtOH under $N_2$ was added a guanidine solution obtained by reaction of 0.46g (0.00482 moles) of guanidine hydrochloride and 0.43g (.00805 moles) of sodium methoxide (with NaCl removed by filtration). The resulting solution was refluxed while being monitored by TLC. After 4 hours the reaction did not appear to be generating any further product and therefore 0.15g (1 equivalent) of guanidine hydrochloride and 0.13g (1.5 equivalent) of sodium methoxide were reacted in 5 ml absolute EtOH , the NaCl removed, and the resulting solution added to the reaction. The reaction was then refluxed an additional 1.5 hours with little or no detectable change in the amount of product as determined by TLC. The solvent was removed *in vacuo* to leave a red-orange residue which was dissolved in 10:1/ $CH_2Cl_2$:$CH_3OH$ placed on a silica flash column

(12 inches), and eluted with like solvent. Fractions containing material of $R_f$ = 0.34 on TLC were combined to give 0.25g product (42.4%), m.p. = 225-233° C. This product was repeatedly recrystallised from absolute ethanol with decolourising carbon to remove a minor yellow component of $R_f$ = 0.25 which could not be eliminated by the chromatographic procedure. The final product was a white crystalline material, weight 0.013g (2.20%), m.p. = 248-51° C, and a single spot on TLC. Elemental analysis as calculated for $C_{20}H_{24}N_6O$, M.W. 364.473:

Calc: C, 65.91; H, 6.64; N, 23.06
Found: C, 63.19; H, 6.50 N, 22.04
NMR is consistent with structure.

Example 36

2,4-diamino-5-(2-methoxy-4-methyl-7-quinolylmethyl)pyrimidine

N-(3-Bromophenyl)-3-oxobutyramide

To a heated (80° C, oil bath temperature)solution of 20.00g (0.12 M) of m-bromoaniline in 200 ml of dry toluene was added dropwise over a period of 30 min. 12g (0.14 M) of diketene in 100 ml of dry toluene. When the addition was completed, the reaction mixture was brought to reflux for 5 hours. The toluene was then removed in vacuo, resulting in a yellow solid. Recrystallisation from toluene afforded 14.70g (48%) of product as light pink crystals: m.p. 94-95° C; NMR ($Me_2SO$-$d_6$) δ 11.10 (br s, 1 H), 7.95 (dd, 1 H, J = 2 Hz), 7.65-7.15 (m, 3 H), 3.55 (s, 2 H), 2.20 (s, 3 H).

Anal . Calcd. for $C_{10}H_{10}BrNO_2$: C, 46.90; H, 3.94; N, 5.47; Br, 31.20.
Found: C, 47.02; H, 3.95; N, 5.44; Br, 31.11.

2-Hydroxy-4-methyl-7-bromoquinoline

6.659 (26.00 mM) of N -(3-bromophenyl)-3-oxobutyramide was heated in 30 mL of conc. sulfuric acid to 120° C (oil bath temperature) for 1.5 hours. The reaction mixture was then poured into ice, whereby precipitate was formed. This was filtered and washed repeatedly with water. After drying, it was recrystallised in 95% ethanol, providing 5.21g (84%) of the product as a white solid: mp 275-276° C; NMR ($Me_2SO$-$d_6$) δ 11.65 (br s, 1 H), 7.60 (d, 1 H, J = 9 Hz), 7.45 (d, 1H, J = 2 Hz), 7.30 (dd, 1 H, J = 9, 2 Hz), 6.70 (d, 1 H J= 1 Hz), 2.35 (d, 3 H, J= 1 Hz).

Anal . Calcd. for $C_{10}H_8BrNO$: C, 50.45; H, 3.37; N, 5.88; Br, 33.56.
Found: C, 50.46; H, 3.39; N, 5.84; Br, 33.56.

2-Chloro-4-methyl-7-bromoquinoline

5.00g of 2-hydroxy-4-methyl-7-bromoquinoline was refluxed in 50 ml of phosphorus oxychloride for 2 hours. The reaction mixture was then poured into a mixture of ice and conc. ammonium hydroxide. The precipitate resulting from this treatment was filtered and then washed repeatedly with water. It was then taken up in 95% ethanol and heated. Undissolved impurities were filtered, and the filtrate was allowed to crystallise. 3.82g (71%)of the product was obtained as white crystals: mp 73-74° C; NMR ($Me_2SO$-$d_6$) δ 8.17 (d, 1 H, J = 2 Hz), 8.10 (d, 1 H, J = 9 Hz), 7.75 (dd, 1 H, J = 9, 2 Hz), 7.55 (d, 1 H, J = 1 Hz), 2.70 (d, 3 Hz J = 1 Hz).

Anal Calcd. for $C_{10}H_7BrClN$: C, 46.82; H, 2.75; N, 5.46.
Found: C, 46.85; H, 2.79; N, 5.42.

2-Methoxy-4-methyl-7-bromoquinoline

A mixture of 3.00g (12.00 mN) of 2-chloro-4-methyl-7-bromoquinoline and 0.63g (12.00 mN) of sodium methoxide was heated to reflux in 30 ml of dry methanol for 48 hours. The methanol was then removed in vacuo, and the resultant concentrate was taken up in methylene chloride. This methylene chloride solution was washed repeatedly with saturated sodium chloride. After drying MgSO₄), solvent removal, and

recrystallisation from toluene, 2.63g (87%) of the product was obtained as white crystals: m.p. 58-60° C; NMR ($Me_2SO-d_6$) δ 7.80 (d, 1 H J= 9 Hz), 7.85 (d, 1 H, J = 2 Hz), 7.55 (dd, 1 H, J= 9,2 Hz), 6.80 (d, 1 H, J = 1 Hz), 3.80 (s, 3 H), 2.55 (d, 3 H, J = 1 Hz). Anal . Calcd. for $C_{11}H_{10}BrNO$: C, 52.40; H, 4.00; N, 5.56; Br, 31.70. Found: C, 52.25; H, 4.00; N, 5.51; Br, 31.59.

2-Methoxy-4-methyl-7-quinolinecarbaldehyde

In a 50 ml flame-dried 3-neck round-bottom flask was dissolved under nitrogen atmosphere 1.50g (5.90 mM) of 2-methoxy-4-methyl-7-bromoquinoline in 20 ml of freshly distilled THF. This THF solution was then cooled to -76° C (dry ice/acetone), followed by the dropwise addition (via a syringe) of 7.65 ml of n-BuLi (1.56 M of n-Buli in hexane). After stirring for 5 minutes 1.26 ml (16.00 mM) of dry N , N -dimethylformamide was added via a syringe. The reaction mixture was then brought to -20° C. Water was slowly added, then 1N HCl. Following ether extraction and recrystallisation from toluene, 0.90g (76%) of the product was obtained as a white solid: mp 111-112° C; NMR ($Me_2SO-d_6$) δ 10.24 (s, 1H), 8.37 (d, 1 H, J = 2 Hz), 8.15 (d, 1 H, J = 8 Hz), 7.85 (dd, 1 H, J = 8, 2 Hz), 7.08 (br. s, 1 H), 4.00 (s, 3 H), 2.65 (d, 3 H, J = 1 Hz).

Anal . Calcd. for $C_{12}H_{11}NO_2$: C, 71.62; H, 5.51; N, 6.96.
Found: C, 71.48; H, 5.55; N, 6.92.

2-(2-Methoxy-4-methyl-7-quinolylmethyl)-3-anilinoacrylonitrile

To a stirred mixture of 0.65g (3.20 mM) of 2-methoxy-4-methyl-7-quinolinecarbaldehyde and 0.52g (3.50 mM) of anilinopropionitrile in 10 ml of dry dimethyl sulfoxide was added in one portion 0.19g (3.50 mM) of sodium methoxide. The resultant mixture was then heated to 90-95° C (internal temperature) for 2 hours after which the dimethyl sulfoxide was removed in vacuo. Addition of distilled water to the resultant concentrate resulted in a brown precipitate. This was filtered, washed repeatedly with water and air-dried. Recrystallisation from absolute ethanol resulted in 0.49g (46%) of product as a light brown solid; m.p. 170-172° C.

Anal . Calcd. for $C_{21}H_{19}N_3O$: C, 76.57; H, 5.81; N, 12.76.
Found: C, 76.34; H, 5.83; N, 12.60.

2,4-Diamino-5-(2-methoxy-4-methyl-7-quinolylmethyl)pyrimidine

0.17g (1.82 mM) of guanidine hydrochloride and 0.13g (2.44 mM) of sodium methoxide were stirred for 5 minutes under a nitrogen atmosphere. The sodium chloride salt formed was then filtered and the filtrate was added to a round-bottom flask containing 0.20g (0.61 mM) of 2-(2-methoxy-4-methyl-7-quinolylmethyl)-3-anilino-acrylonitrile. This resultant mixture was heated to reflux for overnight. An equimolar amount of guanidine as above (after treatment with sodium methoxide) was further added and the reaction mixture was refluxed for an additional 24 hours. The absolute ethanol was then removed in vacuo. To the resultant concentrate was added methanol/water (9:1). Brown precipitate was formed. This was collected and dried. Following two flash column chromatographies (20-26g of silica gel, 230-400 mesh, methylene chloride : methanol - 9:1), 0.025g (14%) of the product was obtained as an off-white solid: m.p. 205-206° C; NMR ($Me_2SO-d_6$) δ 7.90 (d, 1 H, J = 8.50 Hz), 7.65 (s,1 H), 7.60 (d, 1 H, J = 2 Hz), 7.35 (dd, 1 H, J = 8.50, 2 Hz), 7.85 (d, 1 H, J = 1 Hz), 6.16 (br s, 2 H), 5.76 (br s, 2 H), 3.92 (s,3 H), 3.79 (s,2 H), 2.57 (d, 3 H, J = 1 Hz).

Anal . Calcd. for $C_{16}H_{17}N_5O.3/5H_2O$: C, 62.77; H, 5.99; N, 22.88.
Found: C, 62.88; H,, 5.96; N, 22.91.

Example 37 Tablets

| Ingredient | Amount per tablet (mg) Single Active Ingredient | Combination |
|---|---|---|
| 2,4-Diamino-5-(6,7-dimethoxy-2-methyl-4-benzofuranylmethyl) pyrimidine | 100.0 | 80.0 |
| Sulfamethoxazole | --- | 400.0 |
| Lactose | 84.0 | 100.0 |
| Potato starch, dried | 14.3 | 18.0 |
| Magnesium stearate | 0.7 | 1.0 |
| Polyvinylpyrrolidone | 1.0 | 1.0 |

The 2,4-diamino-5-(6,7-dimethoxy-2-methyl-4-benzofuranylmethyl)pyrimidine, lactose and potato starch (and sulfamethoxazole in the combination formulation) are mixed together and then granulated with aqueous polyvinylpyrrolidone. The granules are dried, mixed with the magnesium stearate and then compressed to produce tablets weighing 200 mg each (single active ingredient) or 600 mg each (combination).

Example 38 Tablets

| Ingredient | Amount per tablet (mg) Single Active Ingredient | Combination |
|---|---|---|
| 2,4-Diamino-5-(1-methyl-3-indolylmethyl)-pyrimidine | 100.0 | 80.0 |
| Sulfisoxazole | --- | 160.0 |
| Lactose | 149.0 | 79.0 |
| Corn starch | 149.0 | 79.0 |
| Stearic acid | 2.0 | 2.0 |

The ingredients are thoroughly mixed and then loaded into hard gelatin capsules containing 400 mg each.

Example 39

2,4-Diamino-5-(1,2-dihydro-2,2,4-trimethyl-6(1H)quinolylmethyl)pyrimidine dihydrochloride

1,2-Dihydro-2,2,4-trimethylquinoline (1.73g, 10 mmol) was treated by the method of Example 1 with 2,4-diamino-5-hydroxymethylpyrimidine and worked up in the same manner. The crude product was purified on a silica gel column eluting with methylene chloride:methanol/19:1, followed by recrystallisation in ethanol with 2 equivalents of hydrochloric acid to give the title compound; mp 260-264°. Anal. Calcd. for $C_{17}H_{21}N_5.2HCl.\frac{1}{2}H_2O$: C, 54.12; H, 6.41; N, 18.56. Found: C, 54.40; H, 6.51; N, 18.17.

Example 40

2,4-Diamino-5-(1,2,3,4-tetrahydro-1-quinolylmethyl)pyrimidine

To a solution of 1,2,3,4-tetrahydroquinoline (2.72g, 20.4 mmol) in ethanol (25 ml) was added 2,4-diamino-5-bromomethylpyrimidine hydrobromide (2.00g 5.1 mmol from elemental analyses and $^1$H-NMR) (L.T.Weinstock, D.E. O'Brien and C.C. Cheng, J.Med.Chem.1968, 11, 1238). The mixture was stirred at 25° for 18 hours. The resulting tan precipitate (1.55g) was slurried with concentrated ammonium hydroxide (2 ml)-methanol (25 ml) and chromatographed on a silica gel column. Elution with $CH_2Cl_2$:MeOH /7:1 gave a white solid (0.65g). Recrystallisation from 95% ethanol gave the title compound as a white solid (0.47g, 36%); mp 214-215° dec. Anal. Calcd. for $C_{14}H_{17}N_5$: C, 65,86; H, 6.71; N, 27.43. Found: C, 65.61; H, 6.84; N, 27.52.

Example 41

2,4-Diamino-5-(4-quinolylmethyl)pyrimidine dihydrochloride

The procedure used was that of R.A. Swaringen, Jr., D.A.Yeowell, J.C.Wisowaty, H.A.El Sayad, E.L.Stewart, and M.E.Darnofall, J.Org.Chem.1979, 44 , 4825. Quinoline 4-carboxyaldehyde (30.0g, 0.187 mole), ethylcyanoacetate (21.2g, 0.187 mole) and piperidine (0.95 ml) in benzene (75 ml) were refluxed with removal of water by a Dean-Stark trap for 1 hour. Solvent was removed and the residual white solid dissolved in absolute ethanol (500 ml)and shaken with 10% Pd/C (1.0g) under $H_2$ (50 psi, 345 KPa) for 18 hours. Filtration and evaporation, followed by elution from a silica gel pad with methylene chloride, gave ethyl (4-quinolylmethyl)cyanoacetate as a pink oil (22.2g, 47%); the structure was confirmed by $^1$H-NMR. This material was refluxed in triethyl-orthoformate (200 ml) for 6 hours, with slow collection of distillate (about 50 ml collected during reflux period). The remaining triethylorthoformate was evaporated and the residual purple oil passed through a silica gel pad eluted with methylene chloride to give 2-(ethoxycarbonyl)-2-(diethoxymethyl)-3-(4-quinolyl)propionitrile as a chromatographically homogeneous oil (20.87g, 67%); the structure was confirmed by $^1$H-NMR. 2-(Ethoxycarbonyl)-2-(diethoxymethyl)-3-(4-quinolyl)propionitrile (10.0g, 28.0 mmol), potassium hydroxide (85%, 1.85g, 28 meq) and 2-methoxyethanol (100 ml) were refluxed for 1 hour. Guanidine hydrochloride (5.35g, 56.0 mmol) and sodium methoxide (3.02g, 56.0 mmol) were added and reflux continued for an additional 2 hours. The reaction was evaporated to dryness and the residual solids triturated with water to leave a tan solid. Crystallisation from aqueous ethanol with concentrated hydrochloric acid (7 ml) gave the title compound as pink crystals (6.80g, 75%);

Example 42

A. 4-Bromo-2-methylquinoline

2-Methyl-4-quinolinol (19.0g, 0.119 mole) and phosphoryl bromide (80g, 0.279 mole) were heated at 140° with stirring for 3 hours. The resulting black syrup was poured into ice water and the mixture stirred vigorously for 1 hour. The pH was adjusted to 10 with sodium hydroxide and the oily mixture extracted with methylene chloride (4 x 100 ml). The methylene chloride solution was dried ($MgSO_4$) and evaporated to a black oil mixed with solid (26.8g). Chromatography on a silica gel column eluted with EtOAc :hexanes/1:9 gave 4-bromo-2-bromo-methylquinoline as white needles (7.17g, 20%); mp 92.5-95° dec. Anal . Calcd for $C_{10}H_7Br_2$: C, 39.91; H, 2.34; N, 4.65; Br, 53.10. Found: C, 39.88; H, 2.34; N 4.59; Br, 53.17. Continued elution with EtOAc :hexanes/85:15 gave 4-bromo-2-methylquinoline as a pale yellow liquid (14.11g, 53%); the structure was confirmed by $^1$H-NMR. Anal . Calcd for $C_{10}H_8Br$: C, 54.08; H, N, 6.31; Br, 35.98. Found: C, 54.13; H, 3.17; N, 6.27; Br, 35.85.

B. 2-Methyl-4-quinolinecarbaldehyde

To a solution of 4-bromo-2-methylquinoline (4.71g, 21.2 mmol) in dry tetrahydrofuran (50 ml) under nitrogen at -78° was added 1.5M n-butyllithium in hexanes (13.5 ml). After 2 minutes, dry dimethylformamide (2.0 ml, 25 mmol) was added. After an additional 3 minutes at -78°, ethanol (5 ml) was added, followed by 1N hydrochloric acid. After 5 minutes, at 25°, the solution was neutralised with sodium hydroxide and extracted with methylene chloride (4 x 50ml). The methylene chloride solution was dried ($MgSO_4$) and passed through a silica gel pad to give the title compound as yellow crystals (1.24g, 34%);, mp 72-75°. Anal . Calcd for $C_{11}H_9NO1/10H_2O$: C, 76.37; H, 5.36; N, 8.10. Found: C, 76.13; H, 5.44; N, 7.96.

C. 2,4-Diamino-5-(2-methyl-4-quinolylmethylpyrimidine dihydrochloride

The product of Example 42B was converted to the title compound by the procedure of Example 41 in an overall yield of 10%. Crystallisation from 95% ethanol with concentrated hydrochloric acid gave off-white crystals; mp>300° dec. Anal . Calcd for $C_{15}H_{15}N_5.2HCl.H_2O$: C, 50.57; H, 5.38; N, 19.66; Cl, 19.90. Found: C, 50.66; H, 5.38; N, 19.71; Cl, 19.98.

Example 43

A. 4-Hydroxy-7-methoxy-2-trifluoromethylquinoline

m-Anisidine (12.32g, 0.100 mole) was added dropwise to a stirred, heated (100°) mixture of ethyl-4,4,4-trifluoroacetoacetate (18.41g, 0.100 mole) and polyphosphoric acid (100 ml). The temperature was then maintained at 150° for 1.5 hours. After cooling to room temperature, ice-water (300g) was added cautiously with vigorous stirring. The pH was adjusted to 1 with cold 10% sodium hydroxide and the precipitate was collected. This solid was dissolved in cold 10% sodium hydroxide, except for a trace of dark brown solid which was removed by filtration. The pH of the filtrate was brought to 5 with glacial acetic acid and a tan solid (13.91g) collected. Chromatography on a silica gel column eluted with $MeOH:CH_2Cl_2$/5:95 gave fractions containing 4-hydroxy-5-methoxy-2-trifluoromethylquinoline as a pale yellow solid (6.28g, 27%); mp 125-127°; the structure was confirmed by $^{13}C$-NMR. Anal . Calcd for $C_{11}H_8NO_2F_3$: C,54.33; H, 3.32; N, 5.76; F, 23.44. Found: C, 54.30; H, 3.34; N, 5.74; F, 23.34.

Continued elution of the column gave 4-hydroxy-7-methoxy-2-trifluoromethylquinoline as an off-white solid (5.86g, 24%); mp 253-256°; the structure was confirmed by $^{13}C$-NMR. Anal . Calcd for $C_{11}H_8NO_2F_3 0.2H_2O$: C, 53.54; H, 3.43; N, 5.68; F, 23.09. Found: C, 53.69; H, 3.59; N, 5.62; F, 22.81.

B. 4-Bromo-7-methoxy-2-trifluoromethylquinoline

A mixture of 4-hydroxy-7-methoxy-2-trifluoromethylquinoline (24.6g, 0.101 mole) and phosphoryl bromide (41.4g, 0.144 mole) was maintained at 150-165° for 15 minutes and poured over ice. The resulting mixture was extracted with methylene chloride and the methylene chloride solution washed with saturated aqueous $NaHCO_3$ and dried ($MGSO_4$) . Evaporation left a grey solid (17.63g) which was chromatographed on silica gel eluted with EtOAc :hexanes/1:3 to give the title compound as a pale yellow solid (11.79g, 38%). Sublimation at 70-80°/0.25 mm Hg (33 Pa) gave white crystals; mp 120-124°. Anal . Calcd for $C_{11}H_7NOBrF_3$: C, 43.16; H, 2.31; N, 4.58; Br, 26.11; F, 18.62. Found: C, 42.96; H, 2.37; N, 4.54; Br, 25.99; F, 18.57. Continued elution of the column gave 4,8-dibromo-7-methoxy-2-trifluoromethylquinoline as a white solid (1.01g, 3%); mp 85-87°. Anal . Calcd for $C_{11}H_6NOBr_2F_3$: C, 34.32; H, 1.57; N, 3.64; Br, 41.52; F, 14.80. Found C, 34.41; H, 1.57; N, 3.62; Br, 41.43; F, 14.61.

C. 7-Methoxy-2-trifluoromethyl-4-quinolinecarbaldehyde

A solution of 4-bromo-7-methoxy-2-trifluoromethylquinoline (11.64g, 37.4 mmol) in dry tetrahydrofuran (250 ml) under nitrogen was cooled to -78°. A 1.35 M solution of sec -butyllithium in cyclohexane (33.3 ml) was added dropwise over 5 minutes with the temperature above minus 65°C. Dimethylformamide (4.3 ml) was added, followed by ethanol (50 ml) and 1N hydrochloric acid (112ml). The solution was stirred at 25° for 1 hour, neutralised with sodium hydroxide and extracted with methylene chloride (3 x 150 ml). The methylene chloride solution was dried ($MGSO_4$) and evaporated to an orange solid (10.5g) Chromatography on silica gel eluted with EtOAc :hexanes/1:4 gave the title compound as a yellow-orange solid (5.33g, 56%). Sublimation at 90°/0.25 mm Hg (33 Pa) gave the title compound as a pale yellow powder; mp 135-137°. Anal . Calcd for $C_{12}H_8NF_3O_2$: C, 56.48; H, 3.16; N, 5.49; F, 22.33. Found: C, 56.32; H, 3.21; N, 5.41; F, 22.61.

D. 2,4-Diamino-5-(7-methoxy-2-trifluoromethyl-4-quinolylmethyl)pyrimidine

The product of example 43C was converted to the title compound by the procedure of Example 41 in an overall yield of 14%. The title compound was crystallised from 95% ethanol to give white crystals; mp 256-259° dec. Anal . Calcd for $C_{16}H_{14}N_5F_3O$: C, 55.01; H, 4.04; N, 20.05; F, 15.60. Found: C, 54.99; H, 4.05; N, 19.99; F, 15.44.

Example 44

A. 7-Trifluoromethyl-4-quinolinecarbaldehyde

4-Bromo-7-trifluoromethylquinoline was prepated from 4-hydroxy-7-trifluoromethyl-quinoline by the procedure of Example 43B in 28% yield, after sublimation at 100°/0.2 mm Hg (26 Pa) to give a pale yellow solid; mp 68-70°. The title compound was prepared from 4-bromo-7-trifluoromethylquinoline (16.0g), 58.0 mmol) by the procedure of Example 42B. Chromatography on silica gel eluted with EtOAc:$CH_2Cl_2$/1:9 gave an off-white solid (3.00g, 23%); mp 63-64.5°. Anal. Calcd for $C_{11}H_6F_3NO$: C, 58.68; H, 2.69; N, 6.22; F, 25.31. Found: C, 58.82; H, 3.06; N, 6.10; F, 25.10.

B. 2,4-Diamino-5-(7-trifluoromethyl-4-quinolylmethyl)pyrimidine dihydrochloride

The product of Example 44A was converted to the title compound by the procedure of Example 41 in an overall yield of 17%. Crystallisation from 95% ethanol with concentrated hydrochloric acid gave a white solid; mp 214-218° dec. Anal. Calcd for $C_{15}H_{12}N_5F_3 2HCl.1\frac{1}{4}H_2O$: C, 43.44, H, 4.01; N, 16.89; F, 13.74; Cl, 17.10. Found: C, 43.10; H, 3.64; N, 16.69; F, 13.98; Cl, 17.13.

## Example 45

**In vitro Antibacterial Activity of Compound : Compared to Trimethoprim, Expressed as M.I.C. Compound/M.I.C. Trimethoprim where M.I.C. = Minimum Inhibitory Concentration in micrograms/ml.***

| Organism | Example Number | | | | | | M.I.C. Trimethoprim, µg/ml |
|---|---|---|---|---|---|---|---|
| | 10 | 12 | 14 | 20 | 26 | 31 | |
| Streptococcus pyogenes CN10 | 1.0 | 1.0 | 0.1 | - | 0.1 | 0.1 | 0.1 |
| Staphylococcus aureus CN491 | 0.3 | 1.0 | 0.1 | 1.0 | 0.1 | 3.0 | 0.1 |
| Vibrio cholerae ATCC14035 | 0.3 | 1.0 | 0.3 | 3.0 | - | 0.1 | 0.1 |
| Mycobacterium smegmatis S3254 | 0.3 | 1.0 | 1.0 | 3.0 | 1.0 | 0.3 | 1.0 |
| Salmonella typhosa CN512 | 1.0 | 3.0 | 1.0 | 3.0 | 10.0 | 1.0 | 0.1 |
| Escherichia coli CN314 | 1.0 | 10.0 | 3.0 | 3.0 | 5.0 | 3.0 | 0.3 |
| Serratia marcescens CN2398 | 1.0 | 3.0 | 1.0 | 3.0 | 1.0 | 3.0 | 10.0 |
| Klebsiella pneumoniae CN3632 | 1.0 | 10.0 | 1.0 | 3.0 | 2.0 | 3.0 | 1.0 |
| Proteus mirabilis S2409 | 1.0 | 10.0 | 0.3 | 3.0 | 5.0 | 3.0 | 3.0 |

* Numbers less than 1 indicate a potency greater than that of trimethoprim
2,4-diamino-5-(8-methoxy-2,4-dimethyl-6-quinolylmethyl)pyrimidine has an $LD_{50} > 500$ mg/kg i.p.

Example 46 -

2,4-diamino-5-(1,2,3,4-tetrahydro-2-isoquinolylmethyl)pyrimidine

To a solution of 1,2,3,4-tetrahydroisoquinoline (5.33g, 40.0mmol) in ethanol (25ml) was added 2,4-diamino-5-bromoethylpyrimidine hydrobromide (3.64g, 10.0mmol from elemental analysis and $^1$H-NMR). The mixture was stirred at 25° for 14 hours, filtered, and the filtrate refluxed for 2 hours. On cooling, the solution deposited white solid which was extracted with refluxing 95% ethanol (50ml). The filtered ethanol solution deposited title compound on cooling as white powder (0.56g, 22%), mp 221-225° dec. Anal. Calcd. for $C_{14}H_{17}N_5$: C, 65.86: H, 6.7l; N, 27.43. Found: C, 65.60, H, 6.74; N, 27.5l

## Claims

1. A compound of the formula (II)

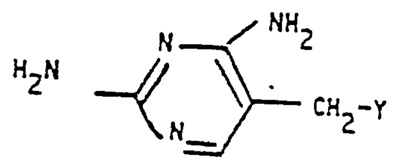

(II)

or a salt, N-oxide or acyl derivative thereof, wherein Y is a group:

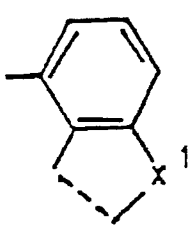 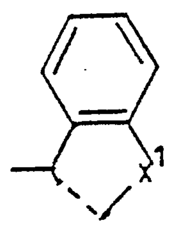 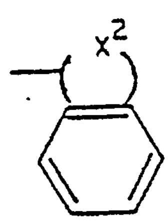 or 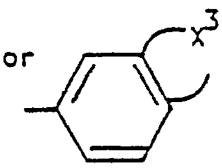

$X^1$ is an oxygen or sulphur atom, a group $CH_2$, a group $S(O)_n$ where n is 1 or 2, a group $NR^1$ wherein $R^1$ is hydrogen or $C_{1-4}$ alkyl; ($X^2$) is a six-membered ring containing a nitrogen atom; ($X^3$) is a six-membered ring optionally containing a nitrogen, and the dotted lines represent single or double bonds, and the group Y is optionally substituted by one to three substituents selected from halogen, $C_{2-4}$alkenyl, $C_{1-4}$ alkylthio, amino, mono-( $C_{1-4}$-alkyl)-substituted amino, di-( $C_{1-4}$-alkyl)-substituted amino, morpholino, piperidino, pyrrolidino, piperazino, hydroxy, nitro, $C_{1-4}$ alkoxycarbonyl, or $C_{1-4}$ alkyl or $C_{1-4}$ alkoxy, each optionally substituted by halogen, hydroxy or $C_{1-3}$ alkoxy.

2. A compound according to claim 1 of the formula (III):

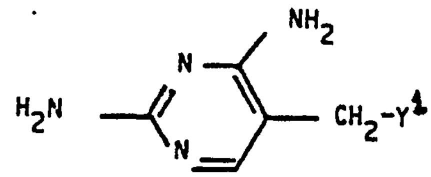

**wherein $Y^1$ is a group**

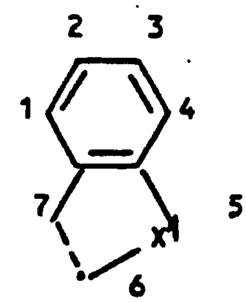

which is linked to the pyrimidinylmethyl moiety at the 1 or 7 position and is optionally substituted at the 2,3,4 or 6 positions or at the 7 position when the linkage to the pyrimidinyl moiety is at the 1 position, wherein $X^1$ and the dotted line are as hereinbefore defined in claim 1.

3. A compound according to claim 1 of the formula (VI):

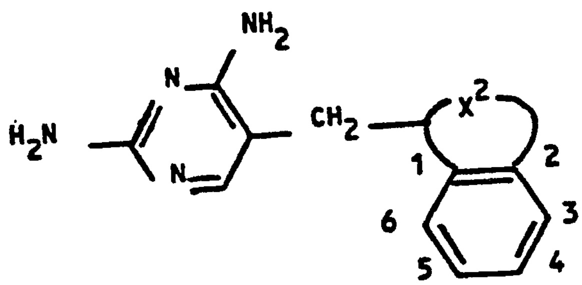

(VI)

or a salt, N-oxide or acyl derivative thereof, wherein ($X_2$ ) is a six-membered ring containing a nitrogen atom, both the phenyl ring and the ($X_2$ ) ring being optionally substituted other than at the 6- position of the phenyl ring.

4. A compound according to claim 1 of the formula (IX):

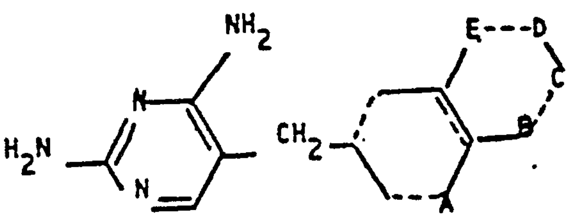

(IX)

or a salt, N-oxide or acyl derivative thereof, optionally containing a nitrogen atom at one of positions A, B, C, D or E, in which the dotted lines represent double bonds unless one of the rings contains a nitrogen atom in which case the dotted lines in this ring represent single or double bonds, the bicyclic

ring being optionally substituted as defined in claim 1.

**5.** A compound according to any of claims 1 to 4, wherein the group Y is optionally substituted by one to three substituents as defined in claim, 1.

**6.** A compound according to claim 2, wherein $Y^1$ is a group:

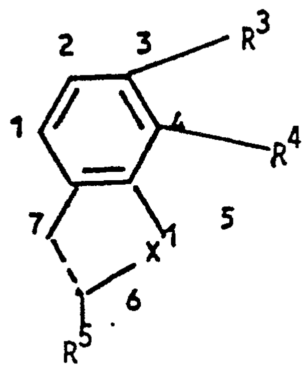

or a salt, N-oxide or acyl derivative thereof; which group is linked to the pyrimidinylmethyl moiety at the 1 or 7 position; $X^1$ is oxygen, sulphur or a group $NR^1$ or $S(O)_n$ as hereinbefore defined in claim 1; and $R^3$, $R^4$ and $R^5$ are the same or different and are selected from hydrogen, halogen atoms, $C_{2-4}$ alkenyl, $C_{1-4}$ alkylthio, amino, mono- $C_{1-4}$ alkylamino, di-$C_{1-4}$ alkyl substituted amino, morpholino, piperidino, pyrrolidino, piperazino, hydroxy, nitro, $C_{1-4}$ alkoxycarbonyl or $C_{1-4}$alkyl or $C_{1-4}$ alkoxy, each optionally substituted by halogen, hydroxy or $C_{1-3}$ alkoxy, and the dotted line is as defined in claim 1.

**7.** A compound according to claim 6, wherein $R^3$ and $R^4$ are the same or different and each is hydrogen, methyl, methoxy, amino, dimethylamino, methylthio, bromo or chloro; and $R^5$ is hydrogen or methyl.

**8.** A compound according to claim 6 or 7, wherein $Y^1$ is linked to the pyrimidinylmethyl moiety at the one position.

**9.** A compound according to claim 7 of the formula (IV)

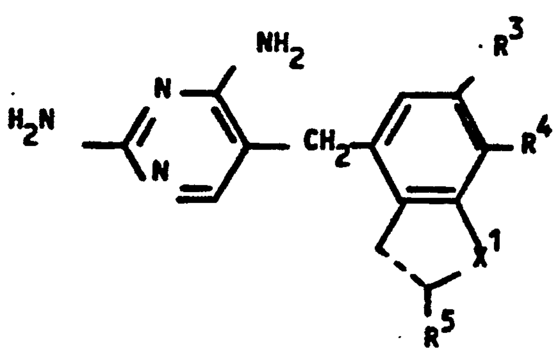

(IV)

or a salt, N-oxide or acyl derivative thereof, wherein $X^1$, $R^3$ to $R^5$ and the dotted line are as defined in claim 7.

**10.** A compound according to either claim 1 or 3 of the formula (VII):

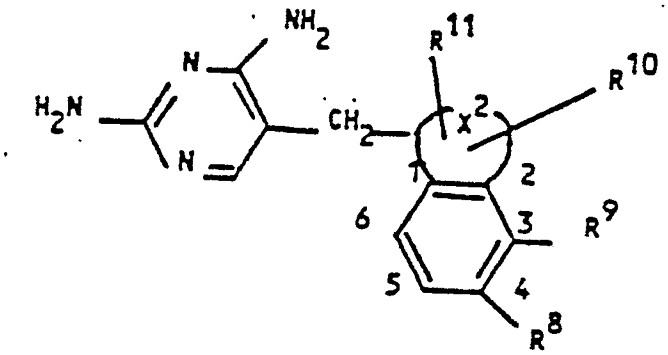

(VII)

or a salt, N-oxide or acyl derivative thereof, wherein ($\bar{X}_2$ ) is a six-membered ring containing three double bonds in which case $X^2$ is -N=, two double bonds In which case $X^2$ is -N=, or $NR^1$-, or one double bond in which case $X^2$ is -$NR^{12}$, wherein $R^{12}$ is a group $R^1$ as hereinbefore defined in claim 1 or is a bond to the 5-methylene bridge to the pyrimidine ring;

$R^8$, $R^9{}_1$ $R^{10}$ and $R^{11}$ are the same or different and each is hydrogen, halogen, $C_{2-4}$ alkenyl, nitro, hydroxy, a group -$SR^6$, wherein $R^6$ is $C_{1-3}$ alkyl, a group -$COR^7$, wherein $R^7$ is methoxy or ethoxy; or each is amino optionally substituted by one or two $C_{1-4}$alkyl groups; morpholino, piperidino, pyrrolidino or $C_{1-4}$ alkyl or $C_{1-4}$ alkoxy, each optionally substituted by halogen, hydroxy, or $C_{1-2}$ alkoxy.

**11.** A compound according to claim 10 wherein $R^8$, $R^9$, $R^{10}$ and $R^{11}$ are the same or different and each is hydrogen, hydroxy, methoxy, ethoxy, methoxyethoxy, methyl, ethyl, propyl, amino, methylamino, dimethylamino, ethylamino, diethylamino, vinyl, allyl, propenyl, halogen, methylthio, ethylthio or pyrrolyl; ($\bar{X}_2$ ) ring contains three double bonds, and the 5-methylene bridge to the pyrimidine ring is joined to the bicyclic ring system at the position in the heterocyclic ring α or β to the 1-position of the phenyl ring.

**12.** A compound according to claim 4 of the formula (X):

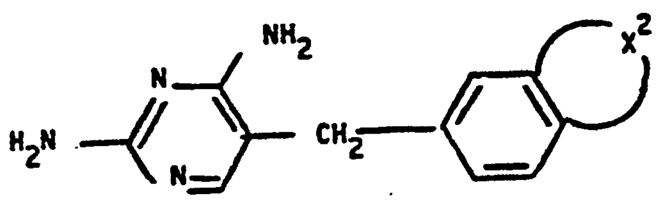

(X)

or a salt, N-oxide or acyl derivative thereof, wherein ($\bar{X}_2$ ) is a six-membered ring containing a nitrogen atom; there being one to three substituents attached to carbon atoms of the ($\bar{X}_2$ ) ring or to a carbon atom of the phenyl ring adjacent to the ($\bar{X}_2$ ) ring, the substituents being selected from methoxy, ethoxy, methyl, ethyl, amino, dimethylamino, pyrrolyl, morpholino, methoxyethoxy, chlorine, bromine, methoxycarbonyl or ethoxycarbonyl.

**13.** A compound according to either claim 4 or 12 of the formula (XI):

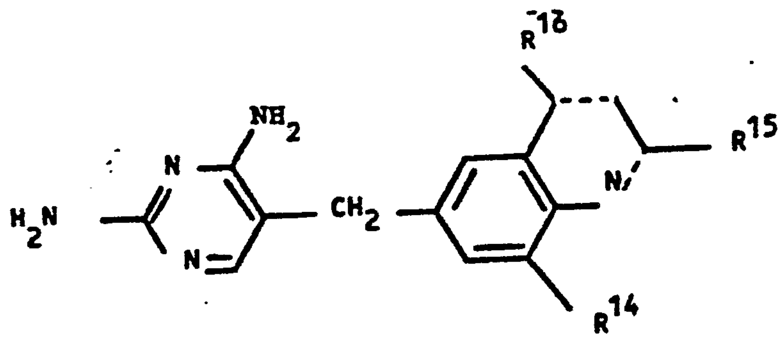

(XI)

or a salt, N-oxide or acyl derivative thereof, wherein the dotted lines represent single or double bonds, $R^{14}$, $R^{15}$ and $R^{16}$ are the same or different and each represents hydrogen, methoxy, ethoxy, methyl, ethyl, amino, dimethylamino, pyrrolyl, morpholino, methoxyethoxy, chlorine, bromine, methoxycarbonyl or ethoxycarbonyl.

**14.** A pharmaceutical composition comprising a compound of the formula (II) as defined in claim 1 herein in combination with a pharmaceutically acceptable carrier.

**15.** A pharmaceutical composition according to claim 14 which includes a sulphonamide as an additional active ingredient.

**16.** A compound of the formula (II), as defined in claim 1 herein, for use in medicine.

**17.** A process for the preparation of a compound of the formula (II), as defined in claim 1 herein, which process comprises.

(a) (i)the reaction of a guanidine salt with a compound of the formula (A) or

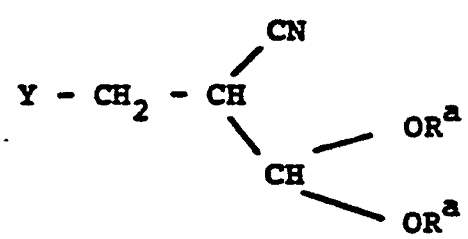

(A)

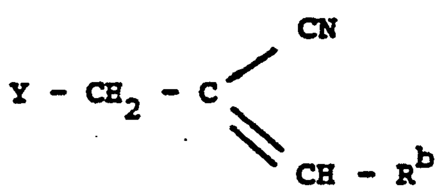

(B)

(B):

Wherein Y is as hereinbefore defined in claim 1 $R^a$ is a $C_{1-4}$ alkyl group and $R^b$ is a nucleophilic leaving group such as a $C_{1-4}$ alkoxy group, for example a methoxy, ethoxy or methoxyethoxy group, or an amino, $C_{1-4}$ alkylamino, benzyl-amino, di-$C_{1-4}$ alkylamino, naphthylamino, optionally substituted anilino, morpholino, piperidino or N-methylpiperazino group and most preferably $R^b$ is an anilino group:

(ii) the reaction of a compound of formula (C):

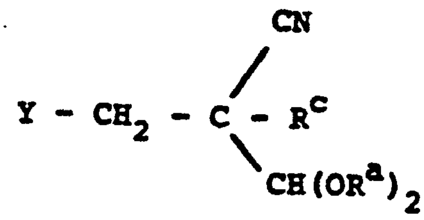

(C)

wherein Y and $R^a$ are as hereinbefore defined in claim 1 and $R^c$ is an alkoxycarbonyl or aldehyde group, with potassium or sodium hydroxide in a $C_{1-4}$ alkanol followed by addition of guanidine;

(iii) the reaction of a compound of the formula (D):

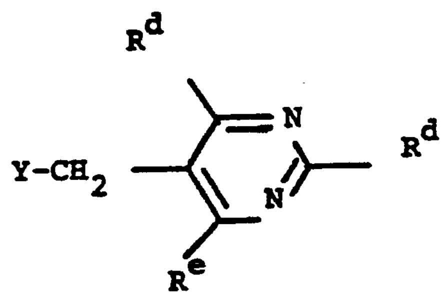

(D)

wherein $R^d$ is an amino group or a leaving group, such as a $C_{1-4}$ alkylthio group or a halogen atom, $R^e$ is a hydrogen or halogen atom, except that both groups $R^d$ cannot be amino groups and Y is as hereinbefore defined in claim 1 with an aminating agent such as ammonia and thereafter when $R^e$ is a halogen atom removing this by hydrogenolysis;

(iv)the reaction of a compound of the formula (E)

(E)

$$Y - CH_2Z$$

wherein Z is a halogen atom or hydroxy or di-$C_{1-4}$ alkyl substituted amino or other leaving group; and Y is as hereinbefore defined, in claim 1 with a compound of the formula (F):

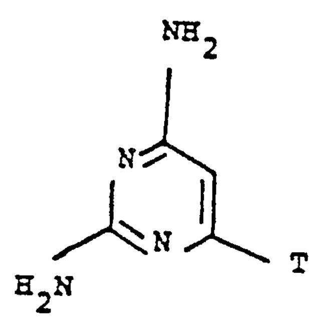

(F)

wherein T is hydrogen or a hydroxy or $C_{1-4}$ alkylthio group, and then converting the group T to hydrogen by hydrogenolyis when T is a $C_{1-4}$ alkylthio group or, when T is a hydroxy group, by first converting it to the mesylate or tosylate derivative or to thio, alkylthio or halogen and then removing this by hydrogenolysis;

(b) when it is required to prepare a compound of the formula (IV) as defined in claim 9 wherein $R^4$ is other than hydrogen, the cyclisation of a compound of the formula (G)

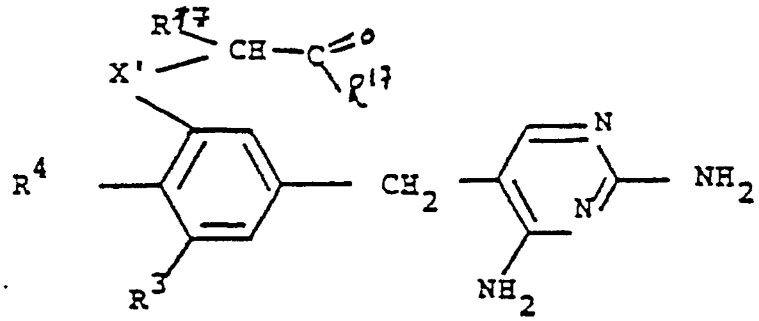

(G)

wherein $X^1$, $R^3$ and $R^4$ are as hereinbefore defined in claim 7 except that $R^4$ is not hydrogen and the two groups $R^{17}$ are the same or different and each is hydrogen or $C_{1-4}$alkyl;

(c) when it is required to prepare a compound of the formula (IV) as defined in claim 9 wherein the 4-position of the phenyl ring is optionally substituted by hydroxy, alkoxy amino or substituted amino or (X) as defined in claim 12, wherein ($\bar{\bar{x}}_2$ ) is a partially saturated aromatic ring in which the nitrogen atom is adjacent to the phenyl ring the reaction of a compound of the formula (H):

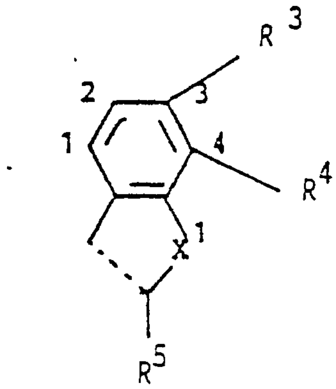

(H)

wherein the 4- position of the phenyl ring is optionally substituted by hydroxy, alkoxy, amino, substituted amino and the ($\bar{\bar{x}}_2$ ) ring and the phenyl ring are substituted by other substituents as hereinbefore defined, in claim 7, or a compound of the formula (L):

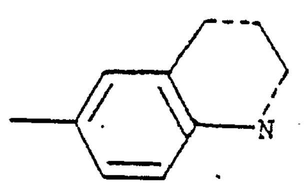

(L)

wherein at least one of the dotted lines is a single bond and, when the dotted line to the nitrogen is a single bond, the nitrogen is substituted with hydrogen or an alkyl group, with 2,4-diamino-5-hydroxymethylpyrimidine or an ether thereof;

(d) the conversion of one compound of the formula (II) to a different compound of the formula (II) for example by the reduction or isomerization of one or two of the double bonds, conversion of a hydroxy group to a $C_{1-4}$ alkylthio group or an optionally substituted $C_{1-4}$ alkoxy group or conversion of an amino group to a $C_{1-4}$ alkylthio group or hydrogen, halogen, or hydroxy via a diazo group or to a substituted amino group by methods well known to those skilled in the art.

(e) when it is required to prepare a compound of formula (IX) wherein a nitrogen atom is at position B and the dotted lines represent double bonds, the condensation/cyclization of a compound of the formula (M):

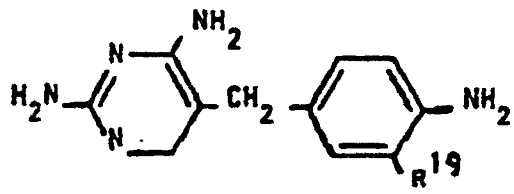

(M)

wherein $R^{19}$ is halogen, $C_{1-4}$ alkyl, $C_{1-4}$ alkoxy or $C_{1-4}$ alkylthio with a compound of the formula (N):

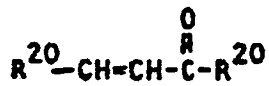

(N)

wherein the groups $R^{20}$ are the same or different and each is hydrogen or $C_{1-4}$ alkyl, under acidic conditions such as dilute ethanolic hydrochloric acid.

(f) When it is required to prepare a compound of Formula (IX) wherein a nitrogen atom is at position A,B,C,D or E and all of the dotted lines represent double bonds, the oxidation of a compound of Formula (IX) wherein not all of the dotted lines represent double bonds under suitable dehydrogenation conditions, such as elevated temperatures in the presence of a dehydrogenation catalyst such as platinum oxide.

**18.** A novel chemical intermediate of any one of formulae (A) to D as defined in claim 17.

**Revendications**

**1.** Composé de formule (II) :

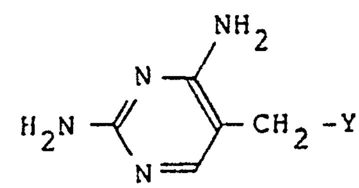

(II)

ou un sel, N-oxyde ou dérivé acylé de celui-ci, où Y est un radical :

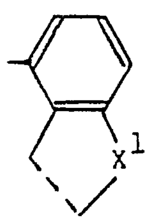

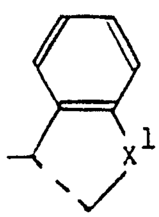

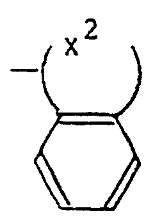

ou

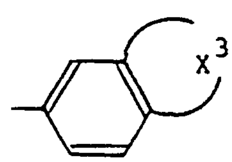

$X^1$ est un atome d'oxygène ou de soufre, un radical $CH_2$, un radical $S(O)_n$, où n = 1 ou 2, un radical $NR^1$ où $R^1$ est hydrogène ou $C_{1-4}$-alcoyle;
($\bar{X}_2$ ) est un cycle de six chaînons contenant un atome d'azote;
( X $^3$) est un cycle de six chaînons contenant éventuellement un atome d'azote, et
les lignes en pointillés représentent des liaisons simples ou doubles, et
le radical Y est éventuellement substitué par un à trois substituants choisis parmi halogène, $C_{2-4}$-alcényle, $C_{1-4}$-alcoylthio, amino, amino mono-( $C_{1-4}$-alcoyl)-substitué, amino di-( $C_{1-4}$-alcoyl)-substitué, morpholino, pipéridino, pyrrolidino, pipérazino, hydroxyle, nitro, $C_{1-4}$-alcoxycarbonyle ou bien $C_{1-4}$-alcoyle ou $C_{1-4}$-alcoxy, dont chacun est éventuellement substitué par halogène, hydroxyle ou $C_{1-3}$-alcoxy.

**2.** Composé suivant la revendication 1 de formule (III) :

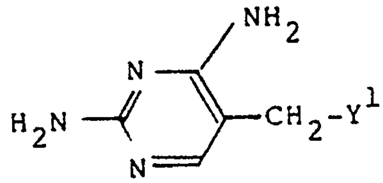

où $Y^1$ est un radical :

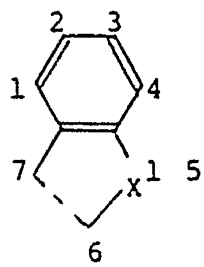

qui est uni à la partie pyrimidinylméthyle à la position 1 ou 7 et qui est éventuellement substitué à la position 2, 3, 4 ou 6 ou à la position 7 lorsque la liaison à la partie pyrimidinyle est à la position 1, où $X^1$ et la ligne en pointillés sont tels que définis ci-dessus dans la revendication 1.

**3.** Composé suivant la revendication 1, de formule (VI) :

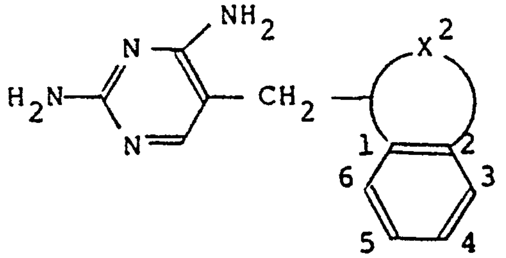

(VI)

ou un sel, N-oxyde ou dérivé acylé de ceux-ci, où ($\bar{X}_2$ ) est un cycle de six chaînons contenant un atome d'azote, le cycle phényle et le cycle ($\bar{X}_2$ ) étant tous deux éventuellement substitués ailleurs qu'à la position 6 du cycle phényle.

**4.** Composé suivant la revendication 1 de formule (IX) :

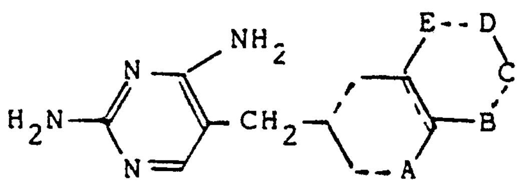

(IX)

ou un sel, N-oxyde ou dérivé acylé de celui-ci, contenant éventuellement un atome d'azote à l'une des positions A, B, C, D ou E, dans lequel les lignes en pointillés représentent des liaisons doubles, sauf si l'un des cycles contient un atome d'azote, auquel cas les lignes en pointillés de ce cycle représentent des liaisons simples ou doubles, le système bicyclique étant éventuellement substitué comme défini dans la revendication 1.

5. Composé suivant l'une quelconque des revendications 1 à 4, dans lequel le radical Y est éventuellement substitué par un à trois substituants tels que définis dans la revendication 1.

6. Composé suivant la revendication 2, où $Y^1$ est un radical :

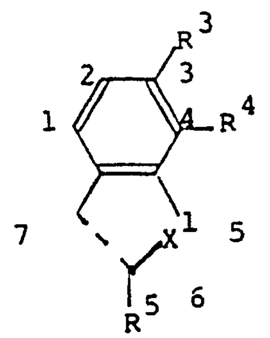

ou un sel, N-oxyde ou dérivé acylé de celui-ci; lequel radical est uni à la partie pyrimidinylméthyle à la position 1 ou 7; $X^1$ est oxygène ou soufre ou un radical $NR^1$ ou $S(O)_n$ tel que défini ci-dessus dans la revendication 1; et $R^3$ $R^4$ et $R^5$ sont identiques ou différents et sont choisis parmi les atomes d'hydrogène et d'halogène et les radicaux $C_{2-4}$-alcényle, $C_{1-4}$-alcoylthio, amino, mono-( $C_{1-4}$-alcoyl)-amino, amino di-( $C_{1-4}$-alcoyl)-substitué, morpholino, pipéridino, pyrrolidino, pipérazino, hydroxyle, nitro, $C_{1-4}$-alcoxycarbonyle ou bien $C_{1-4}$-alcoyle ou $C_{1-4}$-alcoxy, chacun éventuellement substitué par halogène, hydroxyle ou $C_{1-3}$-alcoxy, et la ligne en pointillés est telle que définie dans la revendication 1.

7. Composé suivant la revendication 6, dans lequel $R^3$ et $R^4$ sont identiques ou différents et sont chacun hydrogène, méthyle, méthoxy, amino, diméthylamino, méthylthio, mono ou chloro, et $R^5$ est hydrogène ou méthyle.

8. Composé suivant la revendication 5 ou 6, dans lequel $Y^1$ est uni à la partie pyrimidinylméthyle à la position un.

9. Composé suivant la revendication de formule (IV) :

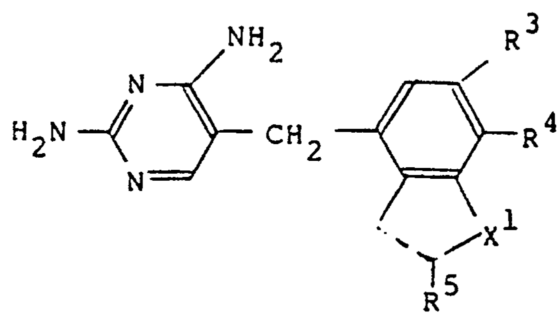

(IV)

ou un sel, N-oxyde ou dérivé acylé de celui-ci, où $X^1$, $R^3$ à $R^5$ et la ligne en pointillés sont tels que définis dans la revendication 7.

10. Composé suivant la revendication 1 ou 3, de formule (VII) :

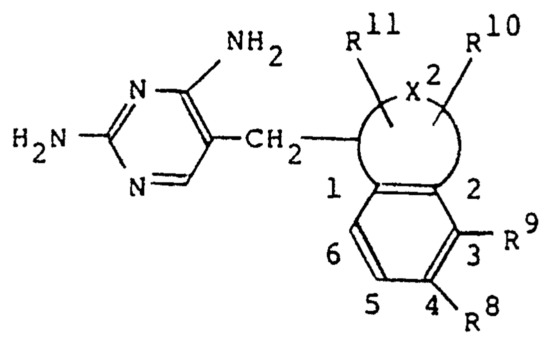

(VII)

ou un sel, N-oxyde ou dérivé acylé de ceux-ci, où ($\bar{X}_2$ ) est un cycle de six chaînons contenant trois doubles liaisons, auquel cas $X^2$ est -N=; deux doubles liaisons auquel cas $X^2$ est -N= ou $NR^1$-; ou une double liaison, auquel cas $X^2$ est -$NR^{12}$, où $R^{12}$ est un radical $R^1$ tel que défini dans la revendication 1 ou est une liaison avec le pont 5-méthylène vers le cycle pyrimidine; $R^8$, $R^9$, $R^{10}$ et $R^{11}$ sont identiques ou différents et sont chacun hydrogène, halogène, $C_{2-4}$-alcényle, nitro, hydroxyle, un radical $SR^6$, où $R^6$ est $C_{1-3}$-alcoyle, un radical -$COR^7$, où $R^7$ est méthoxy ou éthoxy: ou sont chacun amino éventuellement substitué par un ou deux radicaux $C_{1-4}$-alcoyle; morpholino, pipéridino, pyrrolidino ou bien $C_{1-4}$-alcoyle ou $C_{1-4}$-alcoxy, dont chacun est éventuellement substitué par halogène, hydroxyle ou $C_{1-2}$-alcoxy.

**11.** Composé suivant la revendication 10, dans lequel $R^8$, $R^9$, $R^{10}$, $R^{11}$ sont identiques ou différents et sont chacun hydrogène, hydroxyle, méthoxy, éthoxy, méthoxyéthoxy, méthyle, éthyle, propyle, amino, méthylamino, diméthylamino, éthylamino, diéthylamino, vinyle, allyle, propényle, halogène, méthylthio, éthylthio ou pyrrolyle; le cycle ($\bar{X}_2$ ) contient trois doubles liaisons et le pont 5-méthylène vers le cycle pyrimidine est uni au système bicyclique à la position $\alpha$ ou $\beta$ de l'hétérocycle par rapport à la position 1 du cycle phényle.

**12.** Composé suivant la revendication 4 de formule (X) :

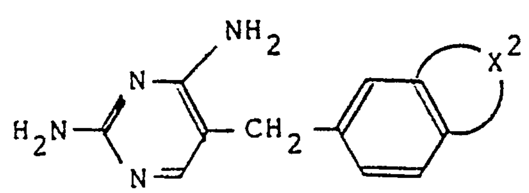

(X)

ou un sel, N-oxyde ou dérivé acylé de celui-ci, où ($\bar{X}_2$ ) est un cycle de six chaînons contenant un atome d'azote; un à trois substituants étant unis aux atomes de carbone du cycle ($\bar{X}_2$ ) ou à un atome de carbone du cycle phényle adjacent au cycle ($\bar{X}_2$ ), les substituants étant choisis parmi méthoxy, éthoxy, méthyle, éthyle, amino, diméthylamino, pyrrolyle, morpholino, méthoxyéthoxy, chlore, brome, méthoxycarbonyle et éthoxycarbonyle.

**13.** Composé suivant la revendication 4 ou 12 de formule (XI) :

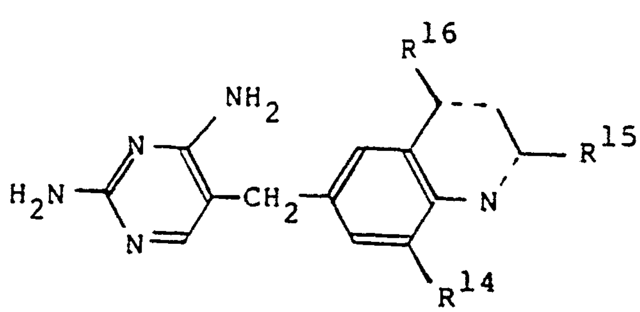

(XI)

ou un sel, N-oxyde ou dérivé acylé de celui-ci, où les lignes en pointillés représentent des liaisons simples ou doubles, $R^{14}$, $R^{15}$ et $R^{16}$ sont identiques ou différents et représentent chacun hydrogène, méthoxy, éthoxy, méthyle, éthyle, amino, diméthylamino, pyrrolyle, morpholino, méthoxyéthoxy, chlore, brome, méthoxycarbonyle ou éthoxycarbonyle.

**14.** Composition pharmaceutique, comprenant un composé de formule (II) tel que défini dans la revendication 1 en combinaison avec un excipient pharmaceutiquement acceptable.

**15.** Composition pharmaceutique suivant la revendication 14, qui comprend un sulfonamide comme constituant actif supplémentaire.

**16.** Composé de formule (II) tel que défini dans la revendication 1, à utiliser en médecine.

**17.** Procédé de préparation d'un composé de formule (II) tel que défini dans la revendication 1, qui comprend :

(a) (i) la réaction d'un sel de guanidine avec un composé de formule (A) ou (B) :

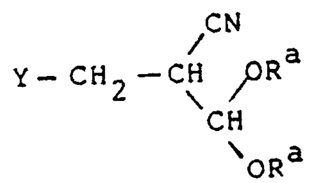

(A)

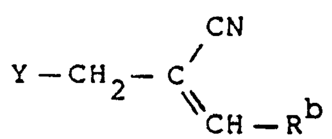

(B)

où Y est tel que défini ci-dessus dans la revendication 1, $R^a$ est un radical $C_{1-4}$-alcoyle et $R^b$ est un radical nucléophile partant tel qu'un radical $C_{1-4}$-alcoxy, par exemple un radical méthoxy, éthoxy ou méthoxyéthoxy, ou un radical amino, $C_{1-4}$-alcoylamino, benzylamino, di-( $C_{1-4}$-alcoyl)-amino, naphtylamino, anilino éventuellement substitué, morpholino, pipéridino ou N-méthylpipérazino, mais le plus avantageusement, $R^b$ est un radical anilino;

(ii) la réaction d'un composé de formule (C) :

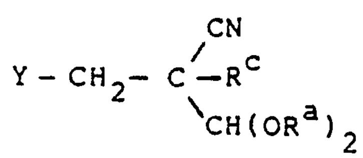

(C)

où Y et $R^a$ sont tels que définis dans la revendication 1 et $R^c$ est un radical alcoxycarbonyle ou aldéhyde, avec l'hydroxyde de sodium ou de potassium dans un $C_{1-4}$-alcanol, suivie de l'addition de guanidine;

(iii) la réaction d'un composé de formule (D) :

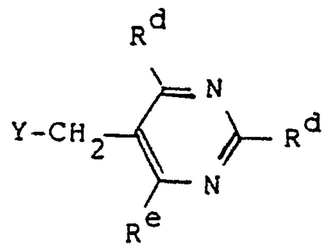

(D)

où $R^d$ est un radical amino ou un radical partant tel qu'un radical $C_{1-4}$-alcoylthio ou un atome d'halogène, $R^e$ est un atome d'hydrogène ou d'halogène, sauf que les deux radicaux $R^d$ ne peuvent être des radicaux amino et Y est tel que défini dans la revendication 1, avec un agent d'amination tel que l'ammoniac, et ensuite, lorsque $R^e$ est un atome d'halogène, l'élimination de celui-ci par hydrogénolyse.

(iv) la réaction d'un composé de formule (E) :

$$Y-CH_2Z \qquad (E)$$

où Z est un atome d'halogène ou un radical hydroxyle ou amino di-( $C_{1-4}$-alcoyl)-substitué ou un autre radical partant et Y est tel que défini ci-dessus dans la revendication 1, avec un composé de formule (F) :

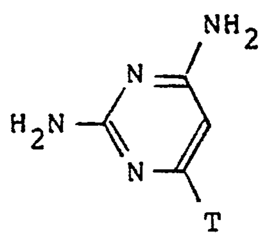

(F)

où T est hydrogène ou un radical hydroxyle ou $C_{1-4}$-alcoylthio, puis la conversion du radical T en hydrogène par hydrogénolyse lorsque T est un radical $C_{1-4}$-alcoylthio ou bien, lorsque T est un radical hydroxyle, par conversion préalable de celui-ci en le mésylate ou tosylate ou en radical thio, alcoylthio ou halogène, puis par élimination de ce dernier par hydrogénolyse;

(b) lorsqu'il est requis de préparer un composé de formule (IV) tel que défini dans la revendication 9, où $R^4$ est autre qu'hydrogène, la cyclisation d'un composé de formule (G) :

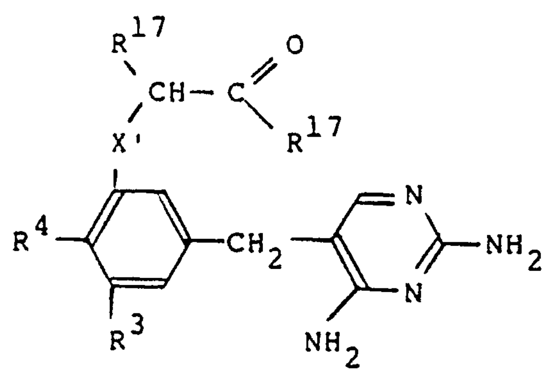

(G)

où $X'_1$ $R^3$ et $R^4$ sont tels que définis ci-dessus dans la revendication 7, sauf que $R^4$ n'est pas hydrogène et les deux radicaux $R^{17}$ sont identiques ou différents et sont chacun hydrogène ou $C_{1-4}$-alcoyle;

(c) lorsqu'il est requis de préparer un composé de formule (IV) tel que défini dans la revendication

9, où la position 4 du cycle phényle est éventuellement substituée par hydroxyle, alcoxy, amino ou amino substitué, ou de formule (X) tel que défini dans la revendication 12, où ($\bar{X}_2$ ) est un cycle aromatique partiellement saturé dans lequel l'atome d'azote est adjacent au cycle phényle, la réaction d'un composé de formule (H) :

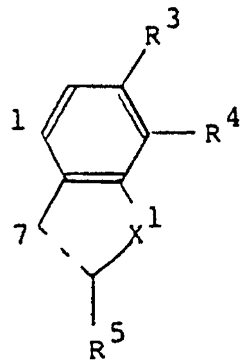

(H)

où la position 4 du cycle phényle est éventuellement substituée par hydroxyle, alcoxy, amino, amino substitué et le cycle ($\bar{X}_1$ ) et le cycle phényle sont substitués par d'autres substituants tels que définis ci-dessus, ou bien d'un composé de formule (L) :

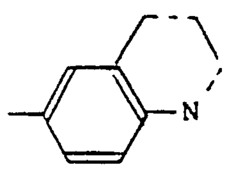

(L)

où au moins l'une des lignes en pointillés est une liaison simple et, lorsque la ligne en pointillés vers l'azote est une liaison simple, l'azote est substitué par hydrogène ou un radical alcoyle, avec la 2,4-diamino-5-hydroxyméthylpyrimidine ou un éther de celle-ci;

(d) la conversion d'un composé de formule (II) en un composé différent de formule (II), par exemple par réduction ou isomérisation d'une ou deux des doubles liaisons, conversion d'un radical hydroxyle en un radical $C_{1-4}$-alcoylthio ou un radical $C_{1-4}$-alcoxy éventuellement substitué, ou conversion d'un radical amino en un radical $C_{1-4}$-alcoylthio ou hydrogène, halogène ou hydroxyle par l'intermédiaire d'un radical diazo, ou en un radical amino substitué suivant des procédés connus de l'homme de métier;

(e) lorsqu'il est requis de préparer un composé de formule (IX), où un atome d'azote occupe la position $\beta$ et les lignes en pointillés représentent des doubles liaisons, la condensation/cyclisation d'un composé de formule (M) :

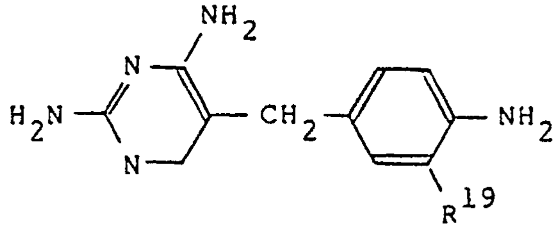

(M)

où $R^{19}$ est halogène, $C_{1-4}$-alcoyle, $C_{1-4}$-alcoxy ou $C_{1-4}$-alcoylthio, avec un composé de formule (N) :

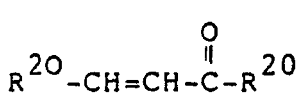

(N)

où les radicaux $R^{20}$ sont identiques ou différents et sont chacun hydrogène ou $C_{1-4}$-alcoyle, dans des conditions acides, par exemple dans l'acide chlorhydrique éthanolique dilué;

(f) lorsqu'il est requis de préparer un composé de formule (IX) où un atome d'azote occupe la position A, B, C, D ou E et toutes les lignes en pointillés représentent des doubles liaisons, l'oxydation d'un composé de formule (IX) où toutes les lignes en pointillés ne représentent pas des doubles liaisons, dans des conditions de déshydrogénation appropriées, par exemple à des températures élevées en présence d'un catalyseur de déshydrogénation tel que l'oxyde de platine.

**18.** Nouvel intermédiaire chimique de l'une quelconque des formules (A) à (D) tel que défini dans la revendication 17.

## Ansprüche

**1.** Verbindung der Formel (II)

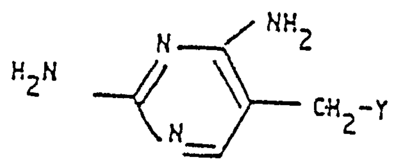

(II)

oder ein Salz, N-Oxid oder Acylderivat davon, in der Y eine der folgenden Gruppen ist:

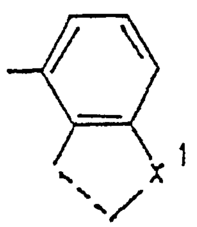

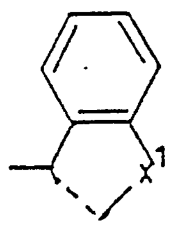

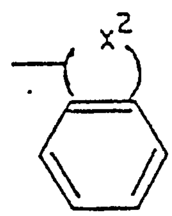

oder

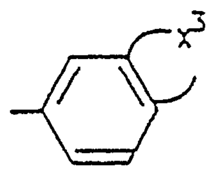

$X^1$ ist ein Sauerstoff- oder Schwefelatom, eine Gruppe $CH_2$, eine Gruppe $S(O)_n$, in der n 1 oder 2 ist, eine Gruppe $NR^1$, in der $R^1$ Wasserstoff oder $C_{1-4}$-Alkyl ist; $(X^2)$ ist ein sechsgliedriger Ring, der ein Stickstoffatom enthält; $(X^3)$ ist ein sechsgliedriger Ring, der gegebenenfalls einen Stickstoff enthält, und die gestrichelten Linien bedeuten Einfach- oder Doppelbindungen, und die Gruppe Y ist gegebenenfalls durch einen bis drei Substituenten, ausgewählt aus Halogen, $C_{2-4}$-Alkenyl, $C_{1-4}$-Alkylthio, Amino, mono-( $C_{1-4}$-alkyl)-substituiertes Amino, di-( $C_{1-4}$-alkyl)-substituiertes Amino, Morpholino, Piperidino, Pyrrolidino, Piperazino, Hydroxy, Nitro, $C_{1-4}$-Alkoxycarbonyl oder $C_{1-4}$-Alkyl oder $C_{1-4}$-Alkoxy, jeweils gegebenenfalls substituiert durch Halogen, Hydroxy oder $C_{1-3}$-Alkoxy, substituiert.

**2.** Verbindung nach Anspruch 1 der Formel (III):

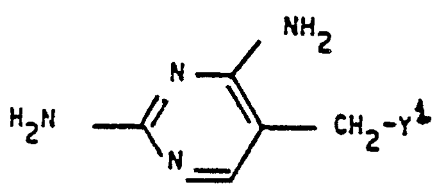

in der $Y^1$ eine Gruppe

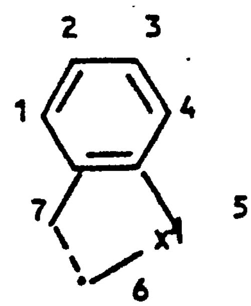

ist, die in der 1- oder 7-Stellung an den Pyrimidinylmethylteil gebunden ist und die gegebenenfalls in der 2-, 3-, 4- oder 6-Stellung oder in der 7-Stellung, wenn die Bindung an den Pyrimidinylteil in der 1-Stellung erfolgt, substituiert ist, und in der $X^1$ und die gestrichelte Linie die in Anspruch 1 angegebene Bedeutung aufweisen.

3. Verbindung nach Anspruch 1 der Formel (VI):

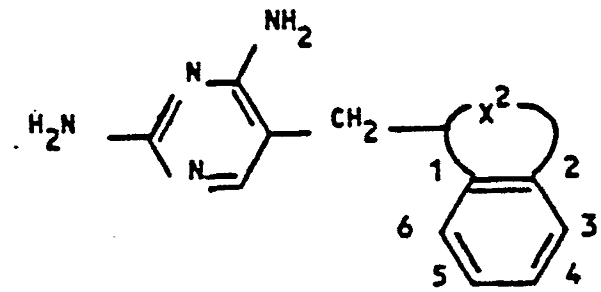

(VI)

oder ein Salz, N-Oxid oder Acylderivat davon, in der ($\bar{x}_2$) ein sechsgliedriger Ring, der ein Stickstoffatom enthält, bedeutet und die beiden Phenylringe und der ($\bar{x}_2$)-Ring gegebenenfalls in einer von der 6-Stellung des Phenylrings verschiedenen Stellung substituiert sind.

4. Verbindung nach Anspruch 1 der Formel (IX):

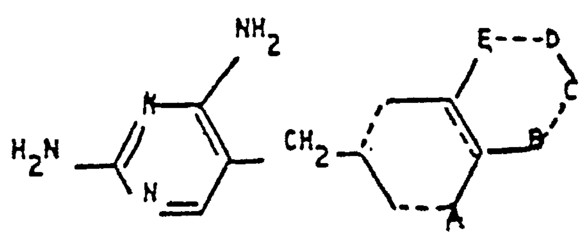

(IX)

oder ein Salz, N-Oxid oder Acylderivat davon, die gegebenenfalls in einer der Stellungen A, B, C, D oder E ein Stickstoffatom enthält, in der die gestrichelten Linien Doppelbindungen bedeuten wenn nicht einer der Ringe ein Stickstoffatom enthält, wobei in diesem Fall die gestrichelten Linien in diesem Ring Einfach- oder Doppelbindungen bedeuten, und in der der bicyclische Ring gegebenenfalls wie in Anspruch 1 definiert, substituiert ist.

5. Verbindung nach einem der Ansprüche 1 bis 4, in der die Gruppe Y gegebenenfalls durch einen bis drei Substituenten, wie in Anspruch 1 definiert, substituiert ist.

6. Verbindung nach Anspruch 2, in der $Y^1$ eine Gruppe:

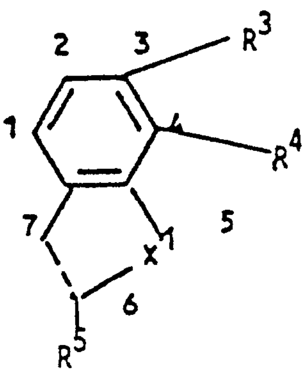

ist, oder ein Salz, N-oxid oder Acylderivat davon, wobei die Gruppe an den Pyrimidinylmethylteil in der 1- oder 7-Stellung gebunden ist; $X^1$ ist Sauerstoff, Schwefel oder eine Gruppe $NR^1$ oder $S(O)_n$, wie vorstehend in Anspruch 1 definiert; $R^3$, $R^4$ und $R^5$ sind gleich oder verschieden und sind aus Wasserstoff, Halogenatomen, $C_{2-4}$-Alkenyl, $C_{1-4}$-Alkylthio, Amino, Mono-$C_{1-4}$-alkylamino, di-$C_{1-4}$-alkylsubstituiertes Amino, Morpholino, Piperidino, Pyrrolidino, Piperazino, Hydroxy, Nitro, $C_{1-4}$-Alkoxycarbonyl oder $C_{1-4}$-Alkyl oder $C_{1-4}$-Alkoxy, jeweils gegebenenfalls substituiert durch Halogen, Hydroxy oder $C_{1-3}$-Alkoxy, ausgewählt, und die gestrichelte Linie weist die in Anspruch 1 gegebene Definition auf.

7. Verbindung nach Anspruch 6, in der $R^3$ und $R^4$ gleich oder verschieden sind und jeweils Wasserstoff, Methyl, Methoxy, Amino, Dimethylamino, Methylthio, Brom oder Chlor bedeuten, und $R^5$ Wasserstoff oder Methyl ist.

8. Verbindung nach Anspruch 6 oder 7, in der $Y^1$ an den Pyrimidinylmethylteil in 1-Stellung gebunden ist.

9. Verbindung nach Anspruch 7 der Formel (IV):

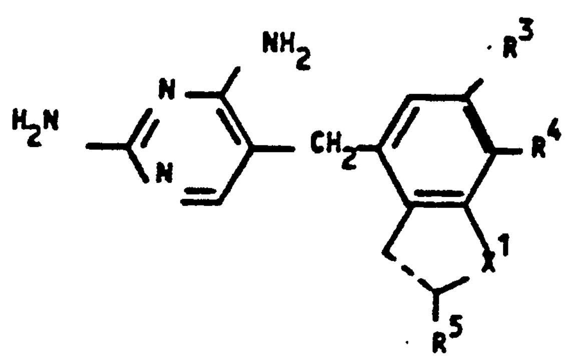

(IV)

oder ein Salz, N-Oxid oder Acylderivat davon, in der $X^1$, $R^3$ bis $R^5$ und die gestrichelte Linie die in Anspruch 7 gegebene Bedeutung aufweisen.

10. Verbindung nach Anspruch 1 oder 3 der Formel (VII):

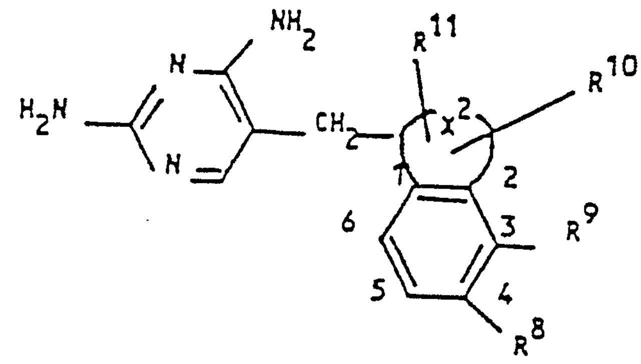

(VII)

oder ein Salz, N-Oxid oder Acylderivat davon, in der $(\bar{x}_2)$ ein sechsgliedriger Ring ist, der drei Doppelbindungen, wobei in diesem Fall $X^2$ -N= ist, zwei Doppelbindungen, wobei in diesem Fall $X^2$ -N= oder $NR^1$-ist, oder eine Doppelbindung enthält, wobei in diesem Fall $X^2$ -N= oder $-NR^{12}$ ist, in der $R^{12}$ eine Gruppe $R^1$ wie in Anspruch 1 definiert ist, oder eine Bindung an die 5-Methylenbrücke zum Pyrimidinring bedeutet;

$R^8$, $R^9$, $R^{10}$ und $R^{11}$ sind gleich oder verschieden und bedeuten jeweils Wasserstoff, Halogen, $C_{2-4}$-Alkenyl, Nitro, Hydroxy, eine Gruppe $-SR^6$, worin $R^6$ $C_{1-3}$-Alkyl ist, eine Gruppe $-COR^7$, in der $R^7$ Methoxy oder Ethoxy bedeutet, oder jeder Rest bedeutet Amino gegebenenfalls durch eine oder zwei $C_{1-4}$-Alkylgruppen substituiert; Morpholino, Piperidino, Pyrrolidino oder $C_{1-4}$-Alkyl oder $C_{1-4}$-Alkoxy, jeweils gegebenenfalls durch Halogen, Hydroxy oder $C_{1-2}$-Alkoxy substituiert.

**11.** Verbindung nach Anspruch 10, in der $R^8$, $R^9$, $R^{10}$ und $R^{11}$ gleich oder verschieden sind und jeweils Wasserstoff, Hydroxy, Methoxy, Ethoxy, Methoxyethoxy, Methyl, Ethyl, Propyl, Amino, Methylamino, Dimethylamino, Ethylamino, Diethylamino, Vinyl, Allyl, Propenyl, Halogen, Methylthio, Ethylthio oder Pyrrolyl bedeuten; der $(\bar{x}_2)$-Ring enthält drei Doppelbindungen und die 5-Methylenbrücke zum Pyrimidinring ist an das bicyclische Ringsystem in der Stellung im heterocyclischen Ring α oder ß zur 1-Stellung des Phenylrings gebunden.

**12.** Verbindung nach Anspruch 4 der Formel (X):

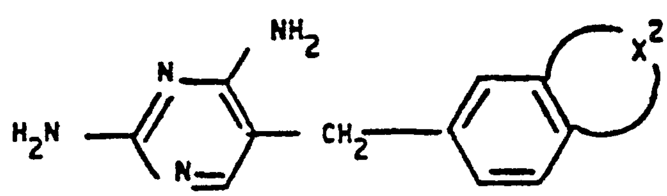

(X)

oder ein Salz, N-Oxid oder Acylderivat davon, in der $(\bar{x}_2)$ ein sechsgliedriger Ring ist, der ein Stickstoffatom enthält; wobei ein bis drei Substituenten an die Kohlenstoffatome des $(\bar{x}_2)$-Rings oder an ein Kohlenstoffatom des Phenylrings benachbart zum $(\bar{x}_2)$-Ring gebunden sind, wobei die Substituenten aus Methoxy, Ethoxy, Methyl, Ethyl, Amino, Dimethylamino, Pyrrolyl, Morpholino, Methoxyethoxy, Chlor, Brom, Methoxycarbonyl oder Ethoxycarbonyl ausgewählt sind.

**13.** Verbindung nach Anspruch 4 oder Anspruch 12 der Formel (XI):

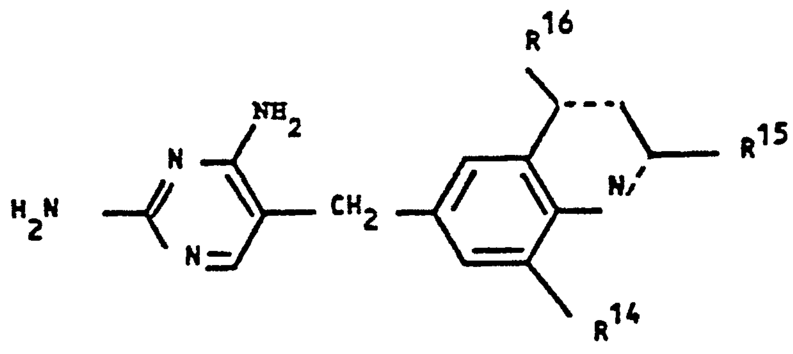

(XI)

oder ein Salz, N-Oxid oder Acylderivat davon, in der die gestrichelten Linien Einfach- oder Doppelbindungen bedeuten, $R^{14}$, $R^{15}$ und $R^{16}$ gleich oder verschieden sind und jeweils Wasserstoff, Methoxy, Ethoxy, Methyl, Ethyl, Amino, Dimethylamino, Pyrrolyl, Morpholino, Methoxyethoxy, Chlor, Brom, Methoxycarbonyl oder Ethoxycarbonyl bedeuten.

**14.** Pharmazeutische Zusammensetzung, enthaltend eine Verbindung der Formel (II) wie in Anspruch 1 definiert, in Kombination mit einem pharmazeutisch annehmbaren Träger.

**15.** Pharmazeutische Zusammensetzung nach Anspruch 14, die als zusätzlichen Wirkstoff ein Sulfonamid enthält.

**16.** Verbindung der Formel (II), wie in Anspruch 1 definiert, zur Verwendung in der Medizin.

**17.** Verfahren zur Herstellung einer Verbindung der Formel (II), wie in Anspruch 1 definiert, wobei das Verfahren umfaßt:

(a) (i) die Reaktion eines Guanidinsalzes mit einer Verbindung der Formel (A) oder (B):

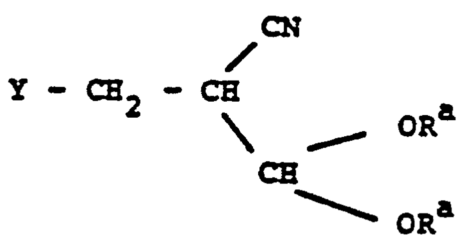

(A)

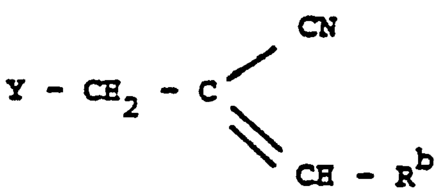

(B)

worin Y die in Anspruch 1 gegebene Bedeutung aufweist, $R^a$ eine $C_{1-4}$-Alkylgruppe und $R^b$ eine nukleophile austretende Gruppe wie eine $C_{1-4}$Alkoxygruppe, z. B. eine Methoxy-, Ethoxy- oder Methoxyethoxygruppe, oder eine Amino-, $C_{1-4}$ -Alkylamino-, Benzylamino-, Di-$C_{1-4}$-Alkylamino-, Naphthylamino-, gegebenenfalls substituierte Anilino-, Morpholino-, Piperidino oder N-Methylpiperazinogruppe ist, wobei $R^b$ am bevorzugtesten eine Anilinogruppe ist;

(ii) die Reaktion einer Verbindung der Formel (C):

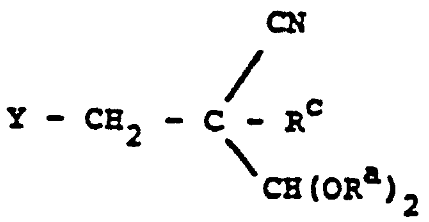

(C)

in der Y und $R^a$ die in Anspruch 1 gegebene Bedeutung aufweisen und $R^c$ eine Alkoxycarbonyl- oder Aldehydgruppe ist, mit Kalium- oder Natriumhydroxid in einem $C_{1-4}$-Alkanol, gefolgt von der Addition von Guanidin;

(iii) die Reaktion einer Verbindung der Formel (D):

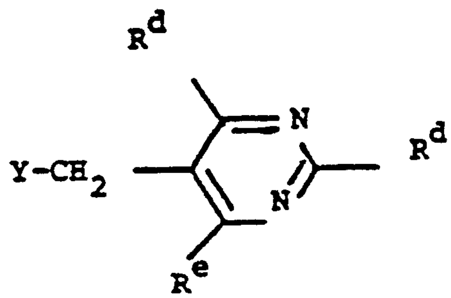

(D)

in der $R^d$ eine Aminogruppe oder eine austretende Gruppe wie eine $C_{1-4}$-Alkylthiogruppe oder ein Halogenatom ist, $R^e$ ein Wasserstoff- oder Halogenatom ist, mit der Ausnahme, daß beide Gruppen $R^d$ nicht Aninogruppen sein können und Y die in Anspruch 1 gegebene Bedeutung aufweist, mit einem Aminierungsmittel wie Ammoniak und anschließend, wenn $R^e$ ein Halogenatom ist, Entfernung von diesem durch Hydrogenolyse;

(iv) die Reaktion einer Verbindung der Formel (E):

$$Y - CH_2Z \qquad (E)$$

in der Z ein Halogenatom oder Hydroxy oder di-$C_{1-4}$-alkylsubstituiertes Amino oder eine andere austretende Gruppe ist, und Y die in Anspruch 1 gegebene Bedeutung aufweist, mit einer Verbindung der Formel (F):

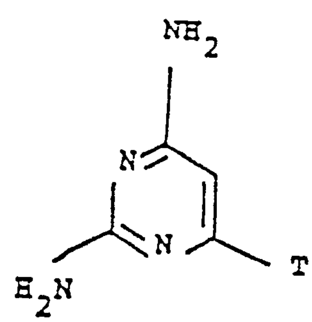

(F)

in der T Wasserstoff oder eine Hydroxy- oder $C_{1-4}$-Alkylthiogruppe ist, und dann Überführung der Gruppe T in Wasserstoff durch Hydrogenolyse wenn T eine $C_{1-4}$-Alkylthiogruppe ist, oder wenn T eine Hydroxygruppe ist, zunächst durch Überführung der Gruppe in das Mesylat- oder Tosylatderivat oder in Thio, Alkylthio oder Halogen, und dann Entfernung von diesem durch Hydrogenolyse;

(b) wenn es erforderlich ist, eine Verbindung der Formel (IV), wie in Anspruch 9 definiert, herzustellen, in der $R^4$ von Wasserstoff verschieden ist, die Cyclisierung einer Verbindung der Formel (G)

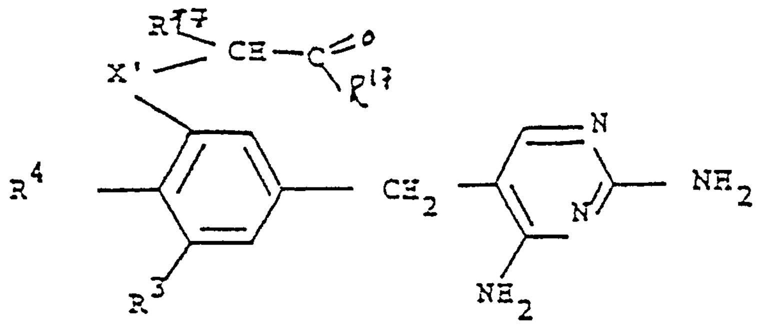

(G)

in der $X^1$, $R^3$ und $R^4$ die in Anspruch 7 gegebene Bedeutung aufweisen, ausgenommen, daß $R^4$ nicht Wasserstoff ist und beide Gruppen $R^{17}$ gleich oder verschieden sind und jeweils Wasserstoff oder $C_{1-4}$-Alkyl bedeuten;

(c) wenn es erforderlich ist, eine Verbindung der Formel (IV), wie in Anspruch 9 definiert, in der die 4-Stellung des Phenylrings gegebenenfalls durch Hydroxy, Alkoxy, Amino oder substituiertem Amino substituiert ist oder (X) wie in Anspruch 12 definiert, in der ($\bar{\bar{x}}_2$ ) ein teilweise gesättigter aromatischer Ring ist, in dem das Stickstoffatom benachbart zum Phenylring ist, herzustellen, die Reaktion einer Verbindung der Formel (H):

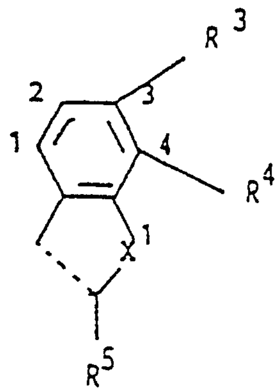

(H)

in der die 4-Stellung des Phenylrings gegebenenfalls durch Hydroxy, Alkoxy, Amino, substituiertem Amino substituiert ist und der ($X^1$)-Ring und der Phenylring durch andere Substituenten als die vorstehend in Anspruch 7 definierten substituiert sind, oder einer Verbindung der Formel (L):

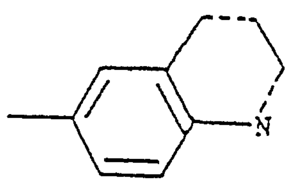

(L)

in der mindestens eine der gestrichelten Linien eine Einfachbindung ist und, wenn die gestrichelte Linie zum Stickstoff eine Einfachbindung darstellt, der Stickstoff durch Wasserstoff oder eine Alkylgruppe substituiert ist, mit 2,4-Diamino-5-hydroxymethylpyrimidin oder einem Ether davon,

(d) die Überführung einer Verbindung der Formel (II) in eine dazu verschiedene Verbindung der Formel (II), beispielsweise durch Reduktion oder Isomerisierung von einer oder zwei der Doppelbindungen, Überführung einer Hydroxygruppe in eine $C_{1-4}$-Alkylthiogruppe oder eine gegebenenfalls substituierte $C_{1-4}$-Alkoxygruppe oder Überführung einer Aminogruppe in eine $C_{1-4}$-Alkylthiogruppe oder Wasserstoff, Halogen, oder Hydroxy über eine Diazogruppe oder in eine substituierte Aminogruppe durch bekannte Verfahren;

(e) wenn es erforderlich ist, eine Verbindung der Formel (IX) herzustellen, in der ein Stickstoffatom

in Stellung B steht und die gestrichelten Linien Doppelbindungen bedeuten, die Kondensation/ Cyclisierung einer Verbindung der Formel M:

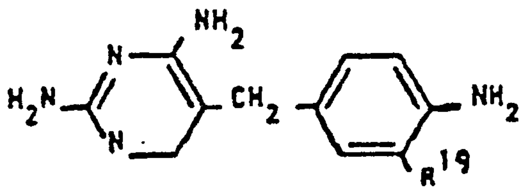

(M)

in der $R^{19}$ Halogen, $C_{1-4}$-Alkyl, $C_{1-4}$-Alkoxy oder $C_{1-4}$-Alkylthio ist, mit einer Verbindung der Formel (N):

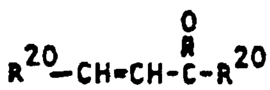

(N)

in der die Gruppen $R^{20}$ gleich oder verschieden sind und jeweils Wasserstoff oder $C_{1-4}$-Alkyl bedeuten, unter sauren Bedingungen wie in verdünnter ethanolischer Chlorwasserstoffsäure;

(f) wenn es erforderlich ist, eine Verbindung der Formel (IX) herzustellen, in der ein Stickstoffatom in der A-, B-, C-, D- oder E-Stellung steht und alle gestrichelten Linien Doppelbindungen bedeuten, die Oxidation einer Verbindung der Formel (IX), in der nicht alle der gestrichelten Linien Doppelbindungen bedeuten, unter geeigneten Dehydrogenierungsbedingungen wie erhöhten Temperaturen in Gegenwart eines Dehydrogenierungskatalysators wie Platinoxid.

**18.** Neue chemische Zwischenverbindung nach einer der Formeln (A) bis (D), wie in Anspruch 17 definiert.